(19)

Europäisches Patentamt

European Patent Office

Office européen des brevets

(11) EP 4 428 866 A1

(12) **EUROPEAN PATENT APPLICATION**
published in accordance with Art. 153(4) EPC

(43) Date of publication:
11.09.2024 Bulletin 2024/37

(21) Application number: 22886869.1

(22) Date of filing: 20.10.2022

(51) International Patent Classification (IPC):
$G16C\ 60/00$ (2019.01) $\quad G06F\ 16/23$ (2019.01)

(52) Cooperative Patent Classification (CPC):
G06F 16/23; G16C 60/00

(86) International application number:
PCT/JP2022/039200

(87) International publication number:
WO 2023/074542 (04.05.2023 Gazette 2023/18)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 01.11.2021 JP 2021178996

(71) Applicant: Sony Group Corporation
Tokyo 108-0075 (JP)

(72) Inventors:
• SHIRASAWA, Raku
Tokyo 108-0075 (JP)
• TETSUKAWA, Hiroki
Tokyo 108-0075 (JP)
• NUMAOKA, Chisato
Tokyo 108-0075 (JP)

(74) Representative: D Young & Co LLP
3 Noble Street
London EC2V 7BQ (GB)

(54) **SYNTHESIS MATERIAL SELECTION METHOD, MATERIAL PRODUCTION METHOD, SYNTHESIS MATERIAL SELECTION DATA STRUCTURE, AND PRODUCTION METHOD**

(57) A synthetic material selection method according to the present disclosure performs material selection for selecting synthesis target materials based on material physical property information and execution possibility information of a database, instructs a control device to perform synthesis processing for the selected materials, and updates the execution possibility information of the database based on a result of the synthesis processing from the control device.

FIG.2

START

S1 SET RANGE OF MATERIAL SEARCH SPACE AND SET TARGET VALUE

S2 SET MATERIAL SEARCH SPACE

S3 SELECT CANDIDATE MATERIALS

S4 SYNTHESIZE

S5 SUCCEEDED IN SYNTHESIS? — NO

YES

S6 MEASURE

S7 SUCCEEDED IN MEASUREMENT? — NO

YES

S8 ACHIEVED TARGET VALUE? — NO / NO

S10 CONSTRUCT/UPDATE PHYSICAL PROPERTY PREDICTION MODEL

S9 CONSTRUCT/UPDATE EXECUTION POSSIBILITY MODEL

YES

S11 CONSTRUCT/UPDATE PHYSICAL PROPERTY PREDICTION MODEL

S12 CONSTRUCT/UPDATE EXECUTION POSSIBILITY MODEL

END

EP 4 428 866 A1

**Description**

Field

The present disclosure relates to a synthetic material selection method, a material manufacturing method, a synthetic material selection data structure, and a manufacturing method.

Background

Various techniques concerning synthesis of novel materials have been provided. For example, there has been known a method of searching for a material to be newly synthesized by searching for a combination of physical property parameters of materials (for example, Patent Literature 1).

Citation List

Patent Literature

Patent Literature 1: WO 2017/221444 A

Non Patent Literature

Non Patent Literature 1: "Bayesian molecular design with a chemical language model" Hisaki Ikebata, Kenta Hongo, Tetsu Isomura, Ryo Maezono, Ryo Yoshida, Journal of Computer-Aided Molecular Design volume 31, pages379-391 (2017)
Non Patent Literature 2: "Machine-learned metrics for predicting the likelihood of success in materials discovery" Kim, Y., Kim, E., Antono, E. et al., npj Comput Mater 6, 131 (2020)

Summary

Technical Problem

However, in the related art explained above, in a search for candidates for a material not synthesized in the past, candidates for a material to be newly synthesized is searched only based on information of a database constructed in advance. Therefore, there is a problem in that it is not possible to search for a material considering "executability of synthesis" determined based on the fact of past material manufacturing results obtained using actual material manufacturing equipment. More specifically, for example, when searching for a material "which not synthesized in the past" using a database, the material search is performed without asking a reason "why the material was not synthesized". Therefore, there is a problem in that a material not successfully synthesized in the past cannot be appropriately searched for and selected considering executability of synthesis such as difficulty of synthesis in actual material manufacturing equipment.

Therefore, the present disclosure proposes a synthetic material selection method, a material manufacturing method, a synthetic material selection data structure, and a manufacturing method that can enable material synthesis based on executability of synthesis. Solution to Problem

According to the present disclosure, a synthetic material selection method includes performing material selection for selecting synthesis target materials based on material physical property information and execution possibility information of a database; instructing a control device to perform synthesis processing for the selected materials; and updating the execution possibility information of the database based on a synthesis processing result from the control device.

Brief Description of Drawings

FIG. 1 is a diagram illustrating a configuration example of a material searching and manufacturing system of the present disclosure.
FIG. 2 is a flowchart illustrating a processing procedure by the material searching and manufacturing system.
FIG. 3 is a flowchart illustrating a processing procedure concerning execution possibility.

FIG. 4 is a diagram illustrating an example of a physical property database according to an embodiment of the present disclosure.

FIG. 5 is a diagram illustrating an example of an executability database according to the embodiment of the present disclosure.

FIG. 6 is a diagram illustrating an example of a material table according to the embodiment of the present disclosure.

FIG. 7 is a diagram illustrating an example of prediction of physical properties according to the embodiment of the present disclosure.

FIG. 8 is a diagram illustrating an example of evaluation of execution possibility according to the embodiment of the present disclosure.

FIG. 9 is a diagram illustrating an example of a search using a physical property prediction model.

FIG. 10 is a diagram illustrating an example of a search using the physical property prediction model and an execution possibility model.

FIG. 11 is a diagram illustrating a detailed example of processing using the physical property prediction model.

FIG. 12 is a diagram illustrating a detailed example of the processing using the physical property prediction model.

FIG. 13 is a diagram illustrating a detailed example of a search using the physical property prediction model and the execution possibility model.

FIG. 14 is a diagram illustrating a detailed example of the search using the physical property prediction model and the execution possibility model.

FIG. 15 is a diagram illustrating a detailed example of the search using the physical property prediction model and the execution possibility model.

FIG. 16 is a diagram illustrating an example of an executability database.

FIG. 17 is a diagram illustrating an example of an executability database.

FIG. 18 is a diagram illustrating an example of an executability database.

FIG. 19 is a diagram illustrating an example of an executability database.

FIG. 20 is a diagram illustrating an example of an executability database.

FIG. 21 is a diagram illustrating an example of an executability database.

FIG. 22 is a sequence chart illustrating an example of a processing procedure in the material searching and manufacturing system.

FIG. 23 is a sequence chart illustrating an example of a processing procedure in the material searching and manufacturing system.

FIG. 24 is a diagram illustrating an example of a configuration of the material searching and manufacturing system.

FIG. 25 is a diagram illustrating a configuration example of an information processing device of the present disclosure.

FIG. 26 is a diagram illustrating an example of a configuration of a material manufacturing device.

FIG. 27 is a diagram illustrating an example of a configuration of a material manufacturing device.

FIG. 28 is a diagram illustrating an example of a configuration of a material manufacturing device.

FIG. 29 is a hardware configuration diagram illustrating an example of a computer that implements functions of the information processing device.

FIG. 30 is a diagram illustrating an example of a material manufacturing result concerning a search.

FIG. 31 is a diagram illustrating an example of a material manufacturing result concerning a search. Description of Embodiments

Embodiments of the present disclosure are explained in detail below with reference to the drawings. Note that a synthetic material selection method, a material manufacturing method, a synthetic material selection data structure, and a manufacturing method according to the present application are not limited by this embodiment. In the embodiments explained below, the same parts are denoted by the same reference numerals and signs to omit redundant explanation.

The present disclosure is explained according to order of items described below.

1. Embodiment

1-1. Overview of a configuration and processing for a material searching and manufacturing system according to an embodiment of the present disclosure

1-1-1. Device configuration of the material searching and manufacturing system
1-1-2. Overall processing flow of the material searching and manufacturing system

1-2. Components of the material searching and manufacturing system

1-2-1. Material manufacturing device

1-2-2. Control system

1-2-2-1. Data
1-2-2-2. Model

1-3. Update processing example of databases and models after a search and material manufacturing processing

1-3-1. Search using only a physical property prediction model and update of the databases and the models after the material manufacturing processing
1-3-2. Search using the physical property prediction model and an execution possibility model and the update of the databases and the models after the material manufacturing processing

1-4. Details of the search and update processing for the databases and the models after the material manufacturing processing

1-4-1. Search using only the physical property prediction model and the update of the databases and the model after the material manufacturing processing
1-4-2. Search using the physical property prediction model and the execution possibility model and the update of the databases and the models after the material manufacturing processing

1-5. Example and material manufacturing results
1-6. Example of an executability database

2. Other processing flow examples

2-1. Processing concerning synthesis
2-2. Processing concerning synthesis
2-3. System configuration example and the like

2-3-1. Information processing device example
2-3-2. Configuration Example and the like of the material manufacturing device

3. Object and effects of the present disclosure
4. Others
5. Hardware configuration

[1. Embodiment]

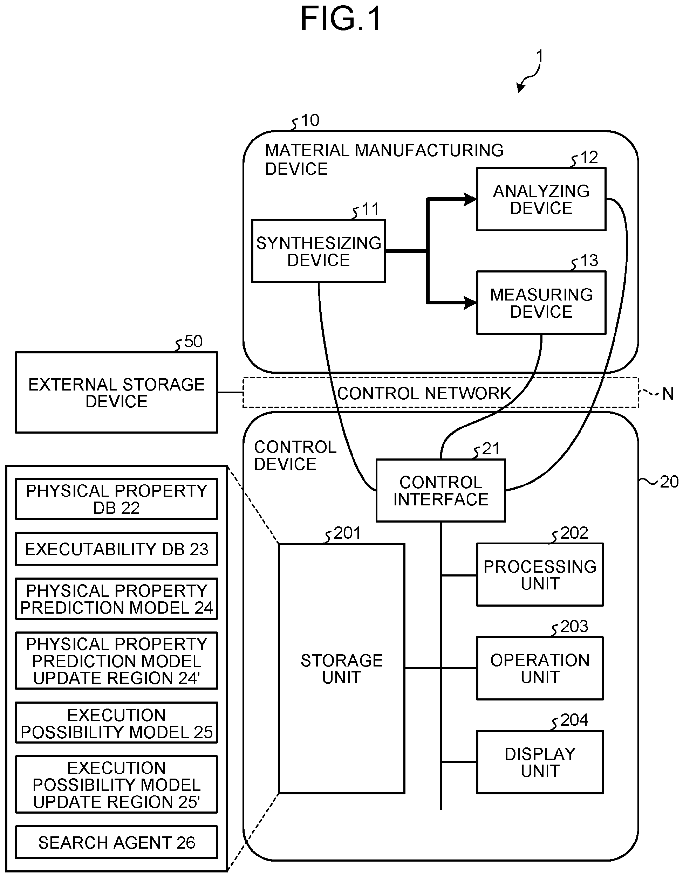

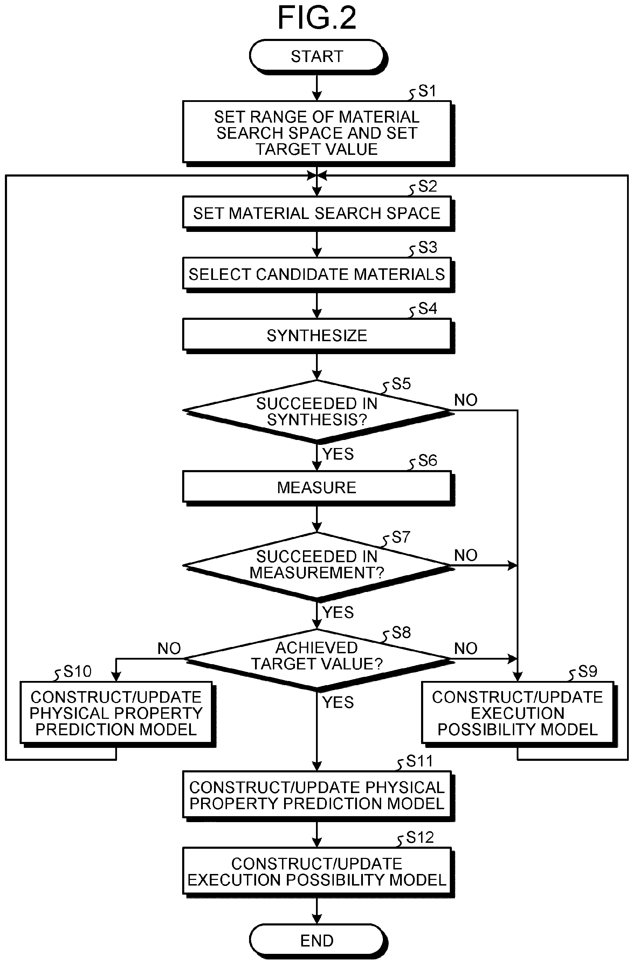

[1-1. Overview of a configuration and processing of a material searching and manufacturing system according to an embodiment of the present disclosure]
In the following explanation, first, a configuration of a material searching and manufacturing system 1, which is an example of an information processing system (also referred to as "processing system") that performs various kinds of processing explained below and an overview of processing performed by the material searching and manufacturing system 1 are explained with reference to FIG. 1 and FIG. 2 and, thereafter, details of components and the processing are explained in order. Note that, in the following explanation, points concerning the configuration and the processing of the material searching and manufacturing system 1 are mainly explained. Detailed explanation about contents of the related art in material search and material synthesis is omitted as appropriate. Any device included in the material searching and manufacturing system 1 may perform processing for which the material searching and manufacturing system 1 explained below is described as a subject of the processing.

[1-1-1. Device configuration of the material searching and manufacturing system]

First, a configuration of the material searching and manufacturing system 1 illustrated in FIG. 1 is explained. FIG. 1 is a diagram illustrating a configuration example of the material searching and manufacturing system of the present disclosure. As illustrated in FIG. 1, the material searching and manufacturing system 1 includes a material manufacturing device 10, a control device 20, and an external storage device 50. For example, the material manufacturing device 10 and the control device 20 are communicably connected by wire or radio via a predetermined communication network (a control network N in FIG. 1). The control device 20 and the material manufacturing device 10 can exchange information with each other through the control network N. The control network N may be any of a dedicated network that can communicate via radio or wire, a local area network, and a wide area network referred to as the Internet or Cloud.

The material manufacturing device 10 includes a synthesizing device 11 that performs material synthesis, an analyzing device 12 that specifies a synthesized material, and a measuring device 13 that performs physical property evaluation. Components (devices) in the material manufacturing device 10 such as the synthesizing device 11, the analyzing device 12, and the measuring device 13 can mutually transfer a material. Configurations of the components are explained below. The external storage device 50 is a server device that retains information used for processing in the material searching and manufacturing system 1. For example, the external storage device 50 provides information to the control device 20.

The control device 20 includes a storage unit 201 (a HDD, an SSD, a ROM, or the like) including a control interface 21 for controlling the material manufacturing device 10, a physical property database 22 (a physical property DB in FIG. 1) for retaining physical property data, an executability database 23 (an executability DB in FIG. 1) for retaining information concerning whether synthesis and/or measurement has been successfully executed, a physical property prediction model 24 for predicting physical properties, an execution possibility model 25 for predicting whether synthesis and/or measurement can be executed, and a search agent 26 (hereinafter referred to as search agent 26) that is a computer program, and a processing unit 202 (an operation unit 203 including a processor (a CPU, a GPU, a GPGPU, a neural network accelerator, or a combination of two or more thereof, or the like.), a user interface (a keyboard, a mouse, a touch panel, a microphone (voice input), a camera (image input such as face)) for operating the control device, and the like) that reads the search agent 26 from the storage unit 201 and performs processing, and a display unit 204 (a display or the like) that displays a processing result of the processing unit 202 and the like.

The components in the control device 20 such as the control interface 21, the processing unit 202, the storage unit 201, the operation unit 203, and the display unit 204 can mutually exchange information via a common control bus/data bus. The physical property prediction model 24 and the execution possibility model 25 may be stored in the storage unit 201 in which the physical property database 22 (the physical property DB in FIG. 1) or the executability database 23 (the executability DB in FIG. 1) are stored or may be stored in another storage device. The physical property prediction model 24, the execution possibility model 25, or the search agent 26 may also be stored in the storage unit 201 in which the physical property prediction model 24 and the execution possibility model 25 are stored or may be stored in another storage device. Further, a part of the storage unit 201 may be configured as a device (for example, the external storage device 50 or the like) separate from the control device 20 and connected via a not-illustrated network interface connected to the control network N. The storage unit 201 and another storage device (for example, the external storage device 50) may cooperate to configure virtual storage using a function of a not-illustrated operating system. Although not illustrated, data communication via a communication control device/communication infrastructure such as a router or a switch may be performed between the control interface 21 and the control network N and between the control network N and the synthesizing device 11, the analyzing device 12, or the measuring device 13.

Note that, as explained above, the search agent 26 may function as a computer program and implement functions or may function as a not-illustrated dedicated processor and implement the functions using hardware, cooperate with the processor, or operate as a part of the processor. Further, as explained below, the physical property prediction model 24 and the execution possibility model 25 are updated by relearning. The physical property prediction model 24 and the execution possibility model 25 may be machine learning models implemented by a machine learning algorithm such as Bayesian optimization, a neural network, or an SVM (Support Vector Machine).

Here, the storage unit 201 further stores a software program for executing a not-illustrated machine learning algorithm. By processing the program, the processing unit 202 can read the physical property prediction model 24 from the storage unit 201 and update the physical property prediction model 24 using not-illustrated machine learning data. In this case, the processing unit 202 copies the physical property prediction model 24 halfway being updated to a physical property prediction model update region 24' and generates a model after the update of the physical property prediction model 24.

In this way, an updated physical property prediction model can be generated based on the physical property prediction model 24. That is, the processing unit 202 can manufacture a machine learning model after the update by copying the physical property prediction model 24, which is a machine learning model read from the storage unit 201, to the physical property prediction model update region 24' by the software program for executing the machine learning algorithm and applying the machine learning data to the copy. By writing the manufactured machine learning model after the update as the physical property prediction model 24 again in the storage unit 201, the machine learning model after the update can be continuously used as the physical property prediction model 24.

As explained above, the control device 20 can perform processing for manufacturing the physical property prediction model 24, which is the updated machine learning model. Similarly, an updated execution possibility model can be generated based on the execution possibility model 25. In this case, an execution possibility model update region 25' of the storage unit 201 is used. That is, the processing unit 202 can manufacture the machine learning model after update by copying the execution possibility model 25, which is the machine learning model read from the storage unit 201, to the execution possibility model update region 25' by the software program for executing the machine learning algorithm and applying the machine learning data to the copy. By writing the manufactured machine learning model after the update in the storage unit 201 again as the execution possibility model 25, the machine learning model after the update can be continuously used as the execution possibility model 25.

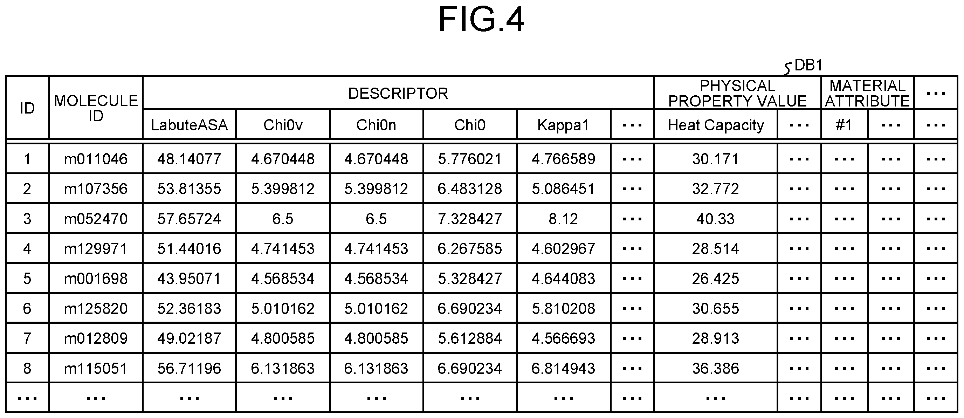

As explained above, the control device 20 can perform processing for manufacturing the execution possibility model 25, which is the updated machine learning model. Data structures illustrated in FIG. 4 to FIG. 20 can be displayed on the display unit 204 according to input from the operation unit 203. The processing unit 202 can perform an instruction to start, stop, or correct the processing illustrated in FIG. 2 according to input from the operation unit 203.

[1-1-2. Overall processing flow of the material searching and manufacturing system]

Next, an overall processing flow of the material searching and manufacturing system is explained with reference to FIG. 2. FIG. 2 is a flowchart illustrating a processing procedure by the material searching and manufacturing system. For example, FIG. 2 illustrates an example of a material manufacturing method performed by the material searching and manufacturing system 1. For example, the material searching and manufacturing system 1 is driven by the search agent 26 and performs a material search according to the following procedure.

As illustrated in FIG. 2, the material searching and manufacturing system 1 sets a range of a material search space and a target value of material physical properties (Step S1). Then, the material searching and manufacturing system 1 sets the material search space (Step S2). For example, the material searching and manufacturing system 1 uses a prediction result by the physical property prediction model 24 and (or) a prediction result by the execution possibility model 25 to prioritize materials included in the material search space. For example, the prediction result (output) of the physical property prediction model 24 or the execution possibility model 25 may be regression analysis data. Details are explained below. The physical property prediction model 24 and the execution possibility model 25 may be models of machine learning using machine learning algorithms such as Bayesian optimization, neural network, and SVM. Details about the models are explained below.

Then, the material searching and manufacturing system 1 selects candidate materials based on the priority of the materials (Step S3). For example, the control device 20 of the material searching and manufacturing system 1 selects synthesis target materials based on material physical property information in the physical property database 22 and execution possibility information in the executability database 23. The material searching and manufacturing system 1 synthesizes the candidate materials (Step S4). For example, the control device 20 of the material searching and manufacturing system 1 instructs the material manufacturing device 10 of the material searching and manufacturing system 1 to perform synthesis processing for the selected materials. The material manufacturing device 10 performs the synthesis processing for the materials according to the instruction from the control device 20.

When failed in the synthesis (Step S5: No), the material searching and manufacturing system 1 updates the data to indicate that the material searching and manufacturing system 1 has "failed" in the synthesis in an item indicating "executability" in the executability database 23 and further updates a "prediction value" of "executability" to be low under a condition that the material searching and manufacturing system 1 has failed in the synthesis in the execution possibility model 25 (Step S9) and returns to Step S2 and repeats the processing. For example, the material manufacturing device 10 of the material searching and manufacturing system 1 transmits a result of the synthesis processing to the control device 20 of the material searching and manufacturing system 1. The control device 20 updates the execution possibility information of the executability database 23 based on the synthesis processing result from the material manufacturing device 10. By updating the executability database 23 and the execution possibility model 25 in this way, at the time of the next material search, a prediction value of "execution possibility" of the materials for which the material searching and manufacturing system 1 has failed in the synthesis is set to be low under the same synthesis condition such that, in the next and subsequent selection of candidate materials, the priority can be lowered under the same condition as the condition of the synthesis failure and a probability that materials other than the materials for which the material searching and manufacturing system 1 has failed in the synthesis are selected can be increased.

When succeeded in the synthesis (Step S5: Yes), the material searching and manufacturing system 1 measures material physical properties (Step S6).

When failed in the physical property measurement (Step S7: No), the material searching and manufacturing system 1 updates the data to indicate that the synthesis has been "successful" but the measurement has been "unsuccessful" in the item indicating "executability" in the executability database 23, further updates the "prediction value" of the "execution possibility" to be low under the condition in which the measurement has been unsuccessful in the execution possibility model 25 (Step S9), and returns to Step S2 and repeats the processing. By updating the executability database 23 and the execution possibility model 25 in this way, at the time of the next material search, under the same synthesis condition, the prediction value of "execution possibility" is set to be low for a material for which the material searching and manufacturing system 1 has been unsuccessful in the measurement such that the priority can be made low under the same condition as the physical property measurement failure in the selection of the next and subsequent candidate materials, and a probability that a material other than the material for which the material searching and manufacturing system 1 has been unsuccessful in the synthesis is selected can be increased.

When succeeded in the measurement (Step S7: Yes), the material searching and manufacturing system 1 determines whether the physical property value has reached the target value (Step S8).

When the physical property value has not reached the target value (Step S8: No), the material searching and manufacturing system 1 updates the "physical property " of the physical property database 22 with the measured physical property value and updates the physical property prediction model 24 such that the measured physical property value is predicted to be lower than the target value under the same synthesis condition for the materials for which the synthesis has been successful (Step S10). In the item indicating "executability" in the executability database 23, the data is updated to indicate that both of the synthesis and the measurement are "successful" and, further, in the execution possibility model 25, the "prediction value" of "execution possibility" is updated to be high under the condition in which the synthesis has been successful (Step S9), and returns to Step S2 and repeats the processing. By updating the physical property database 22 and the physical property prediction model 24 in this way, at the time of the next material search, under the same synthesis condition, the prediction value of the "physical property" is set to be lower than the target value for the material for which the material searching and manufacturing system 1 has failed in the achievement of the target value such that the priority can be lowered under the same condition as that for the material for which the achievement of the target value has been unsuccessful in the next and subsequent selection of candidate materials and a probability that materials other than the materials for which the material searching and manufacturing system 1 has been unsuccessful in the achievement of the target value is selected can be increased.

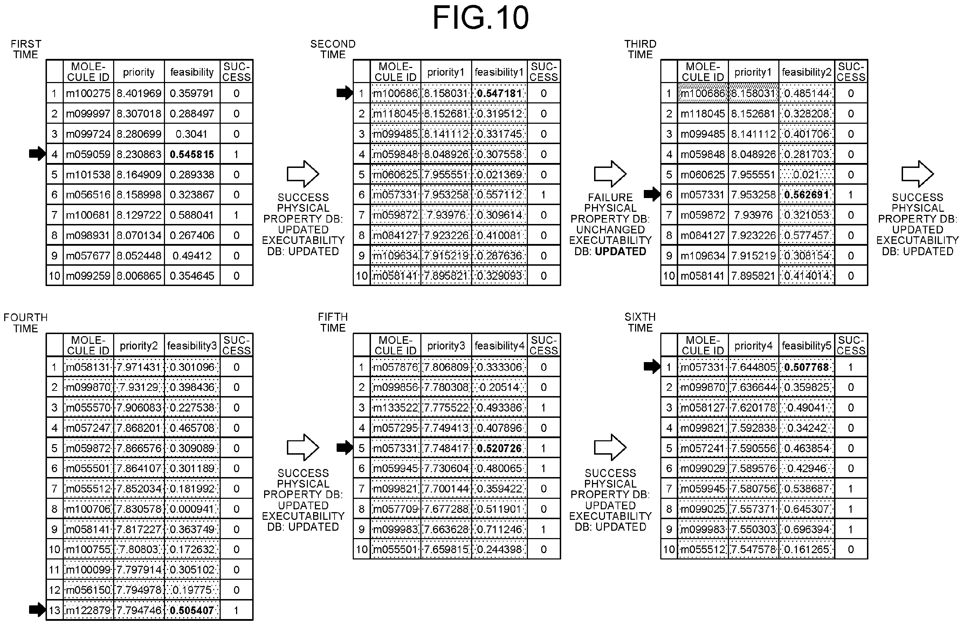

When the physical property value has reached the target value (Step S8: Yes), the material searching and manufacturing system 1 updates the "physical property" in the physical property database 22 with the measured physical property value and updates the physical property prediction model 24 such that, under the same synthesis condition, the measured physical property value is predicted for the materials for which the synthesis has been successful (Step S11). In addition, in the item indicating "executability" in the executability database 23, the material searching and manufacturing system 1 updates data indicating that both the synthesis and the measurement have been "successful" and, further, in the execution possibility model 25, updates the execution possibility model such that the "prediction value" of" execution possibility" becomes 1 or a value as close as possible to 1 for the materials for which the synthesis has been successful (Step S12) and ends the material search. By updating the executability database 23 and the execution possibility model 25 in this way, at the time of the next material search, the prediction value of "execution possibility" becomes 1 in the prediction for the materials for which the synthesis has been successful or, by collating with the "synthesized material list" before the prediction, the priority can be lowered in the next and subsequent selection of candidate materials, and further, the materials for which the material synthesis has already been successful can be prevented from being selected. In the following explanation, for example, in FIG. 10 to FIG. 15, in some case, a prediction result based on the physical property prediction model 24 is properly used as "priority" and a prediction result based on the execution possibility model 25 is properly used as "execution possibility". However, it should be understood that these are as one of embodiments. In the present invention, selection order determined based on the prediction results of these two models is collectively referred to as "priority" as a higher-order concept.

In the related art in which a material search is performed, generally, a computation-costly method such as high-accuracy first-principle calculation, QM/MM calculation, a molecular dynamics method, a roughening molecular dynamics method, or multi-scale simulation has been used for the material search. In the material search based on such a calculation theory, since execution possibility in material manufacturing (synthesize possibility and measurability) was not considered, materials that could not actually be synthesized or materials that could not be measured were selected as candidate materials and synthesis or evaluation were attempted using the selected materials and were unsuccessful. Therefore, appropriate materials were not able to be selected. As explained above, in the related art in which the material search is performed, since the execution possibility (synthesize possibility and measurability) in material manufacturing is not considered, materials that could not be actually synthesized or materials that could not be measured were used as candidate materials, search efficiency was poor.

On the other hand, in the material search in the material searching and manufacturing system 1, since the search is performed taking into account the executability of the synthesis, the material synthesis based on the executability of the synthesis can be enabled. In the material search in the material searching and manufacturing system 1, since the execution possibility model 25 is updated based on the synthesis/measurement processing, by considering the possibility of success, prediction accuracy can be improved and a new material having excellent characteristics (physical properties) can be found with a small number of times of execution. Further, since the selection of materials that should be synthesized is performed considering the executability of synthesis, even when a material search is performed in a set search space, materials excluded from choices in the past only because there is no synthesis result are preferentially selected as synthesis candidates. Therefore, it is possible to first give an opportunity for synthesis processing to materials that have not been searched in the past and to increase possibility of being able to search for new materials.

As explained above, the material searching and manufacturing system 1 is a material searching and manufacturing system incorporating an automatic material manufacturing device, a physical property prediction model, and an execution possibility (synthesize possibility and measurability) model. Therefore, the material searching and manufacturing system 1 can implement highly efficient search and synthesis of materials and can improve search efficiency by confirming synthesize possibility of materials and quickly obtaining a design space for synthesizable and measurable materials.

[1-2. Components of the material searching and manufacturing system]

An overview of the components of the material searching and manufacturing system 1 is explained below.

[1-2-1. Material manufacturing device]

Points concerning the material manufacturing device 10 are explained. First, hardware concerning the material manufacturing device 10 is explained. The synthesizing device 11 internally has a mixing/reaction vessel, a reagent/solvent stock, and a product separation function or is connected to external equipment having these functions. These components can exchange contents with one another. The synthesizing device 11 performs mixing of a reagent and a solvent, synthesis of candidate materials, and separation of a product based on a command from the control device 20. The synthesizing device 11 determines whether these kinds of operation have been successful using at detecting unit (a detection circuit or a detection software program operating on a processor) and reports (transmits) a determination result to the control device 20.

The analyzing device 12 receives a product synthesized by the synthesizing device 11 and determines whether the product matches candidate materials. Consequently, the analyzing device 12 generates an analysis result. The analyzing device 12 includes a detecting unit that determines whether the analysis has been successful (generates a success determination result). The analyzing device 12 transmits an analysis result and a success determination result to the control device 20.

The measuring device 13 receives a material synthesized by the synthesizing device 11 and determined as matching the candidate materials by the analyzing device 12 and measures material physical properties. Consequently, the measuring device 13 generates a measurement result. The measuring device 13 includes a detecting unit that determines whether measurement of physical properties has been successful (generates a success determination result). The measuring device 13 transmits a measurement result and a success determination result to the control device 20.

The material manufacturing device 10 performs material manufacturing for manufacturing a material. For example, the material manufacturing device 10 performs, according to the control by the control device 20, material manufacturing for manufacturing a material. When receiving an instruction for synthesis processing for the materials selected using the material physical property information and the execution possibility information, the material manufacturing device 10 manufactures a material according to the instruction of the synthesis processing. For example, the material manufacturing device 10 performs material manufacturing with the synthesizing device 11 targeting the material indicated by the instruction of the synthesis processing. Note that the material manufacturing device 10 may be configured to cooperate with a plurality of different material manufacturing devices 10 in data sharing and processing control through a communication network. The material manufacturing device 10 may have at least one function of synthesis, analysis, or measurement processing concerning material manufacturing.

Next, software concerning the material manufacturing device 10 is explained. The material manufacturing device 10 may internally include software having a physical property evaluation function for evaluating physical properties or may be used by being connected to external equipment having this function. In this case, the material manufacturing device 10 executes a material simulation according to an instruction from the control device 20. The material manufacturing device 10 determines whether the material synthesis has been successful (generates a success determination result) and transmits the success determination result and the material synthesis result to the control device 20.

Note that the material manufacturing device 10 may be configured by hardware or may be configured by software. A plurality of the material manufacturing devices 10 may be provided and may be connected via a predetermined network such as the Internet. This point is explained below.

[1-2-2. Control system]

Next, points concerning the control device 20 are explained. The physical property prediction model 24 is a regression model that is estimated from data of the physical property database 22 and predicts a physical property value from a material. The regression model may return only a prediction value concerning a material or may return a variance between prediction values.

The execution possibility model 25 is represented by the following Expression (1).

$$F_{FS} = F_{SYNTH} * F_{MEAS} \qquad \cdots (1)$$

Here, $F_{FS}$ corresponds to the execution possibility model 25, $F_{SYNTH}$ corresponds to a synthesize possibility model (Synthesizability Model. Range 0 to 1), and $F_{MEAS}$ is an evaluation possibility model (Measurability Model. Value range 0 to 1). The synthesize possibility model $F_{SYNTH}$ is a model for predicting whether synthesis will be successful and is represented by the following Expression (2).

$$F_{SYNTH} = F_{PATH} * \prod_{N_S} F_{Step} \qquad \cdots (2)$$

Here, $F_{PATH}$ is a synthetic path model, $F_{Step}$ is a step synthesize possibility model (Synthetic Step Model), and $N_S$ is the number of synthesis steps. The synthetic path model $F_{PATH}$ is a model indicating whether a synthetic route is present and is represented by the following Expression (3).

$$F_{PATH} = \begin{cases} 1 & if \ synthetic \ path \ exists \\ 0 & else \end{cases} \qquad \cdots (3)$$

The synthetic path model $F_{PATH}$ has a value of 0 or 1. For example, the synthetic path model $F_{PATH}$ is 1 if there is a synthesis path and is 0 otherwise. The number of synthesis steps $N_S$ satisfies the following Expression (4) .

$$N_S \leq N_{MAX} \qquad \cdots (4)$$

For example, $N_{MAX}$ indicates a maximum value of the number of synthesis steps.
The step synthesize possibility model $F_{Step}$ is a model that predicts whether a one-step synthesis process will successful and is represented by the following Expression (5).

$$S_{Step} = F_{Step}^{Yield} * F_{Step}^{Pid} * F_{Step}^{Iso} \qquad \cdots (5)$$

Here, the following term (6) is a step synthesis yield model (Yield Model. Value range 0 to1).

$$F_{Step}^{Yield} \qquad \cdots (6)$$

The following item (7) is a product identifiability model. Value range 0 to 1).

$$F_{Step}^{Pid} \qquad \cdots (7)$$

The following item (8) is a product isolatability model. Value range 0 to 1).

$$F_{Step}^{Iso} \qquad \cdots (8)$$

The control device 20 predicts a yield of the synthesis step, predicts whether a product can be determined as matching a target material, and whether the product can be isolated, respectively. The evaluation possibility model $F_{MEAS}$ is a model for predicting whether measurement will be successful.

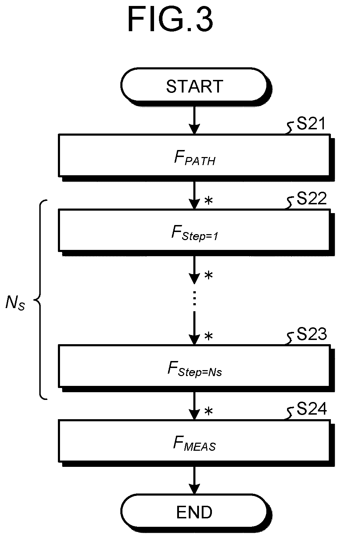

FIG. 3 corresponds to a flow of processing by the execution possibility model 25 explained above. FIG. 3 is a flowchart illustrating a processing procedure concerning execution possibility. The execution possibility model 25 derives values in respective kinds of processing corresponding to Steps S21 to S24 in FIG. 3 and generates an output value (a score) indicating execution possibility based on the values. For example, the execution possibility model 25 derives a value by the synthetic path model $F_{PATH}$ (Step S21). The execution possibility model 25 derives a value by the step synthesize possibility model $F_{Step}$ according to the number of steps (Steps S22 and S23) . Note that, although only first and Ns-th times of the synthesis steps are illustrated in FIG. 3, the execution possibility model25 derives a value by the step synthesize possibility model $F_{Step}$ corresponding to each of the first to Ns-th times. The execution possibility model 25 derives a value by the evaluation possibility model $F_{MEAS}$ (Step S24). Then, the execution possibility model25 derives a value of the execution possibility model $F_{FS}$ using values by the synthetic path model $F_{PATH}$, the step synthesize possibility model $F_{Step}$, the evaluation possibility model $F_{MEAS}$, and the like.

Note that, as a model group included in the execution possibility model 25, models estimated from values all obtained in a material manufacturing process or simulation results may be used or models represented by a constant or a simplified empirical formula may be used.

The search agent 26 determines priority of candidate materials based on a prediction value obtained from the physical property prediction model 24 and/or a value obtained from the execution possibility model 25, selects candidate materials to be synthesized and measured next according to the priority, and executes synthesis and measurement of the selected candidate materials. The synthesis and the measurement are executed by giving an instruction from the search agent 26 to the material manufacturing device 10 through the control interface 21. The control device 20 receives "whether successfully executed" (executability data) and "measured value" (physical property data) reported from the material manufacturing device 10 through the control interface 21 and updates the executability database 23, the execution possibility model25, and the physical property database 22, and the physical property prediction model 24, respectively. The search agent 26 may be present on the same computer device as the control interface 21 or may be present on another computer device.

As explained above, for example, the control device 20 performs material selection for selecting materials using the material physical property information stored in the physical property database 22 and the execution possibility information stored in the executability database 23. For example, the control device 20 instructs the material manufacturing device 10 to perform synthesis processing for the selected materials. For example, the control device 20 updates the execution possibility information stored in the executability database 23 based on the synthesis processing result from the material manufacturing device 10.

[1-2-2-1. Data]

An example of data used by the control device 20 is explained below with reference to the drawings.

First, an example of the physical property database 22 used by the control device 20 is explained with reference to FIG. 4. FIG. 4 is a diagram illustrating an example of a physical property database according to the embodiment of the present disclosure. A database DB1 illustrated in FIG. 4 corresponds to the physical property database 22. The database DB1 stores data (learning data) used for learning of the physical property prediction model 24. In the example illustrated in FIG. 4, the database DB1 includes items such as "ID", "molecule ID", "descriptor", "physical property value", and "material attribute". As illustrated in FIG. 4, the database DB1, which is a physical property database, includes two or more synthesis processing target materials.

The "ID" indicates identification information for identifying data. The "molecule ID" indicates identification information for identifying a molecule. The "Descriptor" indicates a descriptor. In the example illustrated in FIG. 4, a case in which "LabuteASA", "Chi0v", "Chi0n", "Chi0", "Kappa1", and the like are used as descriptors is illustrated. However, FIG. 4 is merely an example and any descriptor can be adopted as the "descriptor". In the item "descriptor", data (a value) obtained by digitizing chemical characteristics and the like of a molecule corresponding to the item is stored.

The "physical property value" indicates a physical property value of the molecule. In the example illustrated in FIG. 4, a case in which "Heat Capacity" is used as a physical property value is illustrated. However, FIG. 4 is merely an example and any physical property value can be adopted as the "physical property value". The "physical property value" may be physical property values concerning various properties such as mechanical properties, thermal properties, electrical properties, magnetic properties, optical properties, electrochemical properties, drug efficacy, toxicity, antibody reaction, interaction with a cell, interaction with an internal organ, intracellular transportability, in-vivo transportability, adsorbability, and solubility. In the item of "physical property value", data (a value) concerning a property of the molecule is stored. In the item "physical property value", when measurement has been successful for the molecule, the value is updated to a measured physical property value. For example, the control device 20 updates the value of the "physical property value" based on a measurement result.

Note that a data structure concerning the "physical property value" illustrated in FIG. 4 is merely an example and the data structure may be, for example, in a list format. For example, the data structure may be in a format of "physical properties" => list (physical property value: value,...). For example, the data structure may be a format such as ID: 1, physical property: {"HeatCapacity" : P1, "Solubility" : P2,...}, or the like.

The "material attribute" indicates an attribute of a molecule (a material) as a material. In the example illustrated in FIG. 4, only "#1" that stores one material attribute is illustrated for explanation. However, "#2", "#3", and the like are provided as many as the number of material attributes. The "material attribute" may be various material attributes such as low molecules, dyes, polymers, fluorescent and identified isotope labels, self-assembled materials and structures, biomaterials (saccharides, peptides, polypeptides, amino acids, proteins, fatty compounds, DNA (Deoxyribonucleic Acid), and the like), organic thin films (deposition and application process), inorganic materials (solid phase method, coprecipitation method, melt quenching method, sol-gel method, and the like), nanoparticles, metal complexes, inorganic thin films (ALD (Atomic layer deposition), sputtering, and the like), synthetic materials based on synthetic biological techniques (material synthesis by genetic recombination and bacterial utilization), and functional materials having a crystal structure, a nanostructure, and a microstructure. In the item of "material attribute", information indicating a material attribute of the molecule (the material) is stored.

Note that the data structure concerning the "material attribute" illustrated in FIG. 4 is merely an example. The data structure may be, for example, a list format. For example, the data structure may be "attribute" => list (attribute value: value,...). For example, the data structure may be ID: 1, attribute: {low molecule, dye,...}, or the like. As explained above, the information (the material physical property information) stored in the database DB1, which is an example of the physical property database 22, is structured to include at least two items. One of the at least two items is an attribute item of materials.

Note that the database DB1 is not limited to the above and may store various kinds of information according to purposes. For example, the database DB1 stores information indicating molecules (materials) successfully synthesized in the past. The database DB1 may store a flag (a value) indicating a molecule (a material) successfully synthesized in the past in association with a molecule ID of the molecule. The database DB1 may exclude molecules successfully synthesized in the past.

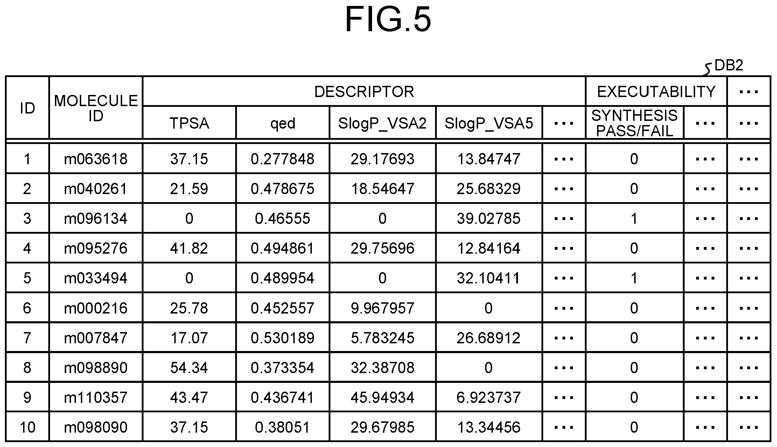

Next, an example of the executability database 23 used by the control device 20 is explained with reference to FIG. 5. FIG. 5 is a diagram illustrating an example of the executability database 23 according to the embodiment of the present disclosure. A database DB2 illustrated in FIG. 5 corresponds to the executability database 23. For example, the database DB2 stores data (learning data) used for learning the execution possibility model 25. In the example illustrated in FIG. 5, the database DB2 includes items such as "ID", "molecule ID", "descriptor", and "executability".

The "ID" indicates identification information for identifying data. The "molecule ID" indicates identification information for identifying a molecule. The "Descriptor" indicates a descriptor. In the example illustrated in FIG. 5, a case in which "TPSA", "qed", "SlogP_VSA2", "SlogP_VSA5", and the like are used as descriptors is illustrated. However, FIG. 5 is merely an example and any descriptor can be adopted as the "descriptor". In the item "descriptor", data (a value) obtained by digitizing chemical characteristics and the like of a molecule corresponding to the item is stored.

The "executability" indicates executability of the molecule. In the example illustrated in FIG. 5, a case in which "synthesis pass/fail" is used as an item concerning executability is illustrated. However, FIG. 5 is merely an example and any executability can be adopted as the "executability". The "executability" may be an element concerning various kinds of executability such as measurability. In the item of "executability", data (a value) indicating executability concerning the element is stored.

Note that the database DB2 is not limited to the above and may store various kinds of information according to purposes. For example, the database DB2 stores information indicating molecules (materials) successfully synthesized in the past. The database DB2 may store a flag (a value) indicating a molecule (a material) successfully synthesized in the past in association with a molecule ID of the molecule. The database DB2 may exclude molecules (materials) successfully synthesized in the past. An example of the executability database 23 other than the database DB2 is explained below.

Here, for data of data records included in the physical property database 22 and the executability database 23, reference links may be retained to be mutually accessible. Alternatively, the agent software may perform merging, filtering operation, and the like between these two databases via relational database management software to make it possible to access data of any data record in an integrated manner. Note that, in the present disclosure, what is written as database is assumed to include a plurality of data tables of the same type. It is assumed that, when there is a description suggesting a data table in the specification, it may mean a table managed in the database and, when a term of table is included in the explanation of the database, it indicates a database as a superordinate concept.

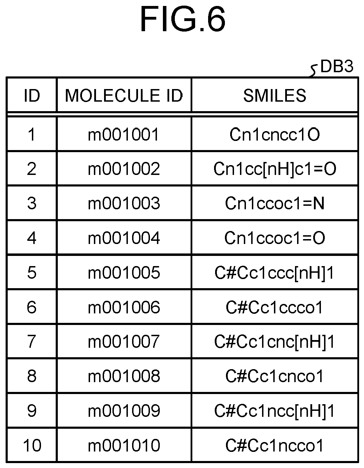

Next, an example of a material table used by the control device 20 is explained with reference to FIG. 6. FIG. 6 is a diagram illustrating an example of a material table according to the embodiment of the present disclosure. A table DB3 illustrated in FIG. 6 is included in the control device 20. For example, the table DB3 stores data (learning data) used for learning the execution possibility model 25. In the example illustrated in FIG. 6, the table DB3 includes items such as "ID", "molecule ID", and "SMILES".

The "ID" indicates identification information for identifying data. The "molecule ID" indicates identification information for identifying a molecule. "SMILES" indicates expression by a molecule SMILES notation. In the item "SMILES", information obtained by converting a chemical structure of the molecule into a character string in alphanumeric characters of ASCII code is stored. Note that, when materials other than organic molecules such as inorganic materials are handled, "material ID" or the like may be used instead of the "molecule ID" and "synthesis ratio" or the like may be used instead of the "SMILES". The "material ID" indicates identification information for identifying a material. The "synthesis ratio" is a row of numerical values indicating element species configuring a material and a content ratio of the element species.

Note that the table DB3 is not limited to the above and may store various kinds of information according to purposes.

[1-2-2-2. Model]

Next, an example of an overview of processing using a model by the control device 20 is explained with reference to the drawings.

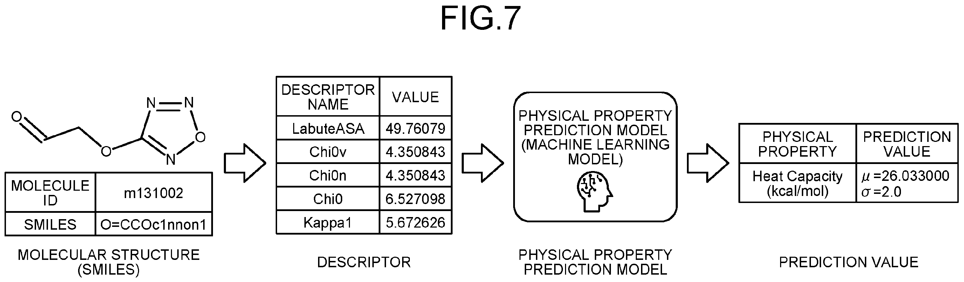

First, an example of prediction of physical properties using the physical property prediction model 24 by the control device 20 is explained with reference to FIG. 7. FIG. 7 is a diagram illustrating an example of prediction of physical properties according to the embodiment of the present disclosure.

As illustrated in FIG. 7, the control device 20 derives values corresponding to descriptors using a character string in which a molecule identified by a molecule ID is expressed by the SMILES notation. Then, the control device 20 inputs the values corresponding to the descriptors to the physical property prediction model 24 to thereby output a prediction value of a physical property from the physical property prediction model 24. In FIG. 7, the physical property prediction model 24 receives values corresponding to descriptors of "LabuteASA", "Chi0v", "Chi0n", "Chi0", and "Kappa1" as input and outputs an average and a variance predicted for a physical property "Heat Capacity" as prediction values of the physical property "Heat Capacity". For example, the control device 20 inputs a material feature value concerning a molecule (a material) to the physical property prediction model 24 to thereby output a prediction value of a physical property (hereinafter also referred to as "first prediction value") from the physical property prediction model 24. For example, the physical property prediction model 24 outputs a first prediction value according to input of material feature values of two or more materials configuring the materials. The material feature values referred to herein are, for example, a numerical string uniquely obtained from a molecular structure and may be, for example, a molecular fingerprint (ECFP4) or a Coulomb matrix obtained from a bonding relation among atoms in a molecule, a molecular descriptor calculated from various configurational and structural features such as the number of contained atomic species, a feature vector of a molecule extracted by a graph-convolutional neural network (GCN), or the like.

The input and output explained above are merely examples and the physical property prediction model 24 may receive various kinds of information as input and output the various kinds of information. For example, the physical property prediction model 24 may output a standard deviation rather than the variance. The physical property prediction model 24 may receive values corresponding to the descriptors as input and output one value concerning a physical property and a value indicating a certainty factor of the value. In this case, the control device 20 may take one value concerning the physical properties as an average and calculate a variance based on the certainty factor. For example, the control device 20 may calculate the standard deviation smaller as the certainty factor is higher. A plurality of physical property prediction models 24 may be generated by being learned for of physical properties. The physical property prediction model 24 may be a model learned to output a plurality of physical properties.

The control device 20 trains and updates the physical property prediction model 24 according to a learning algorithm using the data stored in the physical property database 22 as learning data. Note that the learning of the physical property prediction model 24 by the control device 20 is so-called supervised learning and, although detailed explanation thereof is omitted, the control device 20 may learn the physical property prediction model 24 with any processing if the physical property prediction model 24 can be learned.

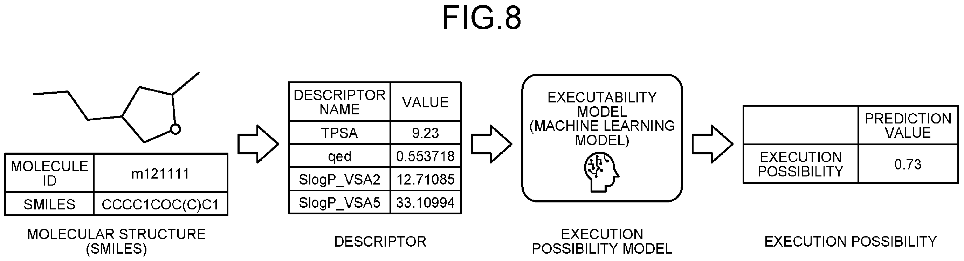

First, an example of prediction of execution possibility by the control device 20 using the execution possibility model 25 is explained with reference to FIG. 8. FIG. 8 is a diagram illustrating an example of evaluation of execution possibility according to the embodiment of the present disclosure.

As illustrated in FIG. 8, the control device 20 derives values corresponding to descriptors using a character string in which a molecule identified by a molecule ID is expressed by the SMILES notation. Then, the control device 20 inputs the values corresponding to the descriptors to the execution possibility model 25 to thereby output a prediction value of a physical property (hereinafter also referred to as "second prediction value") from the execution possibility model 25. For example, the execution possibility model 25 outputs the second prediction value according to input of a material feature value of each of two or more materials configuring materials. In FIG. 8, the execution possibility model 25 receives values corresponding to descriptors of "TPSA", "qed", "SlogP_VSA2", and "SlogP_VSA5" as input and outputs a value indicating the executability. For example, the execution possibility model 25 receives values corresponding to the descriptors as input and outputs a value indicating execution possibility between 0 to 1. The execution possibility model 25 outputs a value indicating that the execution possibility is higher as the value is closer to 1. For example, the control device 20 inputs a material feature value concerning a molecule (a material) to the execution possibility model 25 to thereby output a value indicating the execution possibility from the execution possibility model 25.

The control device 20 trains and updates the execution possibility model 25 according to a learning algorithm using the data stored in the executability database 23 as learning data. Note that the learning of the execution possibility model 25 by the control device 20 is so-called supervised learning and, although detailed explanation thereof is omitted, if the execution possibility model 25 can be learned, the control device 20 may learn the execution possibility model 25 with training processing by any learning algorithm.

[1-3. Update processing example of databases and models after material manufacturing processing]

Next, an example of a search and an example of update processing for databases and models after material manufacturing processing are explained.

[1-3-1. Search using only a physical property prediction model and update of databases and models after material manufacturing processing]

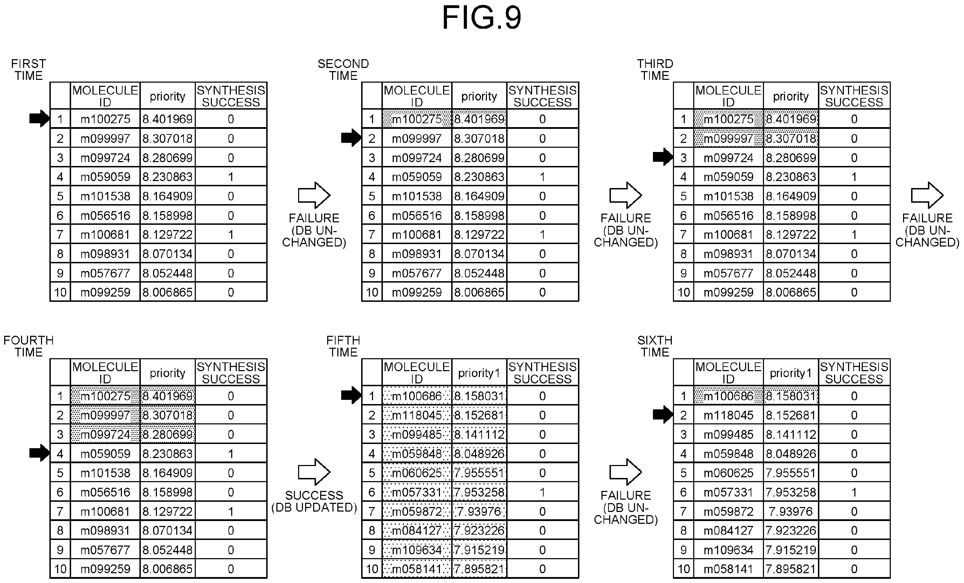

First, an example of processing using only the physical property prediction model 24 is explained with reference to FIG. 9. FIG. 9 is a diagram illustrating an example of a search using a physical property prediction model. The processing illustrated in FIG. 9 corresponds to the flow of the processing of the flowchart illustrated in FIG. 2 except processing concerning the executability database 23.

Each of six tables (hereinafter also referred to as "search tables #1") in FIG. 9 is list information of materials used for selection of materials and corresponds to a state at a processing start point in time in each of first to sixth times of processing (the processing illustrated in FIG. 2) targeting molecules. The six search tables #1 illustrated in FIG. 9 indicate update of the search tables #1 after the first processing to the sixth processing (the processing illustrated in FIG. 2). For example, a table arranged in the upper left part in FIG. 9 corresponds to the search table #1 in the first time. For example, a table arranged at the upper center in FIG. 9 corresponds to the search table #1 in the second time, that is, the search table #1 after the first processing.

The search tables #1 include items such as "molecule ID", "priority", and "synthesis success". The "molecule ID" indicates identification information for identifying a molecule. The "priority" is a value indicating priority. For example, the "priority" is a value (a score) output by the physical property prediction model 24 in response to input of information concerning the molecule corresponding thereto. For example, the "priority" is a first prediction value of a material based on material physical property information. The first prediction value is output from the physical property prediction model 24 according to, for example, input based on a material feature value of each of two or more materials configuring materials registered in the physical property database 22. In the search tables #1, priority is allocated in order from a first place in descending order of values of the "priority".

Note that a numerical value ("1" or the like) arranged at the end of the "priority" indicates the number of times of update. For example, the "priority" having no numerical value at the end indicates the "priority" before update, that is, at a processing start point in time. "Priority1" indicates "priority" at a point in time when the update after the first processing was performed. As explained above, a number after the "priority" is for convenience of indicating the number of times of update. The "priority" and the "priority" having the numerical value at the end are the same item "priority".

The "synthesis success" indicates whether synthesis will be successful. Note that FIG. 9 illustrates a state in which a value is stored in the "synthesis success" in order to show where the synthesis will be successful. However, the value of the "synthesis success" of the molecule before synthesis processing may be unknown. Note that the search tables #1 may include an item indicating success or failure of measurement processing such as "measurement".

In the first processing, the material searching and manufacturing system 1 performs synthesis processing targeting a molecule identified by a molecule ID "m 100275" having the priority in the first place. In FIG. 9, the synthesis of the molecule identified by the molecule ID "m 100275" is unsuccessful. The material searching and manufacturing system 1 does not update data. Note that, in FIG. 9, "success (DB update)" or "failure (DB unchanged)" is described together with an outlined arrow between the searches. In this embodiment, DB refers to the physical property database 22. In common in FIG. 9 to FIG. 15, "success" means that the "success" corresponds to successful synthesis and successful measurement ("YES" in S7 of FIG. 2) and "failure" means that "failure" corresponds to failure of synthesis ("NO" in S5 in FIG. 2) or failure of measurement ("NO" in S7 in FIG. 2). That is, in the physical property database 22, when the synthesis and the measurement are successful, the data is updated (DB update) regardless of whether the target value is achieved thereafter (S8 in FIG. 2) but, otherwise, the data is not updated and is unchanged (DB unchanged). Note that, in FIG. 9, the measurement is not referred to but, when the synthesis has been successful, it is assumed that the measurement is also always successful.

In the processing in the second time and the third time as well, the material searching and manufacturing system 1 fails in synthesizing molecules in the second place and the third place in the priority. The material searching and manufacturing system 1 does not update the data.

In the fourth processing, the material searching and manufacturing system 1 performs synthesis processing or the like targeting a molecule identified by a molecule ID "m059059" in the fourth place in the priority. In FIG. 9, it is assumed that the molecule identified by the molecule ID "m059059" has been successfully synthesized and measured in the fourth processing. In this case, the material searching and manufacturing system 1 updates data concerning a physical property value of the molecule ID "m059059" in the fourth place in the priority in the physical property database 22.

The material searching and manufacturing system 1 further relearns (updates) the physical property prediction model 24 using the physical property database 22 after the update. Then, the material searching and manufacturing system 1 updates the value of "priority" using the physical property prediction model 24 after the update. The "priority1" after a table (the search table #1 in the fifth time) arranged in the lower center in FIG. 9 corresponds to a value of priority derived using the physical property prediction model 24 after the update.

Then, the material searching and manufacturing system 1 performs fifth and sixth processing in order from the first place in the priority based on the value of the "priority1". For example, the material searching and manufacturing system 1 repeats the processing explained above until a molecule satisfying a desired physical property value is synthesized.

[1-3-2. Search using the physical property prediction model and the execution possibility model and update of databases and models after material manufacturing processing]

Next, an example of processing using the physical property prediction model 24 and the execution possibility model 25 is explained with reference to FIG. 10. Processing illustrated in FIG. 10 corresponds to the flow of the processing of the flowchart illustrated in FIG. 2. Note that, in FIG. 10, explanation is omitted as appropriate for the same points as the points illustrated in FIG. 9.

Each of six tables (hereinafter also referred to as "search tables #2") in FIG. 10 is list information of materials used for selection of materials and corresponds to a state at a processing start point in time in each of first to sixth times of the processing (the processing illustrated in FIG. 2) targeting a molecule. The six search tables #2 in FIG. 10 indicate update of the search tables #2 after the first to sixth processing (the processing illustrated in FIG. 2). For example, a table arranged in the upper left part in FIG. 10 corresponds to the search table #2 in the first time. For example, a table arranged in the upper center in FIG. 10 corresponds to the search table #2 in the second time, that is, the search table #2 after the first processing. The search tables #2 illustrated in FIG. 10 illustrate an example of a synthetic material selection data structure used for processing for selecting materials.

The search tables #2 include items such as "molecule ID", "priority", "feasibility", and "success". The "molecule ID" and the "priority" are the same as those illustrated in FIG. 9. The "feasibility" is a value indicating execution possibility. For example, the "feasibility" is a value (a score) output by the execution possibility model 25 in response to input of information concerning a molecule corresponding the "feasibility". For example, the "feasibility" is a second prediction value of a material based on execution possibility information. The second prediction value is output from the execution possibility model 25 according to, for example, input based on a material feature value of each of two or more materials configuring materials registered in the executability database 23. For example, the material searching and manufacturing system 1 sets, as a processing target, a molecule having a value of the "feasibility" equal to or larger than a predetermined threshold (for example, 0.5).

Note that a numerical value ("1" or the like) arranged at the end of the "feasibility" indicates the number of times of update. For example, the "feasibility" having no numerical value at the end indicates the "feasibility" before the update, that is, at a processing start point in time. "Feasibility1" indicates the "feasibility" at a point in time when the update is performed once. As explained above, a number after the "feasibility" is for convenience of indicating the number of times of update. The "feasibility" and the "feasibility" having the numerical value at the end are the same item "feasibility".

The "success" as the item in the search table indicates whether synthesis and measurement will be successful. Note that FIG. 10 illustrates a state in which a value is stored in the "success" to indicate where the synthesis and the measurement will be successful. However, the value of the "success" of the molecule before processing may be unknown. In addition, in FIG. 10, the "success" or the "failure" is described together with an outlined arrow between searches. Here, as explained above, in common in FIG. 9 to FIG. 15, the "success" means that the "success" corresponds to success of the synthesis and success of the measurement ("YES" in S7 in FIG. 2) and the "failure" means that the "failure" corresponds to failure in the synthesis ("NO" in S5 in FIG. 2) or failure in the measurement ("NO" in S7 in FIG. 2). In the physical property database 22, when the synthesis and the measurement are successful, data is updated (physical property DB: updated) regardless of whether the target value is achieved thereafter (S8 in FIG. 2). However, otherwise, the data is not updated and is unchanged (physical property DB: unchanged). On the other hand, in the executability database 23, the data is updated regardless of the "success" or the "failure" (executability DB: update). Note that, in FIG. 9, the measurement is not referred to but, when the synthesis has been successful, it is assumed that the measurement is also always successful.

Since values output by the execution possibility model 25, that is, values of the "feasibility" meaning execution possibility is smaller than the threshold (0.5) in molecules in the first place to the third place in the priority, the material searching and manufacturing system 1 does not set these molecules as processing targets and skips the subsequent processing. As explained above, since the material searching and manufacturing system 1 does not perform processing targeting unnecessary molecules using the information of the "feasibility" concerning the execution possibility, processing efficiency can be improved.

Then, the material searching and manufacturing system 1 performs synthesis processing and the like targeting the molecule identified by the molecule ID "m059059" that is in the fourth place in the priority in the first processing and has a value of the "execution possibility" equal to or larger than the threshold (0.5). In FIG. 10, it is assumed that the molecule identified by the molecule ID "m059059" has been successfully synthesized and measured in the first processing. Therefore, thereafter, the material searching and manufacturing system 1 updates the data concerning the physical property value of the molecule ID "m059059" in the physical property database 22, and adds (or updates) the data concerning the executability of the molecule ID "m059059" in the execution possibility model 25.

The material searching and manufacturing system 1 further relearns (updates) the physical property prediction model 24 using the physical property database 22 after the update. Then, the material searching and manufacturing system 1 updates the value of "priority" using the physical property prediction model 24 after the update. The "priority1" after the table (the second search table #2) arranged in the upper center in FIG. 10 corresponds to a value of the priority derived using the physical property prediction model 24 after the update.

The material searching and manufacturing system 1 relearns (updates) the execution possibility model 25 using the executability database 23 after the update. The material searching and manufacturing system 1 updates the value of the "feasibility" using the execution possibility model 25 after the update. The "feasibility1" after the table (the search table #2 in the second time) arranged in the upper center in FIG. 10 corresponds to a value of the feasibility derived using the execution possibility model 25 after the update.

Then, in the second processing, the material searching and manufacturing system 1 performs synthesis processing or the like targeting a molecule identified by a molecule ID "m 100686" that is in the first place in the priority in the search table #2 after the update and has a value of the "feasibility" equal to or larger than the threshold (0.5). In FIG. 10, it is assumed that synthesis of the molecule identified by the molecule ID "m 100686" has been unsuccessful ("NO" in S7 in FIG. 2). In this case, the material searching and manufacturing system 1 adds (or updates) data concerning executability of the molecule ID "m 100686" in the executability database 23.

For example, the material searching and manufacturing system 1 adds (or updates) information concerning the executability of the molecule identified by the molecule ID "m 100686" to the executability database 23 as information concerning the molecule for which synthesis has been unsuccessful to thereby update the executability database 23 again and relearn (update) the execution possibility model 25 using the executability database 23 after the update. The material searching and manufacturing system 1 updates the value of the "feasibility" using the execution possibility model 25 after the update. "feasibility2" after the table (the search table #2 in the third time) arranged in the upper right part in FIG. 10 corresponds to a value of feasibility derived using the execution possibility model 25 after the second update.

Then, in the third processing, the material searching and manufacturing system 1 performs processing based on the values of the "priority1" and the "feasibility2". The material searching and manufacturing system 1 updates the values of the "priority" and the "feasibility" and performs fourth processing to sixth processing using the updated values. However, since the processing is the same as the processing explained above, detailed explanation of the processing is omitted. For example, the material searching and manufacturing system 1 repeats the processing explained above until a molecule satisfying a desired physical property value is synthesized. As explained above, the material searching and manufacturing system 1 designates materials in a list format including at least one item and selects the materials in the order of the priority. As explained above, the data structure of the search table #2 illustrated in FIG. 10 is a data structure used in the material searching and manufacturing system 1 including the control device 20 including the storage unit 201 and the processing unit 202, stored in the storage unit 201 of the control device 20, and used to select the synthesis target materials in the processing unit 202 of the control device 20. For example, the data structure of the search table #2 illustrated in FIG. 10 includes an ID for identifying a material, priority information indicating priority based on a physical property value of a predicted material, execution possibility information indicating execution possibility of synthesis of the material, and synthesis success/failure information indicating success/failure of synthesis processing. The processing unit 202 of the control device 20 executes processing for searching for materials in descending order of priority based on priority information targeting a material group, the synthesis success/failure information of which indicates unprocessed, and selecting materials, success/failure of the synthesis processing of which is indicated by the synthesis success/failure information satisfies a predetermined standard.

[1-4. Details of the search and the update processing for databases and models after the material manufacturing processing]

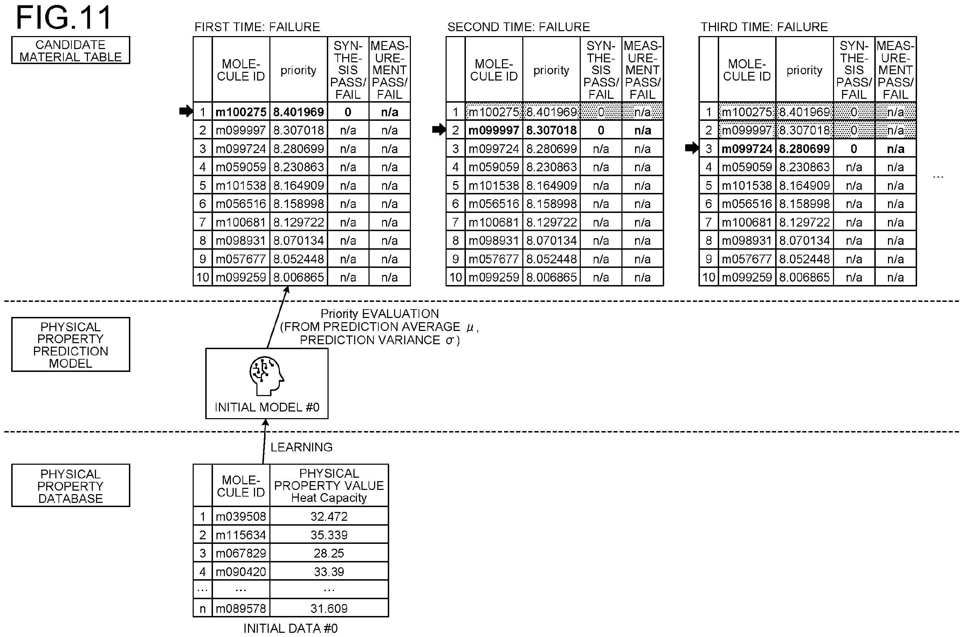

Here, details of the processing illustrated in FIG. 9 and FIG. 10 explained above are explained with reference to FIG. 11 to FIG. 15 illustrating the details of the processing.

[1-4-1. Search using only a physical property prediction model and update of databases and models after material manufacturing processing]

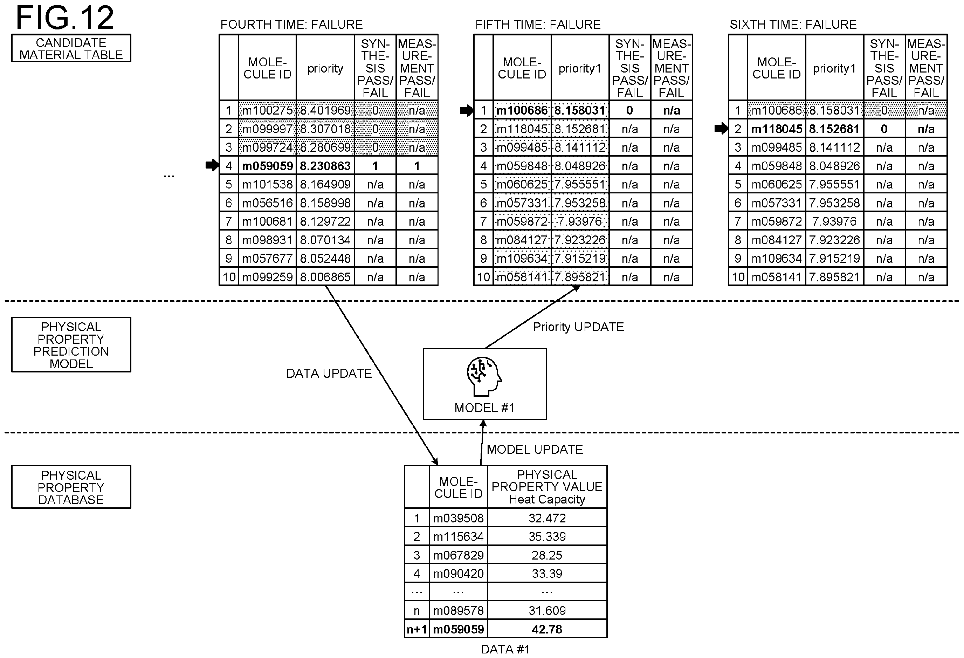

First, details of the processing illustrated in FIG. 9 are explained with reference to FIG. 11 and FIG. 12. FIG. 11 and FIG. 12 are diagrams illustrating detailed examples of processing using the physical property prediction model 24. Specifically, FIG. 11 illustrates details of the first processing to the third processing illustrated in FIG. 9 and FIG. 12 illustrates details of the fourth processing to the sixth processing illustrated in FIG. 9. Note that detailed explanation is omitted for the same points as the points illustrated in FIG. 9. As explained above, "success" means that the "success" corresponds to success in the synthesis and success in the measurement ("YES" in S7 in FIG. 2) and "failure" means that the "failure" corresponds to failure in the synthesis ("NO" in S5 in FIG. 2) or failure in the measurement ("NO" in S7 in FIG. 2).

Six tables illustrated in FIG. 11 and FIG. 12 (hereinafter also referred to as "candidate material tables #1") correspond to the search tables #1 in FIG. 9 but are different from the search tables #1 in that the tables include items of "synthesis pass/fail" and "measurement pass/fail" instead of the item of "synthesis success". The items of the "synthesis pass/fail" and the "measurement pass/fail" in the candidate material table #1 store results of actual processing. Therefore, in the search table #1, the items of the "synthesis pass/fail" and "measurement pass/fail" are "n/a" (values are undecided) for the molecules that are not processed. The material searching and manufacturing system 1 updates, for a processing target and a low molecule the items of the "synthesis pass/fail" and the "measurement pass/fail" in the candidate material table #1 according to processing results of the processing target and the low molecule.

As illustrated in FIG. 11, the material searching and manufacturing system 1 trains an initial model #0 (the physical property prediction model 24) according to a learning algorithm using initial data #0 (data of the physical property database 22 before the update). The material searching and manufacturing system 1 evaluates (derives) priority of molecules using the initial model #0 and performs processing using a value of the priority. Here, various parameters can be used in the evaluation (the derivation) of the priority. However, in order to take into account executability, it is also possible to prioritize molecules in which both of the items of the "synthesis pass/fail" and the "measurement pass/fail" are "n/a" (values are undecided). Consequently, possibility of a search for a new material can be increased considering the executability.

In the first processing, the material searching and manufacturing system 1 performs processing such as synthesis targeting the molecule identified by the molecule ID "m 100275" that is in the first place in the priority. Since the material searching and manufacturing system 1 has failed in the synthesis of the molecule identified by the molecule ID "m 100275", the material searching and manufacturing system 1 changes the synthesis pass/fail of the molecule identified by the molecule ID "m 100275" to "0". Since the material searching and manufacturing system 1 fails in the synthesis of the molecules in the second place and the third place in the priority in the second processing and the third processing, the material searching and manufacturing system 1 changes the synthesis pass/fail of the molecules in the second place and the third place in the priority to "0".

As illustrated in FIG. 12, the material searching and manufacturing system 1 performs synthesis processing targeting the molecule identified by the molecule ID "m059059" that is in the fourth place in the priority in the fourth processing. It is assumed that the material searching and manufacturing system 1 has succeeded in synthesis and measurement of the molecule identified by the molecule ID "m059059". In this case, the material searching and manufacturing system 1 changes the synthesis pass/fail and the measurement pass/fail of the molecule identified by the molecule ID "m059059" to "1" in the candidate material table.

The material searching and manufacturing system 1 updates the physical property value information of the molecule identified by the molecule ID "m059059" in the physical property database 22. Further, the material searching and manufacturing system 1 relearns (updates) the model #0 (the physical property prediction model 24) using the data #1 (the data of the physical property database 22 after the update). Then, the material searching and manufacturing system 1 updates the value of the priority using the physical property prediction model 24 after the update.

Then, the material searching and manufacturing system 1 performs fifth and sixth processing in order from the first place in the priority based on the value of the "priority1". For example, the material searching and manufacturing system 1 repeats the processing explained above until a molecule satisfying a desired physical property value is synthesized.

[1-4-2. Search using the physical property prediction model and the execution possibility model and update of databases and models after the material manufacturing processing]

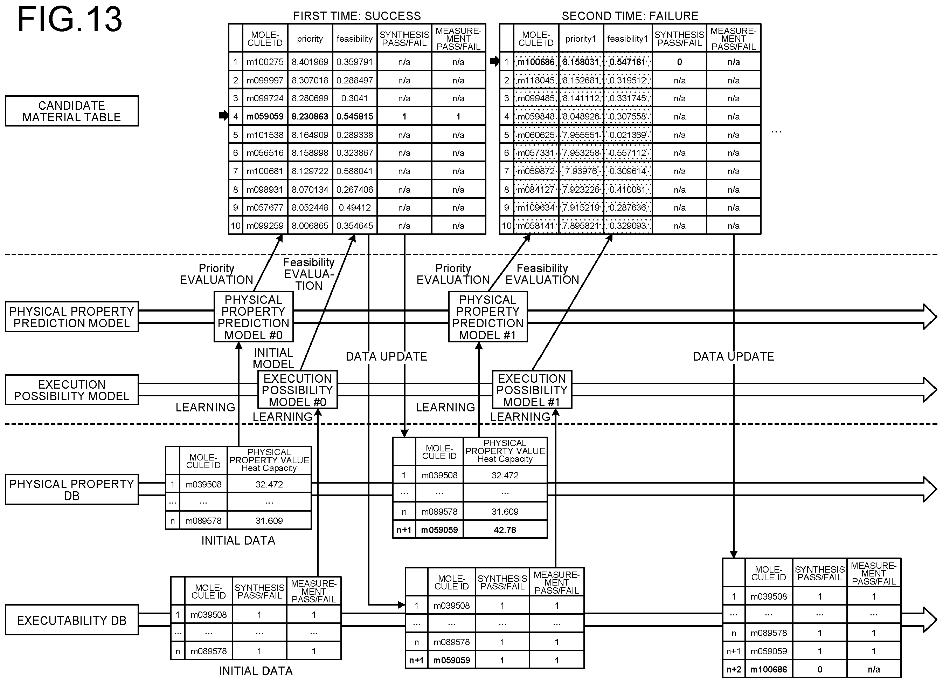

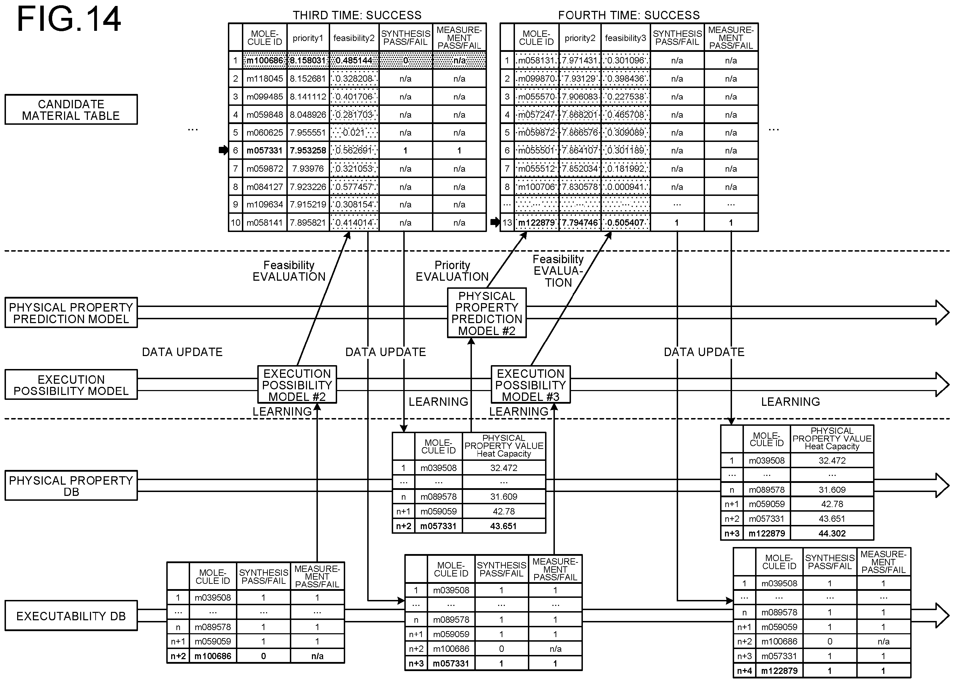

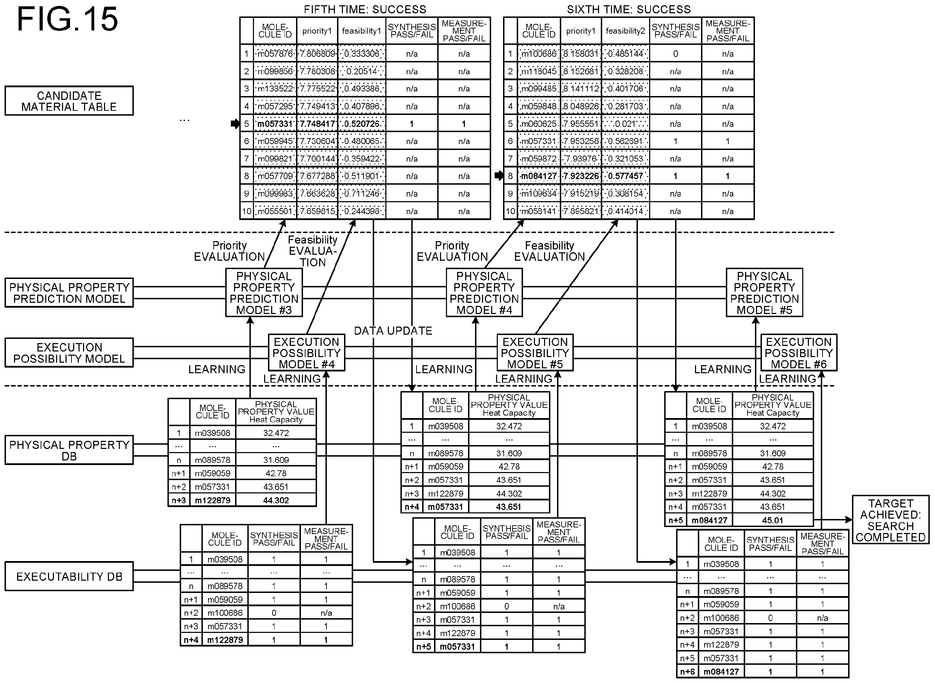

Next, details of the processing illustrated in FIG. 10 are explained with reference to FIG. 13 to FIG. 15. FIG. 13 to FIG. 15 are diagrams illustrating detailed examples of processing using the physical property prediction model 24 and the execution possibility model 25. For example, FIG. 13 to FIG. 15 illustrate an example of a manufacturing method for manufacturing the physical property prediction model 24 and the execution possibility model 25, which are machine learning models, based on a synthesis processing result. Specifically, FIG. 13 illustrates details of the first processing to the second processing illustrated in FIG. 10, FIG. 14 illustrates details of the third processing to the fourth processing illustrated in FIG. 10, and FIG. 15 illustrates details of the fifth processing to the sixth processing illustrated in FIG. 10. Note that detailed explanation is omitted about the same points as the points illustrated in FIG. 10 to FIG. 12. As explained above, "success" means that the "success" corresponds to success in the synthesis and success in the measurement ("YES" in S7 in FIG. 2) and "failure" means that the "failure" corresponds to failure in the synthesis ("NO" in S5 in FIG. 2) or failure in the measurement ("NO" in S7 in FIG. 2).

Six tables illustrated in FIG. 13 to FIG. 15 (hereinafter also referred to as "candidate material tables #2") correspond to the search tables #2 in FIG. 10 but are different from the search tables #2 in that the tables include items of "synthesis pass/fail" and "measurement pass/fail" instead of the item of "success". In the items of "synthesis pass/fail" and "measurement pass/fail" of the candidate material table #2, results of actual processing are stored. However, since this point is the same as the points illustrated in FIG. 11 and FIG. 12, explanation of the points is omitted.

As illustrated in FIG. 13, the material searching and manufacturing system 1 trains and updates the physical property prediction model #0 (the physical property prediction model 24) according to a learning algorithm using initial data (data of the physical property database 22 before update). The material searching and manufacturing system 1 evaluates (derives) priority of molecules using the physical property prediction model #0 after the update and performs processing using a value of the priority.

As illustrated in FIG. 13, the material searching and manufacturing system 1 trains and updates the execution possibility model #0 (the execution possibility model 25) according to a learning algorithm using initial data (data of the executability database 23 before update). The material searching and manufacturing system 1 evaluates (derives) feasibility of the molecules using the execution possibility model #0 after the update and performs processing using a value of the feasibility. Here, in the evaluation (the derivation) of the feasibility, various parameters can be used. However, it is also possible to prioritize those in which both items of "synthesis pass/fail" and "measurement pass/fail" are "n/a" (values are undecided). Consequently, possibility of a search for a new material can be increased considering the executability. In the example illustrated in FIG. 11, the items of the "synthesis pass/fail" and the "measurement pass/fail" are considered in order to consider executability in the evaluation (the derivation) of the priority. However, in FIG. 13, since the priority and the feasibility are separated, the items may not be considered in the evaluation (the derivation) of the priority.

Since values of the "feasibility" of molecules in the first place to the third place in the priority is smaller than the threshold (0.5), the material searching and manufacturing system 1 does not set these molecules as processing targets and skips the processing. Then, the material searching and manufacturing system 1 performs processing such as synthesis targeting the molecule identified by the molecule ID "m059059" that is in the fourth place in the priority in the first processing and has a value of the "feasibility" equal to or larger than the threshold (0.5). In FIG. 10, it is assumed that the molecule identified by the molecule ID "m059059" has been successfully synthesized and measured. In this case, the material searching and manufacturing system 1 changes the synthesis pass/fail and the measurement pass/fail of the molecule identified by the molecule ID "m059059" to "1".

The material searching and manufacturing system 1 updates the physical property value information of the molecule identified by the molecule ID "m059059" in the physical property database 22. Further, the material searching and manufacturing system 1 relearns (updates) the physical property prediction model #1 (the physical property prediction model 24) using the data of the physical property database 22 after the update. Then, the material searching and manufacturing system 1 updates the value of the priority using the physical property prediction model 24 after the update.

The material searching and manufacturing system 1 adds (or updates) the information concerning the molecule identified by the molecule ID "m059059" to the executability database 23 as information of the molecule that has been successfully synthesized to thereby update the executability database 23. The material searching and manufacturing system 1 relearns (updates) the execution possibility model #1 (the execution possibility model 25) using the executability database 23 after the update. Then, the material searching and manufacturing system 1 updates the value of "feasibility" using the execution possibility model #1 after the update.

Then, in the second processing, the material searching and manufacturing system 1 performs synthesis processing targeting the molecule identified by the molecule ID "m 100686" that is in the first place in the priority in the search table #2 after the update and has the value of the "feasibility" equal to or larger than the threshold (0.5). In FIG. 10, it is assumed that synthesis of the molecule identified by the molecule ID "m 100686" has been unsuccessful. Therefore, the material searching and manufacturing system 1 changes the synthesis pass/fail of the molecule identified by the molecule ID "m 100686" to "0".

Then, the material searching and manufacturing system 1 updates data. For example, the material searching and manufacturing system 1 adds (or updates) information concerning the molecule identified by the molecule ID "m 100686" to the executability database 23 as information concerning the molecule for which synthesis has been unsuccessful to thereby update the executability database 23 again. As illustrated in FIG. 14, the material searching and manufacturing system 1 relearns (updates) the execution possibility model #2 (the execution possibility model 25) using the executability database 23 after the update. Then, the material searching and manufacturing system 1 updates the value of "feasibility" using the execution possibility model #2 after the update.

Then, in the third processing, the material searching and manufacturing system 1 performs processing based on the values of the "priority1" and the "feasibility2". As illustrated in FIG. 14 and FIG. 15, the material searching and manufacturing system 1 updates the values of the "priority" and the "feasibility" and performs the fourth processing to the sixth processing using the updated values. Since the processing is the same as the processing explained above, detailed explanation of the processing is omitted. However, the material searching and manufacturing system 1 repeats the processing explained above until a molecule satisfying a desired physical property value is synthesized. Specifically, in the sixth processing, an example is explained in which a molecule satisfying the desired physical property value is synthesized and the target value is achieved in S8 in FIG. 2. That is, the material searching and manufacturing system 1 changes the synthesis pass/fail and the measurement pass/fail of the molecule identified by the molecule ID "m084127" to "1", further updates the physical property value information of the molecule ID "m084127" in the physical property database 22, adds data of the molecule ID "m084127" in the executability database 23, and changes the synthesis pass/fail and the measurement pass/fail to "1". In this case, assuming that the destination has been achieved, the material searching and manufacturing system 1 ends the processing. In the manufacturing method illustrated in FIG. 13 to FIG. 15, the execution possibility model 25 that is a machine learning model is manufactured. In the manufacturing method illustrated in FIG. 13 to FIG. 15, for example, the control device 20 of the material searching and manufacturing system 1 performs material selection according to the execution possibility information output by the execution possibility model 25. In this case, the control device 20 instructs the material manufacturing device 10 to perform synthesis processing for the selected materials and updates the information of the executability database 23 (the execution possibility database) based on a synthesis processing result from the material manufacturing device 10. Then, the control device 20 updates the execution possibility model 25 based on the updated execution possibility information in the executability database 23. Consequently, in the manufacturing method illustrated in FIG. 13 to FIG. 15, the updated execution possibility model 25 is manufactured.

[1-5. Example and material manufacturing results]

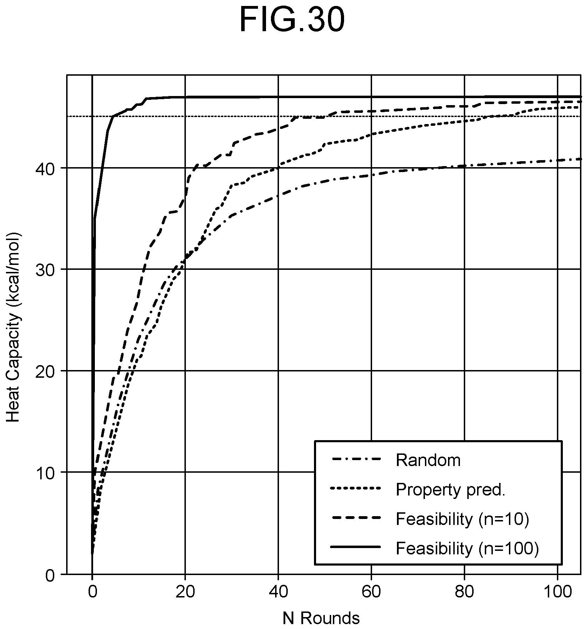

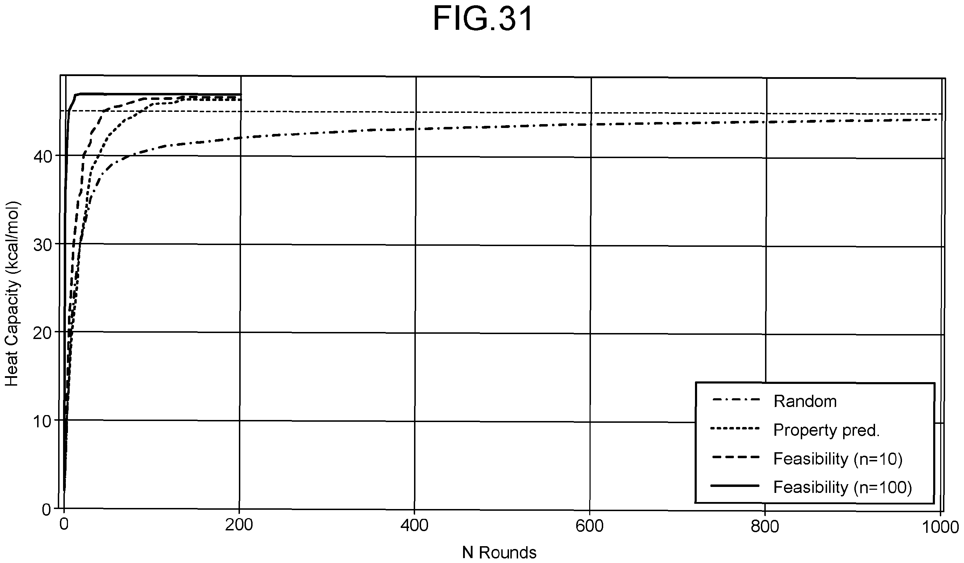

Here, an example in which a specific target data set is used is explained. A material manufacturing result of each of a plurality of methods including the example is explained with reference to FIG. 30 and FIG. 31. FIG. 30 and FIG. 31 are diagrams illustrating examples of material manufacturing results concerning a search.

In the example, a material search utilizing the execution possibility model 25 is performed. For example, in the example, 133,886 molecules included in a QM9 data set, which is a data set of organic molecular structural physical properties, were set as a range of a material search space and a material search aimed at optimizing physical property values was implemented.

In the example, the material manufacturing device 10 is implemented by software (a software material manufacturing device). The material manufacturing device 10 receives one molecular structure present in the QM9 data set as input from the control device 20 and returns synthesis/measurement succeeds/fails (True, False) to the control device 20 as an output #1 and returns a physical property value, which is a measurement result, to the control device 20 as an output #2 only when and synthesis/measurement has been successful. The success or failure of the synthesis/measurement was determined by providing a threshold for LogP (a calculated value), which is a function indicating dissolution behavior concerning a solvent. This is equivalent to reproducing, with simple calculation values, the fact in material manufacturing that synthesis/measurement are successful/unsuccessful according to a dissolution behavior regarding the solvent. As the physical property value as the measurement result, a physical property value calculated by first principle calculation of a single molecule included in the QM9 data set was used.

The control device 20 was also implemented as software (see 2 in Table 1). As the search algorithm used in the search agent 26, each of two types (a random search and a search using the physical property prediction model 24) of algorithms set as comparative examples and an algorithm corresponding to the synthetic material selection method of the present disclosure (a search by the physical property prediction model 24 and the execution possibility model 25) was implemented and performance was compared.

In the following explanation, the search algorithms (see 5 in Table 1) indicating material manufacturing results are explained with reference to Table 1. Table 1 shows various kinds of information concerning material manufacturing. First, a comparative example #1 and a comparative example #2 to be compared with the synthetic material selection method of the present disclosure are explained. The comparative example #1 corresponds to the random search and the comparative example #2 corresponds to the search by the physical property prediction model 24.

Table 1

| 1. Material manufacturing device | Implemented as software<br>· Input: Molecular structure<br>· Output #1: Success/failure [True, False] of synthesis/measurement (LogP calculation value)<br>· Output #2: (When succeeding in synthesis/measurement) a physical property value as a measurement result |
|---|---|
| 2. Control device | Implemented as software<br>· Physical property database: Retain a molecular structure and a physical property value.<br>· Physical property prediction model: Predict a physical property value from the molecular structure according to a machine learning model (Gaussian Process Regression). Express the molecular structure as a vector with a molecular descriptor.<br>· Executability database: Retain the molecular structure and success or failure of synthesis/measurement<br>· Execution possibility model: Predict success or failure of synthesis/measurement from the molecular structure according to a machine learning model (Gaussian Process Classification). Express the molecular structure as a vector with the molecular descriptor.<br>· Control interface: Function call.<br>· Search agent: Based on a search algorithm, exchange an instruction and an output result with a material manufacturing device through the control interface, store the output result in the physical property and executability databases, and update the physical property prediction and execution possibility models. |
| 3. Range of a material search space | Molecules (133,886 molecules) included in a QM9 dataset |
| 4. Physical property value and target value | Physical property value: Heat Capacity included in the QM9 dataset<br>Target value: 45.0 (kcal/mol) *A maximum value is 46.4 (kcal/mol) |
| 5. Search algorithm | · [Comparative example #1] Random search. Perform material manufacturing trial at random for a molecular structure included in QM9.<br>· [Comparative example #2] Perform material manufacturing trail based on priority of the molecular structure by the physical property prediction model.<br>· [Example #1] Perform material manufacturing trial based on priority of the molecular structure by the physical property prediction and execution possibility models (the number of initial data: 100)<br>· [Example #2] Perform material manufacturing trial based on priority of the molecular structure by the physical property prediction and execution possibility models (the number of initial data: 10) |
| 6. Evaluation indicator | · The number of times of material manufacturing trial (the number of times of call for the material manufacturing device) before an optimum physical property value obtained as a material manufacturing result reaches a target value. Execute the material manufacturing trial 100 or 1000 times and compare average values. |

(Comparative example #1: the random search) <Procedure #1>

Procedure #1-1: Select one molecule at random from unevaluated molecules included in a material space and perform synthesis/measurement. The molecule is set as evaluated.

Procedure #1-2: Synthesis/measurement

(A): When the synthesis/measurement have been unsuccessful: repeat from the procedure 1.

(B): When synthesis/measurement have been successful: a molecular structure and a measurement result (a physical property value) are stored in the physical property database 22.

Procedure #1-3: Complete the random search when a maximum value (a minimum value) of a measured value in the physical property database 22 has reached a target value. If not, repeat the random search from the procedure #1-1.

(Comparative example #2: the search by the physical property prediction model)

In the search by the physical property prediction model 24 (the comparative example #2), an algorithm called Bayesian optimization using Gaussian Process regression that can calculate a predicted mean value ($\mu$) and a predicted variance ($\sigma$) was implemented as a machine learning model. A molecular descriptor (for example, an RdKit descriptor) was used as an input of the machine learning model. A search procedure is as explained in the following procedure #2.

<Procedure #2>

Procedure #2-1: Construct a physical property prediction model 24 (an initial model) that gives equal output for all inputs.
Procedure #2-2: Using the physical property prediction model 24, calculate, for the unevaluated molecules included in the material space, an acquisition function (Fa) indicated by the following definition:

(A): In the case of a maximization problem, calculate the acquisition function Fa using Expression (9).
(B): In the case of a minimization problem, calculate the acquisition function Fa using Expression (10).

$$Fa = \mu + C * \sigma \qquad \cdots (9)$$

$$Fa = \mu - C * \sigma \qquad \cdots (10)$$

Note that C in Expression (9) and Expression (10) is a constant (1 to 10 or the like).

Procedure #2-3: Determine priority of the unevaluated molecules included in the material space based on the acquisition function Fa.
Procedure #2-4: Perform synthesis/measurement of a molecule having highest priority. This molecule is set as evaluated.
Procedure #2-5: Synthesis/measurement

(A): When synthesis/measurement has been unsuccessful: repeat the search from the procedure #2-4.
(B): When synthesis/measurement have been successful: store a molecular structure and a measurement result (a physical property value) in the physical property database 22 and update the physical property prediction model 24.

Procedure #2-6: Complete the search when a maximum value (a minimum value) of measurement values present in the physical property database 22 has reached a target value. If not, repeat the search from the procedure #2-2.

Next, an example #1 and an example #2 corresponding to the synthetic material selection method of the present disclosure are explained. Note that the same points as the points of the contents explained above are explained as appropriate.

(Example #1 and Example #2: A search by the physical property prediction model and the execution possibility model)

Gaussian Process regression was used as a machine learning model for the physical property prediction model 24 and Gaussian Process classification was used as a machine learning model for the execution possibility model 25. A molecular descriptor (for example, an RdKit descriptor) was used as input of the machine learning model. A search procedure is as explained in the following procedure #3. For example, an initial model of the execution possibility model 25 is constructed using evaluated data (successful synthesis data).

<Procedure #3>

Procedure #3-1: Construct the execution possibility model 25 (the initial model) using initial data (the number of data: 100 or 10).
Procedure #3-2: Construct the physical property prediction model 24 (the initial model) that gives equal output for all inputs.
Procedure #3-3: Using the physical property prediction model 24, calculate, for unevaluated molecules included in a material space, an acquisition function (Fa) indicated by the following definition:

(A): In the case of a maximization problem, calculate the acquisition function Fa using Expression (9).
(B): In the case of a minimization problem, calculate the acquisition function Fa using Expression (10).

Procedure #3-4: Determine priority of the unevaluated molecules included in the material space based on the acquisition function Fa.
Procedure #3-5: Evaluate execution possibility of the unevaluated molecules included in the material space (prediction of success or failure of synthesis/measurement) with the execution possibility model 25.
Procedure #3-6: Perform synthesis/measurement of a molecule having high execution possibility (a probability of 0.5 or more or the like) and highest priority. This molecule is set as evaluated.
Procedure #3-7: Synthesis/measurement

(A): When synthesis/measurement have been unsuccessful: update the executability database 23 and the execution possibility model 25 and repeat the search from the procedure #3-5.
(B): When synthesis/measurement have been successful: update the executability database 23 and the execution possibility model 25 and update the physical property database 22 and the physical property prediction model 24 from a molecular structure and a measurement result (a physical property value).

Procedure #3-8: Complete the search when a maximum value (a minimum value) of measurement values retained in the physical property database 22 has reached the target value. If not, repeat the search from the procedure #3-2.

Here, an evaluation index (see 6 in Table 1) is the number of times of trial of material manufacturing until the target value is reached. Trial was performed for 1,000 times for the comparative example #1 and 100 times for the comparative example #2, the example #1, and the example #2. Average values were compared (see Table 1 for detailed setting).

As explained above, in the example #1 and the example #2, initial data used to construct the initial model of the execution possibility model 25 was given (the number of data: 100 or 10). This is equivalent to a case in which, as prior knowledge concerning the material manufacturing device 10, a result as to whether synthesis/measurement are successful is known concerning 100 or 10 molecular structures included in the initial data. For example, in the example #1 corresponding to a case in which the number of data is 100, the initial model of the execution possibility model 25 is constructed using teacher data for 100 molecular structures. For example, in the example #2 corresponding to a case in which the number of data is 10, an initial model of the execution possibility model 25 is constructed using teacher data for 10 molecular structures.

The material manufacturing results illustrated in FIG. 30 and FIG. 31 are explained below. FIG. 30 and FIG. 31 illustrate evaluation results concerning a molecular structure search for the purpose of optimizing (maximizing) Heat Capacity.

In the comparative example #1 (Random indicated by an alternate long and short dash line in FIG. 30 and FIG. 31), which is the random search, an average of 1,000 times or more of material manufacturing trials were required before the target value (45.0 kcal/mol or more) was reached. In the comparative example #2 (Property pred indicated by a dotted line in FIG. 30 and FIG. 31), which is the search using the physical property prediction model 24, an average of 85 times of material manufacturing trials were required before the target value (45.0 kcal/mol or more) was reached. For example, the target value (45.0 kcal/mol or more) is a condition to be compared in determining the target value achievement in Step S8 of FIG. 2.

On the other hand, in the example #1 (Feasibility (n = 100) indicated by a solid line in FIG. 30 and FIG. 31) and the example #2 (Feasibility (n = 10) indicated by a broken line in FIG. 30 and FIG. 31), which are searches using the physical property prediction model 24 and the execution possibility model 25, it was found that the target value can be reached by six times (one hundred initial data) to fifty-one times (ten initial data) in average of material manufacturing trials.

As explained above, in the synthetic material selection method of the present disclosure, it is possible to obtain an efficiency improvement effect of up to 100 times or more (cost of time and expenses is 1/100) with respect to the random search (the comparative example #1) and up to 13 times or more (cost of time and expenses is 1/13) with respect to the search by the physical property prediction model 24 (the comparative example #2). Therefore, in the synthetic material selection method and the material manufacturing method of the present disclosure, material synthesis can be efficiently performed based on executability of synthesis.

[1-6. Example of the executability database]

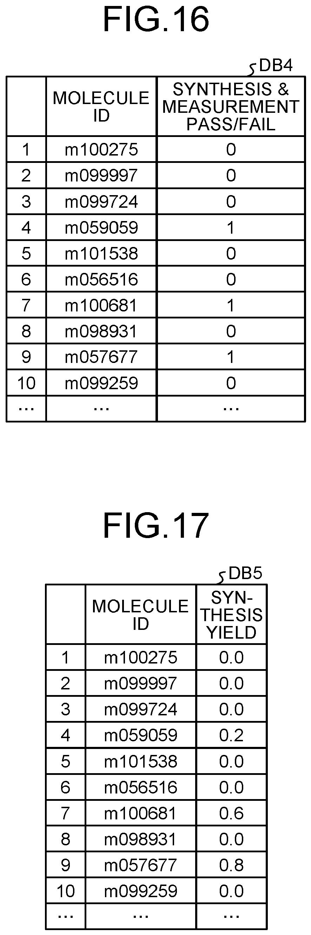

The executability database 23 explained above is merely an example and various data structures may be adopted as the executability database 23. This point is explained with reference to FIG. 16 to FIG. 21. FIG. 16 to FIG. 21 are diagrams illustrating examples of the executability database 23. Note that explanation is omitted as appropriate about the same points as the points of the executability database 23 explained above.

The executability database 23 may be a database DB4 in FIG. 16. In the example illustrated in FIG. 16, the database DB4 includes items such as "molecule ID" and "synthesis & measurement pass/fail". The "molecule ID" indicates identification information for identifying a molecule. The "synthesis & measurement pass/fail" indicates whether synthesis and measurement of a molecule have been successfully executed. For example, the "synthesis & measurement pass/fail" is "1" when both of the synthesis and the measurement of the molecule have been successfully executed and is "0" otherwise. Note that, as illustrated in FIG. 2, when a value of "synthesis & measurement pass/fail" is "1", the physical property prediction model 24 is also updated in addition to the execution possibility model 25. This is equivalent to the "success" in the examples illustrated in FIG. 9 to FIG. 15.

The executability database 23 may be a database DB5 in FIG. 17. In the example illustrated in FIG. 17, the database DB5 includes items such as "molecule ID" and "synthesis yield". The "molecule ID" indicates identification information for identifying a molecule. The "synthesis yield" indicates a yield in synthesis of the molecule.

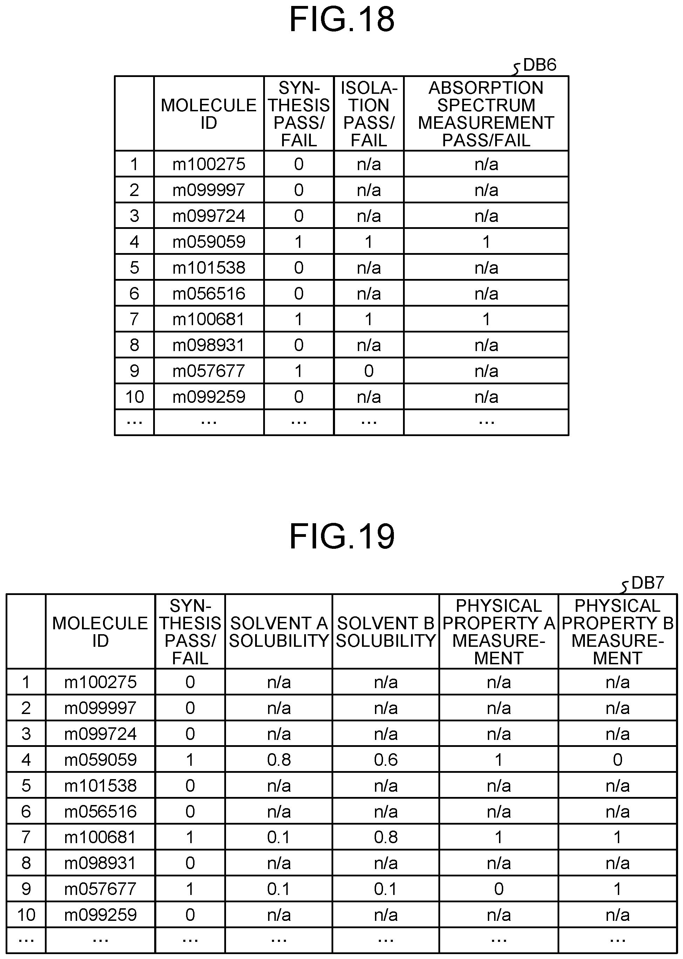

The executability database 23 may be a database DB6 in FIG. 18. In the example illustrated in FIG. 18, the database DB6 includes items such as "molecule ID", "synthesis pass/fail", "isolation pass/fail", and "absorption spectrum measurement pass/fail". The "molecule ID" indicates identification information for identifying a molecule. The "synthesis pass/fail" indicates whether synthesis of the molecule has been successfully executed. The "isolation pass/fail" indicates whether isolation of the molecule has been successfully executed. The "pass/fail of the absorption spectrum measurement" indicates whether the measurement by the absorption spectrum could be performed for the molecule. For example, each of the "synthesis pass/fail", the "isolation pass/fail", and the "absorption spectrum measurement pass/fail" is "1" when the processing has been successfully executed and is "0" otherwise. Note that the "absorption spectrum measurement pass/fail" is an example of the item "measurement pass/fail" illustrated in the examples illustrated in FIG. 13 to FIG. 15.

The executability database 23 may be a database DB7 in FIG. 19. In the example illustrated in FIG. 19, the database DB7 includes items such as "molecule ID", "solvent A solubility", "solvent B solubility", "physical property A measurement", and "physical property B measurement". The "molecule ID" indicates identification information for identifying a molecule. The "synthesis pass/fail" indicates whether synthesis of the molecule has been successfully executed. The "solvent A solubility" indicates a value concerning solubility of the molecule in a solvent A. The "solvent B solubility" indicates a value concerning solubility of the molecule in a solvent B. The "physical property A measurement" indicates whether measurement of a physical property A has been successfully executed. The "physical property B measurement" indicates whether measurement of a physical property B has been successfully executed. For example, each of the "synthesis pass/fail", the "physical property A measurement", and the "physical property B measurement" is "1" when the processing has been successfully executed and is "0" otherwise.

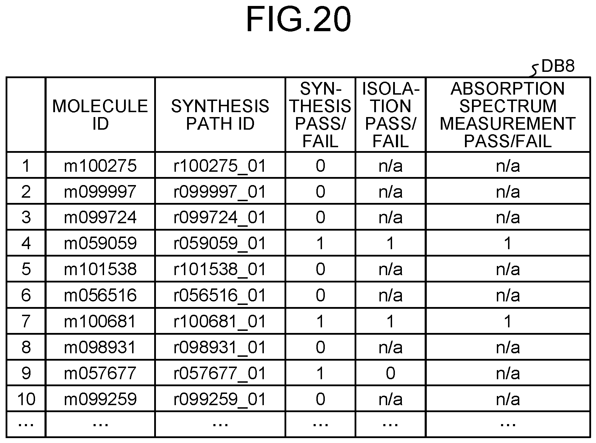

The executability database 23 may be a database DB8 in FIG. 20. In the example illustrated in FIG. 20, the database DB8 includes items such as "molecule ID", "synthesis path ID", "synthesis pass/fail", "isolation pass/fail", and "absorption spectrum measurement pass/fail". The "molecule ID" indicates identification information for identifying a molecule. The "synthesis path ID" indicates identification information for identifying a path (a route) of synthesis of the molecule. The "synthesis pass/fail" indicates whether synthesis of the molecule has been successfully executed. The "isolation pass/fail" indicates whether isolation of the molecule has been successfully executed. The "pass/fail of the absorption spectrum measurement" indicates whether the measurement by the absorption spectrum could be performed for the molecule.

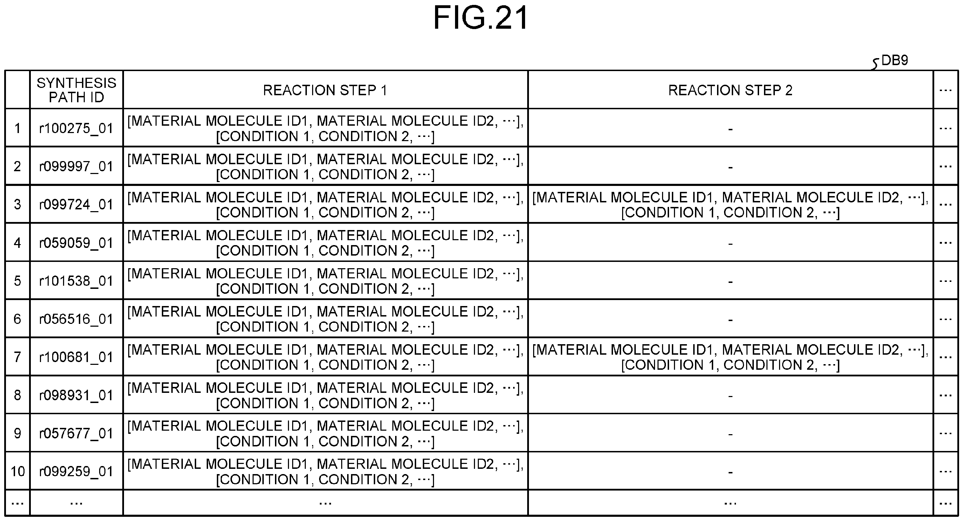

The executability database 23 may be a database DB9 in FIG. 21. In the example illustrated in FIG. 21, the database DB9 includes items such as "synthesis path ID", "reaction step 1", and "reaction step 2". The "synthesis path ID" indicates identification information for identifying a path (a route) of synthesis of the molecule. The "reaction step 1" indicates a first step in the synthesis path identified by the synthesis path ID. The "reaction step 2" indicates a second step in the synthesis path identified by the synthesis path ID. Note that, although only the "reaction step 1" and the "reaction step 2" are illustrated in FIG. 21, the database DB9 has items corresponding to the number of steps of the synthesis path such as "reaction step 3" and "reaction step 4".

[2. Another Processing Flow Example]

The material searching and manufacturing system 1 can execute any processing using the various kinds of information explained above. This point is explained below as an example different from the examples explained above. The material searching and manufacturing system 1 calculates priority based on information concerning physical properties (characteristics) and/or feasibility and creates a synthetic material (molecule) list (including at least one) according to the priority. Items of the list includes at least a molecule ID. Note that the list may include a pair of {molecule ID, synthetic path ID}.

[2-1. Processing concerning synthesis]

First, a processing example concerning synthesis is explained. For example, the material searching and manufacturing system 1 executes the following processing (1) to (8). Note that this synthesis processing is an example of a series of processing performed between the control interface 21, the synthesizing device 11, and the analyzing device 12 in FIG. 1 corresponding to the "synthesis" processing (Step S4) when the search agent performs the processing illustrated in FIG. 2.

(1) The analyzing device 12 transmits the material synthesis list to the synthesizing device 11 via the control interface 21 and instructs the synthesizing device 11 to perform synthesis according to the list.
(2) The synthesizing device 11 reads {molecule ID, (synthesis path ID)} according to priority of the list.
(3) When the synthesis path ID is designated, the synthesizing device 11 acquires a reaction step from the synthesis path ID table using a synthesis path ID as a key and performs synthesis processing.
(4) When conditions designated in the reaction step are satisfied, the synthesizing device 11 performs the synthesis processing. Otherwise, the analyzing device 12 notifies the synthesizing device 11 of information "synthesis is impossible".

(4-1) When receiving the notification of the "synthesis impossible", the synthesizing device 11 notifies the control interface 21 of synthesis failure for a first molecule ID (synthesis failure in FIG. 2) and proceeds to (6) .
(4-2) Otherwise, the synthesizing device 11 performs the processing of the reaction step 1. As a result of the processing, the synthesizing device 11 provides notification of a result of either "normal end" or "abnormal end"
(4-3) In the case of the "abnormal end", the synthesizing device 11 notifies the control interface 21 of synthesis failure for the first molecule ID (synthesis failure in FIG. 2) and proceeds to (6).
(4-4) In the case of the "normal end", the synthesizing device 11 checks whether there is the next reaction step and, when there is the next reaction step, acquires the next reaction step and repeats the processing from (4). When there is no next reaction step, the synthesizing device 11 proceeds to (5).

(5) When the synthesis processing is in the "normally termination", the synthesizing device 11 automatically provides a synthesized material to the analyzing device 12 in order to check whether the synthesis has been successful and performs control to instruct the analyzing device 12 to perform analysis to check whether the synthesized material has been successful in synthesizing a molecule ID.
(5-1) When the analyzing device 12 determines that the synthesis has been successful, the analyzing device 12 notifies the synthesizing device 11 of "synthesis success". Otherwise, the analyzing device 12 provides notification of "synthesis failure".
(6) The synthesizing device 11 notifies the control interface 21 of success or failure of the synthesis. The "success/failure notification" differs depending on the notification received in the process of the synthesis processing:

(6-1) In the case of synthesis impossible, abnormal end, or synthesis failure, the synthesizing device 11 transmits the "synthesis failure".
(6-2) In the case of synthesis success, the synthesizing device 11 transmits "synthesis success".

(7) When there is a material for which synthesis has not been attempted in the list, the synthesizing device 11 reads the next {molecule ID, (synthesis path ID)} and repeats the processing from (3).
(8) In parallel to the processing of the synthesizing device 11, in the case of the "synthesis failure", the search agent 26 determines that synthesis cannot be performed and constructs or updates the "execution possibility model".

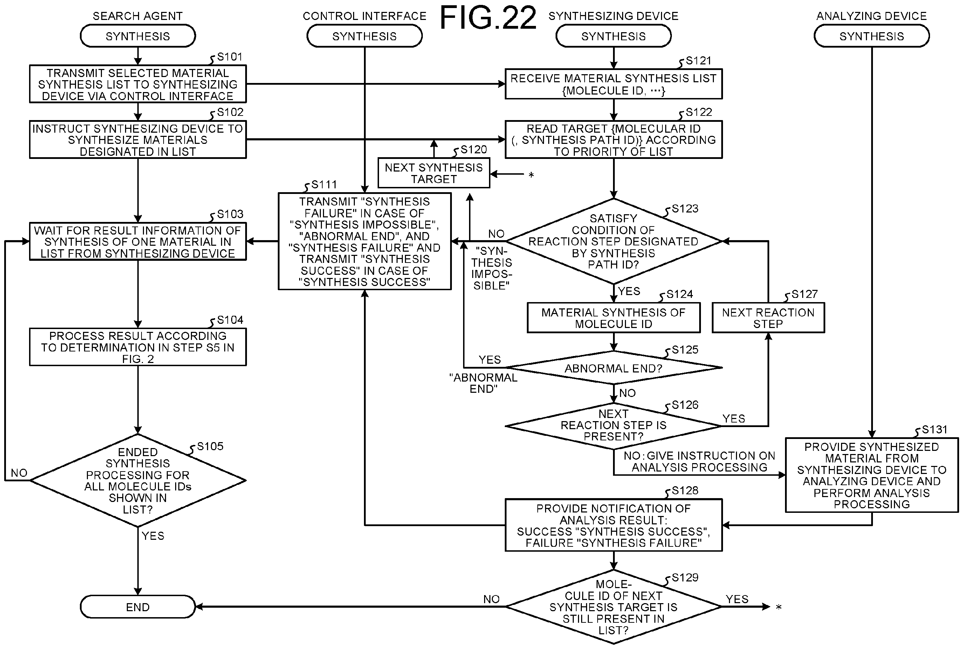

FIG. 22 illustrates a detailed flow of the processing (1) to (8) explained above. FIG. 22 is a sequence chart illustrating an example of a processing procedure in the material searching and manufacturing system. Note that step numbers illustrated in FIG. 22 are added for convenience in order to explain processing by processing subjects and do not specify the order of processing.

The search agent 26 transmits a selected material synthesis list to the synthesizing device 11 via the control interface 21 (Step S101). The search agent 26 instructs the synthesizing device 11 to synthesize a material specified in the list (Step S102). The search agent 26 waits for result information of synthesis of one material in the list from the synthesizing device 11 (Step S103). The search agent 26 processes the result according to the determination in Step S5 in FIG. 2 (Step S104).

When the synthesis processing has not been finished for all molecule IDs illustrated in the list (Step S105: No), the search agent 26 returns to Step S103 and repeats the processing. In addition, when the synthesis processing has been finished for all of the molecule IDs illustrated in the list (Step S105: Yes), the search agent 26 ends the processing.

When receiving the "synthesis impossible", the "abnormal end", and the "synthesis failure" from the synthesizing device 11, the control interface 21 transmits the "synthesis failure" to the search agent 26. When receiving the "synthesis success" from the synthesizing device 11, the control interface 21 transmits the "synthesis failure" to the search agent 26 (Step Sill).

The synthesizing device 11 receives a material synthesis list {molecule ID,...} (Step S121). The synthesizing device 11 reads target {molecule ID (, synthesis path ID)} according to the priority of the list (Step S122). When a condition of the reaction step designated by the synthesis path ID is not satisfied (Step S123: No), the synthesizing device 11 transmits the "synthesis impossible" to the control interface 21, selects the next synthesis target (Step S120), and returns to Step S122 and repeats the processing.

When the condition of the reaction step designated by the synthesis path ID is satisfied (Step S123: Yes), the synthesizing device 11 performs material synthesis of the molecule ID (Step S124). When Step S124 abnormally ends (Step S125: Yes), the synthesizing device 11 transmits the "abnormal end" to the control interface 21, selects the next synthesis target (Step S120), and returns to Step S122 and repeats the processing.

When Step S124 does not abnormally end (Step S125: No), the synthesizing device 11 determines whether there is the next reaction step (Step S126). If there is the next reaction step (Step S126: Yes), the synthesizing device 11 selects the next reaction step (Step S127) and returns to Step S123 and repeats the processing.

When there is no next reaction step (Step S126: No), the synthesizing device 11 gives an analysis processing instruction to the analyzing device 12. The synthesizing device 11 provides notification of an analysis result (Step S128). The synthesizing device 11 notifies the search agent 26 of the "synthesis success" in a case of success and notifies the search agent 26 of information corresponding to the "synthesis failure" in a case of failure via the control interface 21.

When there is still the next synthesis target molecule ID in the list (Step S129: Yes), the synthesizing device 11 returns to Step S120 and repeats the processing. When there is no next synthesis target molecule ID in the list (Step S129: No), the synthesizing device 11 ends the processing.

The analyzing device 12 receives provision of the synthesized material from the synthesizing device 11 and performs analysis processing (Step S131). Then, the analyzing device 12 transmits an analysis result to the synthesizing device 11.

[2-2. Processing concerning measurement]

Next, a processing example concerning measurement is explained. For example, the material searching and manufacturing system 1 executes the following processing (11) to (16). Note that this measurement processing is an example of a series of processing performed with the control interface 21, the synthesizing device 11, and the measuring device 13 illustrated in FIG. 1 according to the "measurement" processing (Step S6) when the search agent performs the processing illustrated in FIG. 2.

(11) The search agent 26 receives a molecule ID for which synthesis has been successful.

(12) The search agent 26 specifies the molecule ID for which synthesis has been successful, designates a physical property that should be measured, and instructs, through the control interface 21, the synthesizing device 11 to measure a material successfully synthesized.

(13) The synthesizing device 11 that has received the instruction automatically performs control to provide the synthetic material specified by the designated molecule ID to the measuring device 13 and instructs the measuring device 13 to start measurement of the physical properties specified for the provided material.

(14) The measuring device 13 notifies the synthesizing device 11 of the "measurement impossible" when the measurement cannot be performed and notifies the synthesizing device 11 of the "measurement end" and a measurement result when the measurement ends normally. The synthesizing device 11 that has received the notification transmits the measurement result via the control interface 21.

(15) When the measurement result is the "measurement impossible", the search agent 26 determines that synthesis cannot be performed and constructs or updates the "execution possibility model".

(16) The search agent 26 further determines whether the target value has been achieved and, when the target value has been achieved, determines that the synthesis has been successful, and constructs or updates the "physical property prediction model". When the target value has not been successfully achieved, the search agent 26 determines that the synthesis cannot be performed and constructs or updates the "execution possibility model".

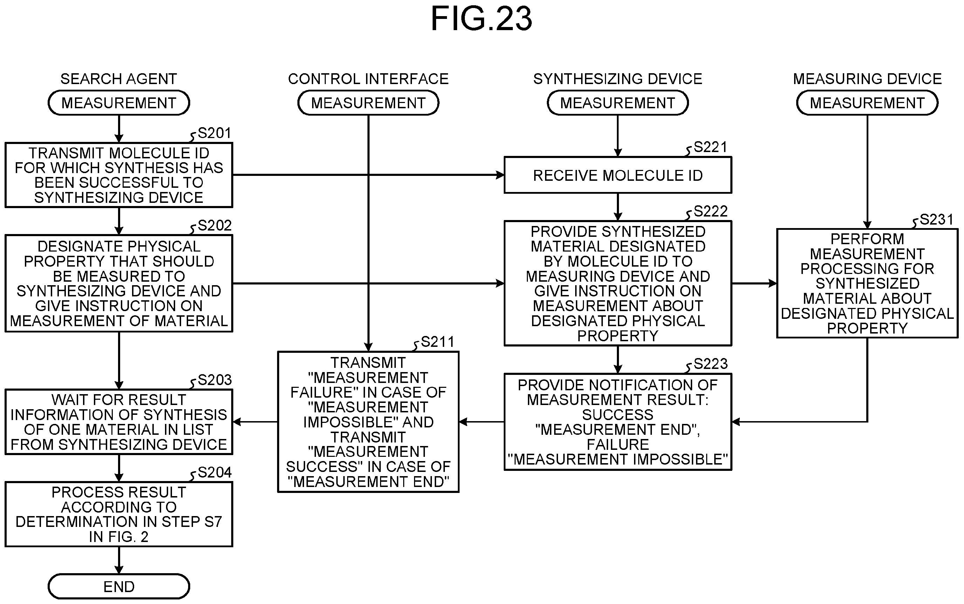

FIG. 23 illustrates a detailed flow of the processing (11) to (16) explained above. FIG. 23 is a sequence chart illustrating an example of a processing procedure in the material searching and manufacturing system. Note that step numbers illustrated in FIG. 23 are added for convenience in order to explain processing by processing subjects and do not specify order of processing.

The search agent 26 transmits the molecule ID for which the synthesis has been successful to the synthesizing device 11 (Step S201). The search agent 26 designates a physical property that should be measured to the synthesizing device 11 and instructs the synthesizing device 11 to measure a material (Step S202). The search agent 26 waits for result information of synthesis of one material in the list from the synthesizing device 11 (Step S203). The search agent 26 processes a result according to the determination in Step S7 of FIG. 2 (Step S204) and ends the processing.

The control interface 21 transmits the "measurement failure" to the search agent 26 when receiving the "measurement impossible" and transmits the "measurement success" to the search agent 26 when receiving the "measurement end" (Step S211).

The synthesizing device 11 receives the molecule ID (Step S221). The synthesizing device 11 provides the synthesized material designated by the molecule ID to the measuring device 13 and instructs the measuring device 13 to measure the designated physical properties (Step S222). The synthesizing device 11 provides notification of a measurement result (Step S223). The synthesizing device 11 notifies the search agent 26 of the "measurement end" in a case of success and notifies the search agent 26 of information corresponding to the "measurement impossible" in a case of failure via the control interface 21.

The measuring device 13 performs measurement processing for the synthesized material for the physical properties designated from the synthesizing device 11 (Step S231). Then, the measuring device 13 transmits a measurement result to the synthesizing device 11.

[2-3. System configuration example and the like]

Note that, in the above example, a synthesis phase and a measurement phase are separated and the search agent 26 instructs the synthesis agent on the synthesis phase and the measurement phase. However, after the synthesis instruction of the search agent 26, the synthesizing device 11 may perform "notification of an analysis result" of the synthesis processing and, thereafter, automatically perform "measurement instruction" of the measurement processing. In the above example, the control interface 21 performs conversion of information. However, the synthesizing device 11 may perform the information conversion.

In the synthesis phase and the measurement phase, when the analyzing device 12 and the measuring device 13 receive instructions, the synthesizing device 11 receives the information, data, and an instruction via the control interface 21, the synthesizing device 11 transmits the information, the data, and the instruction to the analyzing device 12 or the measuring device 13, and the synthesizing device 11 receives result information from the analyzing device 12 or the measuring device 13. However, the analyzing device 12 or the measuring device 13 may directly exchange data with the control interface 21 without intervention of the synthesizing device 11.

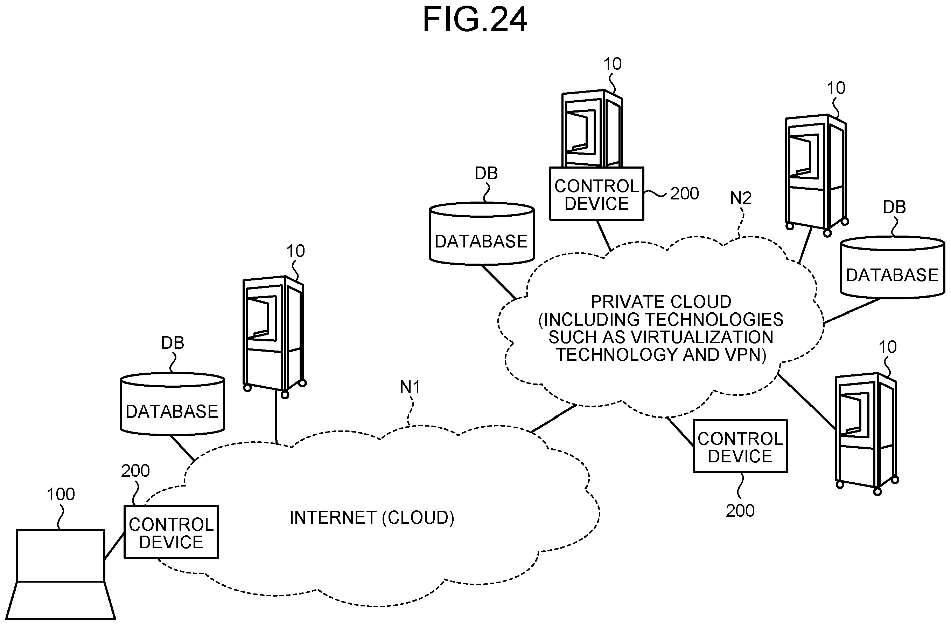

In this case, the search agent 26 (or computer software or hardware corresponding thereto) may be enabled to directly control the analyzing device 12 or the measuring device 13 via the control interface 21. For example, the function of the search agent 26 may be included in a computer such as an information processing device 100 illustrated in FIG. 24. FIG. 24 is a diagram illustrating an example of a configuration of the material searching and manufacturing system. In this case, the control interface 21 may be a computer device (such as a control device 200 in FIG. 24) including a communication interface or may be a wired/wireless communication device. The control device 200 functions as the control device 20.

In the example illustrated in FIG. 24, the information processing device 100 communicates with the other components via a network N1 such as the Internet (Cloud) or a network N2 such as a private Cloud including a virtualization technology and a technology such as a VPN (Virtual Private Network). For example, the information processing device 100 communicates with the material manufacturing device 10, the database DB, and the like via the control device 200. For example, the database DB may be present on a computer device connected to the synthesizing device 11 via a network or may operate on a computer device on the Cloud or on a device integrated with the synthesizing device 11. For example, the database DB may include the same database as the physical property database 22 (the physical property DB in FIG. 1) or the executability database 23 (the executability DB in FIG. 1). Note that the database DB may be present on separate storages like the physical property database 22 (the physical property DB in FIG. 1) and the executability database 23 (the executability DB in FIG. 1), may be integrated into one storage as one database DB, or may be present on a virtual storage device.

The control interface 21 may have a function of accessing a database and may perform processing concerning the material synthesis list performed by the synthesizing device 11 in the synthesis phase. The synthesizing device 11, the analyzing device 12, and the measuring device 13 may be systems having the same control system in one housing or may be independent in separate housings.

[2-3-1. Information processing device example]

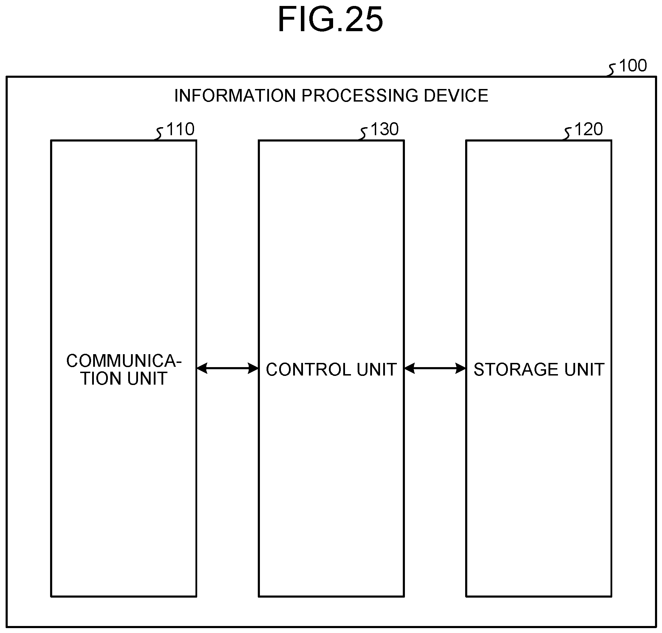

Here, a configuration example of the information processing device 100 is explained. FIG. 25 is a diagram illustrating a configuration example of the information processing device of the present disclosure. For example, the information processing device 100 functions as the control device 20. The information processing device 100 may be integrated with the control device 200.

As illustrated in FIG. 25, the information processing device 100 includes a communication unit 110, a storage unit 120, and a control unit 130. Note that the information processing device 100 may include an input unit (for example, a keyboard and a mouse) that receives various kinds of operation from an administrator or the like of the information processing device 100 and a display unit (for example, a liquid crystal display) for displaying various kinds of information.

The communication unit 110 is implemented by, for example, an NIC (Network Interface Card). The communication unit 110 is connected to a predetermined network (not illustrated) by wire or radio and transmits and receives information to and from the components of the material searching and manufacturing system 1 such as the material manufacturing device 10 and the control device 20. For example, the communication unit 110 transmits information to the material manufacturing device 10 to thereby function as the control interface 21 that controls the material manufacturing device 10.

The communication unit 110 controls the synthesis processing by transmitting and receiving information to and from the material manufacturing device 10. The communication unit 110 instructs the material manufacturing device 10 to perform the synthesis processing. For example, the communication unit 110 transmits information indicating a target material of the synthesis processing to thereby instruct the material manufacturing device 10 to perform the synthesis processing. The communication unit 110 receives a synthesis processing result from the material manufacturing device 10. The communication unit 110 receives a physical property measurement processing result from (the measuring device 13 of) the material manufacturing device 10.

The storage unit 120 is implemented by a semiconductor memory element such as a RAM (Random Access Memory) or a flash memory or a storage device such as a hard disk or an optical disk. The storage unit 120 stores various kinds of information such as the physical property database 22 that retains physical property data, the executability database 23 that retains whether synthesis and/or measurement has been successfully executed, the physical property prediction model 24 that predicts physical properties, and the execution possibility model 25 that predicts whether synthesis and/or measurement can be executed.

The control unit 130 is implemented by, for example, a program (for example, an information processing program according to the present disclosure) stored inside the information processing device 100 being executed using a RAM or the like as a work area by a CPU (Central Processing Unit), an MPU (Micro Processing Unit), or the like. The control unit 130 is implemented by an integrated circuit such as an ASIC (Application Specific Integrated Circuit) or an FPGA (Field Programmable Gate Array).

The control unit 130 executes respective kinds of processing using information received from an external device by the communication unit 110. The control unit 130 executes respective kinds of processing using information stored by the storage unit 120. The control unit 130 controls the material manufacturing device 10 by transmitting information to the material manufacturing device 10 via the communication unit 110.

The control unit 130 executes processing corresponding to the function of the search agent 26. The control unit 130 executes the processing concerning the synthetic material selection explained above. The control unit 130 executes the processing concerning the manufacturing of the material explained above. The control unit 130 instructs the material manufacturing device 10 to synthesize a material to thereby cause the material manufacturing device 10 to execute the processing for manufacturing a material.

The control unit 130 performs material selection for selecting, among the materials stored in the storage unit 120, materials using the material physical property information and the execution possibility information. The control unit 130 instructs the material manufacturing device 10 to perform synthesis processing for the selected materials. The control unit 130 transmits information in a list format to the material manufacturing device 10 according to priority to thereby control the synthesis processing for the material by the material manufacturing device 10. For example, the control unit 130 transmits the search table #2 to the material manufacturing device 10 to thereby control the synthesis processing for the material by the material manufacturing device 10. For example, the control unit 130 transmits information in a list format including designation of a material set as a target of the synthesis processing (a synthesis processing target material) to the material manufacturing device 10 to thereby designate the synthesis processing target material. For example, the control unit 130 transmits the search table #2 in which a flag is attached to the synthesis processing target material to the material manufacturing device 10 to thereby designate the synthesis processing target material. The control unit 130 updates the execution possibility information stored in the storage unit 120 based on a synthesis processing result from the material manufacturing device 10. For example, the control unit 130 updates the information of the search table #2 based on the synthesis processing result from the material manufacturing device 10.

The control unit 130 performs material selection for selecting materials from two or more synthesis processing target materials stored in the storage unit 120. The control unit 130 performs the material selection from the synthesis processing target materials not to include materials successfully synthesized in the past. For example, the control unit 130 refers to the information stored in the storage unit 120, excludes materials successfully synthesized in the past, and performs material selection. Consequently, the control unit 130 can realize selection of a new material. The control unit 130 performs material selection according to priority based on a first prediction value of a material based on the material physical property information. The control unit 130 performs priority material selection based on a second prediction value of the material based on the execution possibility information. The control unit 130 performs the material selection targeting a material, the second prediction value of which is equal to or larger than a predetermined threshold.

When success/failure information of synthesis is success, the control unit 130 updates the execution possibility information stored in the storage unit 120 for the material for which the synthesis has been successful. The control unit 130 updates the execution possibility model 25 based on the updated execution possibility information.

The control unit 130 designates an identifier of the successfully synthesized material and a physical property to be measured and gives an instruction on physical property measurement processing. The control unit 130 updates the material physical property information. The control unit 130 updates the material physical property information of the successfully synthesized material in the storage unit 120 based on a physical property measurement processing result from the material manufacturing device 10. When the physical property measurement processing result satisfies the target value, the control unit 130 updates the material physical property information for the material satisfying the target value stored in the storage unit 120. The control unit 130 updates the physical property prediction model 24 based on the updated material physical property information.

The control unit 130 controls the physical property measurement processing on which the instruction is given for a successfully synthesized material specified by an identifier and provides the identifier and a measured physical property value. When the devices are connected via a network, the control unit 130 transmits the physical property value to the other devices via the communication unit 110. The control unit 130 transmits the identifier of the successfully synthesized material and information indicating the measured physical property value to the other devices via the communication unit 110. The control unit 130 performs material selection using data having a synthetic material selection data structure (also referred to as "synthetic material selection data"). For example, the control unit 130 performs the material selection using data (synthetic material selection data) such as the search table #2 in FIG. 10 or the candidate material table #2 in FIG. 13 to FIG. 15. Specifically, the control unit 130 performs the material selection using the synthetic material selection data including an ID (for example, a molecule ID) for identifying a material, priority information (for example, a value of priority) indicating priority based on a physical property value of the material to be predicted, execution possibility information (for example, a value of feasibility) indicating execution possibility of synthesis of the material, and synthesis success/failure information (for example, a synthesis success/failure value) indicating success/failure of the synthesis processing. Using the synthetic material selection data, the control unit 130 performs a search targeting a material group, synthesis success/failure information of which indicates unprocessed, in descending order of the priority based on the priority information and selects materials, success/failure of the synthesis processing of which is indicated by the synthesis success/failure information satisfies a predetermined standard. For example, the control unit 130 performs a search targeting a molecular group, a value of synthesis pass/fail of which is unknown (n/a), in descending order of values of priority and selects materials, a value of feasibility of which is equal to or larger than a predetermined threshold.

[2-3-2. Configuration example and the like of the material manufacturing device]

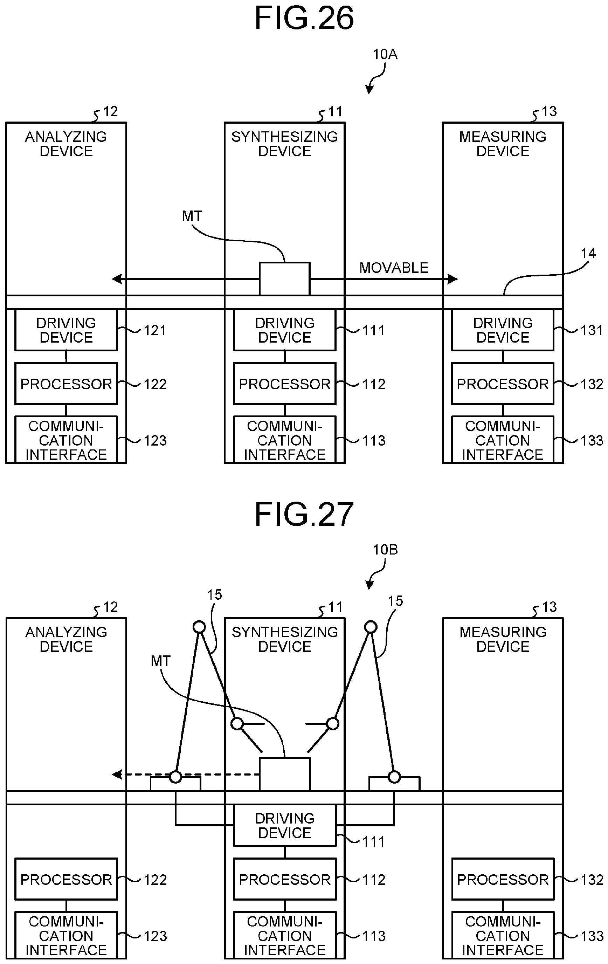

Here, the material manufacturing device 10 desirably includes a mechanism for automatically moving a synthetic material between the synthesizing device 11 and the analyzing device 12 or the measuring device 13 (also referred to as "material moving mechanism"). This point is explained below with reference to FIG. 26 to FIG. 28. FIG. 26 to FIG. 28 are diagrams illustrating an example of a configuration of the material manufacturing device. Note that, in the following explanation, the material manufacturing devices 10A, 10B, and 10C are explained according to an aspect of the material moving mechanism. However, components other than the components concerning the material moving mechanism are the same as the components of the material manufacturing device 10. For the material manufacturing device 10, explanation is omitted as appropriate about the same points as the points of the contents explained above.

The material manufacturing device 10A illustrated in FIG. 26 includes a material moving path 14 as a material moving mechanism. The synthesizing device 11 includes a driving device 111 that drives the material moving path 14, a processor 112 that executes control and synthesis processing for the driving device 111, and a communication interface 113 for communicating with the other devices. The analyzing device 12 includes a driving device 121 that drives the material moving path 14, a processor 122 that executes control and analysis processing for the driving device 121, and a communication interface 123 for communicating with the other devices. The measuring device 13 includes a driving device 131 that drives the material moving path 14, a processor 132 that executes control and measurement processing for the driving device 131, and a communication interface 133 for communicating with the other devices. The material moving path 14 illustrated in FIG. 26 is a belt conveyor or the like provided across the synthesizing device 11, the analyzing device 12, and the measuring device 13. A material MT illustrated in FIG. 26 is movable among the synthesizing device 11, the analyzing device 12, and the measuring device 13 according to an operation of the material moving path 14.

A material manufacturing device 10B illustrated in FIG. 27 includes a robot arm 15 as a material moving mechanism. The synthesizing device 11 includes the driving device 111 that drives the robot arm 15, the processor 112 that executes control and synthesis processing for the driving device 111, and the communication interface 113 for communicating with the other devices. In addition, the analyzing device 12 includes the processor 122 that executes analysis processing and the communication interface 123 for communicating with the other devices. The measuring device 13 includes the processor 132 that executes measurement processing and the communication interface 133 for communicating with the other devices. The robot arm 15 illustrated in FIG. 27 is driven by the driving device 111 of the synthesizing device 11. The material MT illustrated in FIG. 27 is movable among the synthesizing device 11, the analyzing device 12, and the measuring device 13 according to an operation of the robot arm 15.

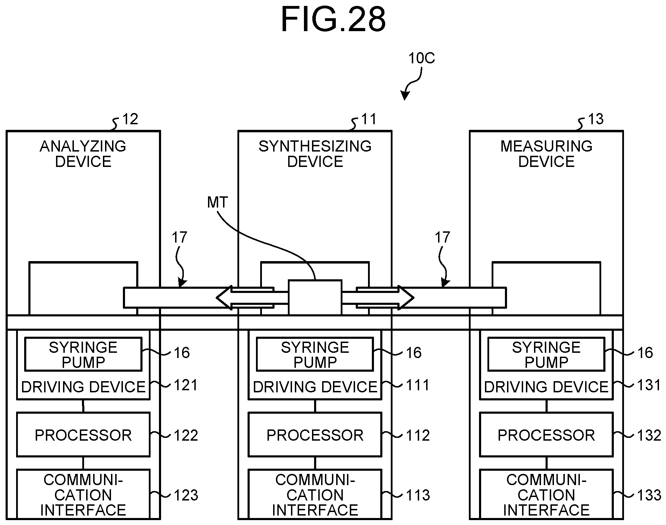

Further, a material manufacturing device 10C illustrated in FIG. 28 includes a flow path by a syringe pump 16 and a tube 17 as a material moving mechanism. The synthesizing device 11 includes the driving device 111 that drives the syringe pump 16, the processor 112 that executes control and synthesis processing for the driving device 111, and the communication interface 113 for communicating with the other devices. In addition, the analyzing device 12 includes the processor 122 that executes analysis processing and the communication interface 123 for communicating with the other devices. The measuring device 13 includes the processor 132 that executes measurement processing and the communication interface 133 for communicating with the other devices. The syringe pump 16 illustrated in FIG. 28 is driven by the driving device 111 of the synthesizing device 11. A synthesized material (a sample as a solution) MT illustrated in FIG. 28 is movable among the synthesizing device 11, the analyzing device 12, and the measuring device 13 through a flow path by the tube 17 according to an operation of the syringe pump 16.

Note that the above is merely an example and the material manufacturing device 10 may include a material moving mechanism of any form as long as a material can be moved among the devices. The material manufacturing device 10 executes processing for manufacturing a material. The material manufacturing device 10 executes, according to an operation by a user or the like of the material manufacturing device 10, processing for manufacturing a material. The material manufacturing device 10 executes, according to an instruction from an external device, processing for manufacturing a material. For example, the material manufacturing device 10 executes, according to an instruction from the control device 20, processing for manufacturing a material.

In the material manufacturing device 10A illustrated in FIG. 26, the material manufacturing device 10B illustrated in FIG. 27, or the material manufacturing device 10C illustrated in FIG. 28, the material synthesized by the synthesizing device 11 is automatically moved to the measuring device 13 and the measuring device 13 performs processing for measuring a physical property value on which an instruction is given targeting the moved material. The measuring device 13 transmits a physical property measurement processing result generated by the measurement processing to the other devices. For example, (the measuring device 13 of) the material manufacturing device 10 transmits the physical property measurement processing result to the other devices such as the control device 20, the information processing device 100, and the control device 200.

[3. Object and effects of the present disclosure]

As explained above, the synthetic material selection method according to the present disclosure performs material selection for selecting synthesis target materials using material physical property information of a database (in the above example, the information indicated by in the database DB1 and the like. The same applies below) and execution possibility information (in the above example, the information indicated in the database DB2 and the like. The same applies below), instructs a control device (in the above example, the material manufacturing device 10, the control device 20, the information processing device 100, the control device 200, or the like. The same applies below) to perform synthesis processing for the selected materials, and updates the execution possibility information of the database based on a synthesis processing result from the control device. As explained above, for example, the synthetic material selection method executed by the material searching and manufacturing system 1 includes performing material selection for selecting synthesis target materials using the material physical property information and the execution possibility information of the database, giving an instruction on the synthesis processing for the selected materials, and updating the execution possibility information in the database based on a synthesis processing result. Consequently, the synthetic material selection method can enable material synthesis based on executability of synthesis.

In the synthetic material selection method according to the present disclosure, the database includes two or more synthesis processing target materials. Consequently, the synthetic material selection method can select materials using the database including the two or more synthesis processing target materials.

In the material selection in the synthetic material selection method according to the present disclosure, the materials are selected not to include materials successfully synthesized in the past. Consequently, the synthetic material selection method can select materials not to include materials successfully synthesized in the past.

The material selection in the synthetic material selection method according to the present disclosure is performed according to priority based on a first prediction value of a material based on material physical property information. Consequently, the synthetic material selection method can select materials corresponding to the priority based on the first prediction value of the material.

In the synthetic material selection method according to the present disclosure, the first prediction value is output based on a material feature value of each of two or more materials configuring materials registered in the database input to a physical property prediction model (in the above example, the physical property prediction model 24. The same applies below). Consequently, the synthetic material selection method can select materials corresponding to priority based on the first prediction value of the material predicted by the physical property prediction model.

The material selection in the synthetic material selection method according to the present disclosure is performed according to priority based on a second prediction value of the material based on the execution possibility information. Consequently, the synthetic material selection method can select materials corresponding to the second prediction value of the material based on the execution possibility information.

In the material selection in the synthetic material selection method according to the present disclosure, materials are selected from materials whose the second prediction value is equal to or larger than a predetermined threshold. Consequently, the synthetic material selection method can select appropriate materials.

In the synthetic material selection method according to the present disclosure, the second prediction value is output based on the material feature value of each of the two or more materials configuring the materials registered in the database, the material feature value being input to the execution possibility model 25. Consequently, the synthetic material selection method can select materials corresponding to the second prediction value of the material based on the execution possibility information.

In the synthetic material selection method according to the present disclosure, the selected materials are selected in order of priority, the selected materials are designated in a list format including at least one item, and the items include identifiers and priority of the materials. Consequently, the synthetic material selection method can appropriately select materials.

In the synthetic material selection method according to the present disclosure, the control device controls the synthesis processing for the materials provided in the list format according to the priority and provides a synthesis processing result for each of the materials, and the synthesis processing result is identifiers of the materials of the controlled synthesis processing and success/failure information of synthesis. Consequently, the synthetic material selection method can appropriately select materials.

Furthermore, in the synthetic material selection method according to the present disclosure, the control of the synthesis processing includes processing for instructing a synthesizing device (in the above example, the synthesizing device 11 or the material manufacturing device 10. The same applies below) to perform the synthesis processing and receiving a synthesis processing result from the synthesizing device. Consequently, the synthetic material selection method can make the synthesis processing result available.

In the update of the execution possibility information in the synthetic material selection method according to the present disclosure, when the success/failure information of the synthesis is success, the execution possibility information is updated for the material for which the synthesis has been successful in the database, and the execution possibility model 25 is updated based on the updated execution possibility information. Consequently, the synthetic material selection method can appropriately select materials using the updated model.

In addition, the synthetic material selection method according to the present disclosure includes processing for designating, to the control device, based on success/failure information of the synthesis, an identifier of a successfully synthesized material and a physical property to be measured, instructing the control device to perform physical property measurement processing, and updating material physical property information of the successfully synthesized material in the database based on a physical property measurement processing result from the control device. Consequently, the synthetic material selection method can select appropriate materials using the updated material physical property information.

In the synthetic material selection method according to the present disclosure, the update of the material physical property information includes, when the physical property measurement processing result satisfies the target value, updating the material physical property information for the material satisfying the target value in the database and updating the physical property prediction model based on the updated material physical property information. Consequently, the synthetic material selection method can select appropriate materials using the updated physical property prediction model.

In the synthetic material selection method according to the present disclosure, the control device controls the physical property measurement processing on which the instruction is given for the successfully synthesized material specified by the identifier and provides the identifier and the measured physical property value. Consequently, the synthetic material selection method can provide information indicating appropriately measured physical property values and materials corresponding to the physical property values.

In the synthetic material selection method according to the present disclosure, the control of the physical property measurement processing includes processing for automatically moving the material synthesized by the synthesizing device to a measuring device (in the above example, the measuring device 13. The same applies below), instructing the measuring device to perform the physical property measurement on which the instruction is given, and receiving a physical property measurement processing result from the measuring device. Consequently, the synthetic material selection method can make the physical property measurement processing result available.

In the synthetic material selection method according to the present disclosure, attributes of the materials includes at least one of low molecules, dyes, polymers, fluorescent and identified isotope labels, self-assembled materials and structures, biomaterials (saccharides, peptides, polypeptides, amino acids, proteins, fatty compounds, DNA (Deoxyribonucleic Acid), and the like), organic thin films (deposition and application process), inorganic materials (solid phase method, coprecipitation method, melt quenching method, sol-gel method, and the like), nanoparticles, metal complexes, inorganic thin films (ALD (Atomic layer deposition), sputtering, and the like), synthetic materials based on synthetic biological techniques (material synthesis by genetic recombination and bacterial utilization), and functional materials having a crystal structure, a nanostructure, and a microstructure. Consequently, the synthetic material selection method can appropriately select materials using the information concerning the attributes of the materials.

In the synthetic material selection method according to the present disclosure, the material physical property information in the database is structured to include at least two items and one of the at least two items is an attribute item of the materials. Consequently, in the synthetic material selection method, the materials can be appropriately selected using the information concerning the attributes of the material physical property information including the two or more items.

As explained above, the material manufacturing method according to the present disclosure manufactures a material by performing material selection for selecting synthesis target materials using the material physical property information and the execution possibility information in the database, instructing the control device to perform the synthesis processing for the selected materials, and updating the execution possibility information of the database based on the synthesis processing result from the control device. Consequently, the synthetic material selection method can manufacture a synthesized material based on executability of the synthesis.

As explained above, the synthetic material selection data structure according to the present disclosure is a synthetic material selection data structure used in an information processing device (in the above example, the control device 20, the information processing device 100, the control device 200, or the like. The same applies below) that selects synthesis processing target material, the synthetic material selection data structure including an ID for identifying a material, priority information indicating priority based on a physical property value of a material to be predicted, execution possibility information indicating execution possibility of material synthesis, and synthesis success/failure information indicating success/failure of synthesis processing. The synthetic material selection data structure is used in processing in which the information processing device searches for, targeting a material group, synthesis success/failure information of which indicates unprocessed, materials in descending order of the priority based on the priority information and selects materials, success/failure of synthesis processing of which indicated by the synthesis success/failure information satisfies a predetermined standard. Consequently, the synthetic material selection method can enable material synthesis based on executability of synthesis.

As explained above, the material searching and manufacturing system 1 is a material searching and manufacturing system that is dramatically efficient in time and cost by avoiding materials highly likely to be unsuccessful in synthesis/measurement and reducing unnecessary synthesis/measurement.

As explained above, in the material searching and manufacturing system 1, a new material that was not synthesized in the past is selected as a synthesis target based on information of a current database, the database is updated in real time using a result of attempting synthesis in an actual synthesizing device, and, even when the synthesis has been successful, data can be updated in real time using a result of measuring whether a material having an expected property has been successfully generated, and the execution possibility model 25 and the physical property prediction model are constructed using the result. Therefore, when material selection is performed next time, it is possible to construct a material searching and manufacturing system that is dramatically efficient in terms of time and cost by avoiding materials highly likely to be unsuccessful in synthesis/measurement and reducing unnecessary synthesis/measurement.

As explained above, the material searching and manufacturing system 1 is a highly accurate physical property prediction system that utilizes a physical property database in which physical property values with uniform material manufacturing conditions are accumulated.

As explained above, the material searching and manufacturing system 1 includes the database in which the physical property values are accumulated and can construct and update the physical property prediction model such that data can be updated based on the latest measurement data of a synthesized object in conjunction with the synthesizing device. Consequently, the synthetic material selection method has an effect that physical properties of a material that can be newly synthesized can be predicted with higher accuracy compared with the method of the related art.

As explained above, the material searching and manufacturing system 1 is a material synthesis possibility prediction system and a measurement success probability prediction system that utilize the executability database 23 in which the execution possibility data (success or failure of synthesis/measurement) with the complete material manufacturing conditions are accumulated.

As explained above, in the material searching and manufacturing system 1, a new material is selected based on information of the database at the time of material selection and the database is updated in real time using a result of trying synthesis in the actual synthesizing device. In addition, even when the synthesis has been successful, data can be updated in real time using a result of measuring whether a material having an expected property has been successfully generated, and the execution possibility model 25 and the physical property prediction model 24 are constructed using the result. Therefore, it is possible to construct a material synthesize possibility prediction system and a measurement success probability prediction system utilizing the executability database 23 in which executability data (success or failure of synthesis/measurement) with uniform material manufacturing conditions are accumulated.

As explained above, the material searching and manufacturing system 1 is a material synthesis/search system that optimizes, with high efficiency, materials, synthesis processes, and measurement processes such as low molecules, dyes, polymers, fluorescent and identified isotope labels, self-assembled materials and structures, biomaterials (saccharides, peptides, polypeptides, amino acids, proteins, fatty compounds, DNA, and the like), organic thin films (deposition and application process), inorganic materials (solid phase method, coprecipitation method, melt quenching method, sol-gel method, and the like), nanoparticles, metal complexes, inorganic thin films (ALD, sputtering, and the like), and synthetic materials based on synthetic biological techniques (material synthesis by genetic recombination and bacterial use). The material searching and manufacturing system 1 is a structure search system that performs synthesis/measurement and characteristic optimization of functional materials having a crystal structure, a nanostructure, and a microstructure and is a structure search system that searches for stable and metastable crystal structure and aggregate structure.

As explained above, in the material searching and manufacturing system 1, a new material is selected using information concerning material attributes and the database is updated in real time using a result of trying synthesis in the actual synthesizing device. In addition, even if the synthesis has been successful, data can be updated in real time using a result of measuring whether a material having an expected property has been successfully generated, and the execution possibility model 25 and the physical property prediction model 24 are constructed using the result. Therefore, it is possible to construct a material synthesize possibility prediction system and a measurement success probability prediction system utilizing the executability database 23 in which executability data (success or failure of synthesis/measurement) with uniform material manufacturing conditions are accumulated.

As explained above, materials that can be selected in the database of the present disclosure are managed by descriptors, material physical property information of the materials is structured to include at least two or more data items, and a part of the structured data items concerns attributes of the materials.

Note that the attributes are, for example, attributes concerning a functional material and includes, for example, low molecules, dyes, polymers, fluorescent and identified isotope labels, self-assembled materials and structures, biomaterials (saccharides, peptides, polypeptides, amino acids, proteins, fatty compounds, DNA (Deoxyribonucleic Acid), and the like), organic thin films (deposition and application process), inorganic materials (solid phase method, coprecipitation method, melt quenching method, sol-gel method, and the like), nanoparticles, metal complexes, inorganic thin films (ALD (Atomic layer deposition), sputtering, and the like), synthetic materials based on synthetic biological techniques (material synthesis by genetic recombination and bacterial utilization), a crystal structure, a nanostructure, and a microstructure.

As explained above, the material searching and manufacturing system 1 is a search system that efficiently optimizes all characteristics and physical properties (mechanical properties, thermal properties, electrical properties, magnetic properties, optical properties, electrochemical properties, drug efficacy, toxicity, antibody reaction, interaction with cells, interaction with internal organs, intracellular transportability, in-vivo transportability, adsorbability, solubility, and the like) that can be evaluated by material manufacturing by physical, chemical, and biological methods.

The characteristics measured in the present disclosure may be, for example, all characteristics and physical properties (mechanical properties, thermal properties, electrical properties, magnetic properties, optical properties, electrochemical properties, drug efficacy, toxicity, antibody reaction, interaction with cells, interaction with internal organs, intracellular transportability, in-vivo transportability, adsorbability, solubility, and the like) that can be evaluated by material manufacturing by physical, chemical, and biological methods.

As explained above, in the material searching and manufacturing system 1, a new material is selected based on information concerning all the characteristics and physical properties that can be evaluated by material manufacturing by physical, chemical, and biological methods, and the database is updated in real time using a result of trying synthesis in an actual synthesizing device and, even when synthesis has been successful, data can be updated in real time using a result of measuring whether a material having an expected property has been successfully generated, and the execution possibility model 25 and the physical property prediction model 24 are constructed using the result. Therefore, it is possible to construct a manufacturing system for a new material utilizing the executability database 23 in which executability data (success or failure of synthesis/measurement) with uniform material manufacturing conditions are accumulated.

Note that the material searching and manufacturing system 1 explained above is merely an example of a processing system that performs various kinds of processing. The processing system may be a system used in various uses. For example, the processing system may be a processing system used in the following uses.

Concerning material synthesis and physical property measurement, since the executability database 23 including both of success data and failure data is obtained, it is possible to respectively find characteristics of materials for succeeding in the material synthesis and physical property measurement and characteristics of materials for failing in the material synthesis and physical property measurement. By utilizing these characteristics of the materials, it is possible to implement a material searching and manufacturing system having an automatic expansion function for setting a range of a material search space. For example, the processing system is a search system that presents, by itself, optimum device function expansion, for example, indicating automatic detection and an improvement policy for a problem/rate-limiting process of the material manufacturing device from an analysis of the success data and the failure data.

For example, the processing system is a material searching and manufacturing system that properly uses, according to a situation, a plurality of material manufacturing devices accessible through the Internet. It is possible to construct a universal execution possibility prediction system by exchanging executability data one another among the devices and integrating the executability data.

In this case, there is an automatic material synthesis system that can easily perform data management in addition to creation of a workflow of material synthesis and device control such as designation of a material, a dispensing amount, and the like using dedicated software. However, causing the automatic material synthesis system to cooperate with the configuration or the processing of the present disclosure, it is possible to reflect analysis data and the like during material manufacturing in a database and, further, it is possible to automatically select next candidate materials based on success/failure of synthesis in the most recent material synthesis, a degree of target achievement in an analysis result, and the like. Therefore, it is possible to perform a material search that can more efficiently select a material having a high success probability.

By adopting the material searching and manufacturing system that properly uses a plurality of devices accessible through the Internet, it is possible to construct a universal execution possibility prediction system by exchanging executability data among the devices and integrating the executability data.

The control network only has to be connected to the Internet via a wired network or a wireless network such as Wi-Fi (registered trademark) (Wireless-Fidelity) or 4G/5G.

The synthesizing device and the like used in the present disclosure are assumed to be large-scale. However, the synthesizing device and the like can transmit and receive data in real time via a control interface by making it possible to integrally manage distributed material manufacturing devices as one system using a virtualization technology on the Cloud.

Consequently, it is possible to perform the next material search and, further, continuously control the material manufacturing device, and continue the material synthesis by, via a network, giving an instruction on synthesis of materials selected in the material search of the present disclosure, receiving data concerning success or failure of the synthesis, further giving an instruction on an analysis, and receiving data concerning success or failure of the analysis.

For example, the executability database 23 of the present disclosure is a comparable and reusable executability database based on standardization of executability data. For example, the processing system is a cost prediction system for highly accurate material development utilizing the executability database 23. For example, a service provided by the processing system is a highly accurate material search service utilizing the execution possibility model 25.

[4. Others]

Among the kinds of processing explained in the embodiments explained above, all or a part of the processing explained as being automatically performed can be manually performed or all or a part of the processing explained as being manually performed can be automatically performed by a publicly-known method. Besides, the processing procedures, the specific names, and the information including the various data and parameters explained in the document and illustrated in the drawings can be optionally changed except when specifically noted otherwise. For example, the various kinds of information illustrated in the figures are not limited to the illustrated information.

The illustrated components of the devices are functionally conceptual and do not always need to be physically configured as illustrated in the figures. That is, a specific form of distribution and integration of the devices is not limited to the illustrated form. All or a part the devices can be configured by being functionally or physically distributed and integrated in any unit according to various loads, usage conditions, and the like.

The embodiments and the modifications explained above can be combined as appropriate in a range in which the processing contents are not contradictory.

The effects described in the present specification are merely illustrations and are not limited and other effects may be present.

[5. Hardware configuration]

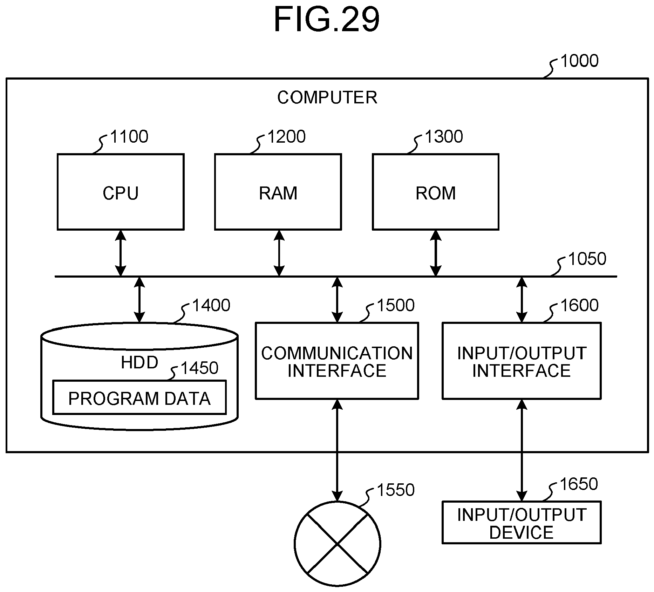

The information equipment such as the information processing device 100, the control device 200, and the material manufacturing device 10 according to the embodiments and the modifications explained above are implemented by, for example, a computer 1000 having a configuration as illustrated in FIG. 29. FIG. 29 is a hardware configuration diagram illustrating an example of a computer that implements the functions of the information processing device. The information processing device 100 is explained as an example below. The computer 1000 includes a CPU 1100, a RAM 1200, a ROM (Read Only Memory) 1300, an HDD (Hard Disk Drive) 1400, a communication interface 1500, and an input/output interface 1600. The units of the computer 1000 are connected by a bus 1050.

The CPU 1100 operates based on programs stored in the ROM 1300 or the HDD 1400 and controls the units. For example, the CPU 1100 develops the programs stored in the ROM 1300 or the HDD 1400 in the RAM 1200 and executes processing corresponding to various programs.

The ROM 1300 stores a boot program such as a basic input output system (BIOS) executed by the CPU 1100 at a start time of the computer 1000, a program depending on hardware of the computer 1000, and the like.

The HDD 1400 is a computer-readable recording medium that non-transiently records a program to be executed by the CPU 1100, data to be used by such a program, and the like. Specifically, the HDD 1400 is a recording medium that records an information processing program such as the information processing program according to the present disclosure that is an example of program data 1450.

The communication interface 1500 is an interface for the computer 1000 to connect to an external network 1550 (for example, the Internet). For example, the CPU 1100 receives data from the other equipment and transmits data generated by the CPU 1100 to the other equipment via the communication interface 1500.

The input/output interface 1600 is an interface for connecting an input/output device 1650 and the computer 1000. For example, the CPU 1100 receives data from an input device such as a keyboard or a mouse via the input/output interface 1600. The CPU 1100 transmits data to an output device such as a display, a speaker, or a printer via the input/output interface 1600. The input/output interface 1600 may function as a media interface that reads a program and the like recorded in a predetermined recording medium (a medium). The medium is, for example, an optical recording medium such as a DVD (Digital Versatile Disc) or a PD (Phase change rewritable Disk), a magneto-optical recording medium such as an MO (Magneto-Optical disk), a tape medium, a magnetic recording medium, or a semiconductor memory.

For example, when the computer 1000 functions as the information processing device 100, the CPU 1100 of the computer 1000 implements the functions of the control unit 130 and the like by executing an information processing program such as an information processing program loaded on the RAM 1200. In the HDD 1400, an information processing program such as an information processing program according to the present disclosure and data in the storage unit 120 are stored. Note that the CPU 1100 reads the program data 1450 from the HDD 1400 and executes the program data. However, as another example, the CPU 1100 may acquire these programs from another device via the external network 1550.

Note that the present technology can also take the following configurations.

(1)
A synthetic material selection method comprising:

performing material selection for selecting synthesis target materials based on material physical property information and execution possibility information of a database;
instructing a control device to perform synthesis processing for the selected materials; and
updating the execution possibility information of the database based on a synthesis processing result from the control device.

(2) The synthetic material selection method according to (1), wherein
the database includes two or more synthesis processing target materials.
(3) The synthetic material selection method according to (1) or (2), wherein,

in the material selection, materials are selected not to include materials successfully synthesized in past.

(4) The synthetic material selection method according to any one of (1) to (3), wherein
the material selection is performed according to priority based on a first prediction value of a material based on the material physical property information.

(5) The synthetic material selection method according to (4), wherein
the first prediction value is output from a physical property prediction model according to input based on a material feature value of each of two or more materials configuring the materials registered in the database.

(6) The synthetic material selection method according to (4) or (5), wherein
the material selection is performed according to priority based on a second prediction value of a material based on the execution possibility information.

(7) The synthetic material selection method according to (6), wherein,
in the material selection, materials are selected from materials whose the second prediction value is equal to or larger than a predetermined threshold.

(8) The synthetic material selection method according to (6) or (7), wherein
the second prediction value is output from an execution possibility model according to input based on a material feature value of each of the two or more materials configuring the materials registered in the database.

(9) The synthetic material selection method according to any one of (4) to (8), wherein
the selected materials are selected in order of the priority, the selected materials are designated in a list format including at least one item, and the items include identifiers and priority of the materials.

(10) The synthetic material selection method according to (9), wherein
the control device controls synthesis processing for the materials provided in the list format according to the priority and provides the synthesis processing result for each of the materials, and the synthesis processing result is identifiers and success/failure information of synthesis of the materials for which the synthesis processing is controlled.

(11) The synthetic material selection method according to (10), wherein
the control of the synthesis processing includes processing for instructing a synthesizing device to perform the synthesis processing and receiving the synthesis processing result from the synthesizing device.

(12) The synthetic material selection method according to (11), wherein,
in the update of the execution possibility information, when the success/failure information of the synthesis is success, in the database, the execution possibility information is updated for the material for which the synthesis has been successful and the execution possibility model is updated based on the updated execution possibility information.

(13) The synthetic material selection method according to (11) or (12), further comprising
processing for, while designating an identifier of the successfully synthesized material and a physical property to be measured, instructing, based on the success/failure information of the synthesis, the control device to perform physical property measurement processing and updating material physical property information of the successfully synthesized material in the database based on a result of the physical property measurement processing from the control device.

(14) The synthetic material selection method according to (13), wherein
the update of the material physical property information includes processing for, when the result of the physical property measurement processing satisfies a target value, in the database, updating the material physical property information for the material satisfying the target value and updating a physical property prediction model based on the updated material physical property information.

(15) The synthetic material selection method according to (13) or (14), wherein
the control device controls the physical property measurement processing on which an instruction is given for the successfully synthesized material specified by the identifier and provides the identifier and the measured physical property value.

(16) The synthetic material selection method according to any one of (13) to (15), wherein
the control of the physical property measurement processing includes processing for automatically moving a material synthesized by the synthesizing device to a measuring device, instructing the measuring device to perform the physical property measurement on which the instruction is given, and receiving the result of the physical property measurement processing from the measuring device.

(17) The synthetic material selection method according to any one of (1) to (16), wherein
the material physical property information of the database is structured to include at least two items, and one of the at least two items is an attribute item of the materials.

(18) A material manufacturing method for manufacturing a material with a processing for:

   performing material selection for selecting a synthesis target material using material physical property information and execution possibility information in a database;
   instructing a control device to perform synthesis processing for the selected materials; and

updating the execution possibility information of the database based on a synthesis processing result from the control device.

(19) A data structure used in an information processing system including a storage unit and a processing unit, stored in the storage unit, and used for selecting synthesis target materials in the processing unit, the data structure comprising:

an ID for identifying a material; priority information indicating priority based on a physical property value of a material to be predicted; execution possibility information indicating execution possibility of synthesis of the material; and synthesis success/failure information indicating success/failure of synthesis processing, wherein the synthetic material selection data structure is used in processing in which, while targeting a material group, the synthesis success/failure information of which indicates unprocessed, the processing unit searches for materials in descending order of the priority based on the priority information and selects a material, success/failure of the synthesis processing of which is indicated by the synthesis success/failure information satisfies a predetermined standard.

(20) A manufacturing method comprising:

performing material selection according to execution possibility information output by a machine learning model; instructing a control device to perform synthesis processing for the selected materials; updating information of an executability database based on a synthesis processing result from the control device; updating the machine learning model based on the updated execution possibility information of the database; and manufacturing the updated machine learning model.

(21) The synthetic material selection method described in any one of (1) to (16), in which attributes of the materials includes at least one of a small molecule, a dye, a polymer, a fluorescent/identified isotope label, a self-assembled material/structure, a biomaterial (saccharides, peptides, polypeptides, amino acids, proteins, fatty compounds, DNA, and the like), an organic thin film (deposition and application process), an inorganic material (a solid phase method, a coprecipitation method, a melt quenching method, a sol-gel method, and the like), a nanoparticle, a metal complex, an inorganic thin film (ALD, sputtering, and the like), a synthetic material based on a synthetic biological technique (material synthesis by genetic recombination and bacterial use), and a functional material having a crystal structure, a nanostructure, and a microstructure.
(22) The synthetic material selection method described in any one of (13) to (16), in which the processing on which an instruction is given by the physical property measurement processing is a characteristic that can be evaluated by material manufacturing by a physical, chemical, or biological method.
(23) The synthetic material selection method described in (22), in which the characteristics include at least one of a mechanical property, a thermal property, an electrical property, a magnetic property, an optical property, an electrochemical property, a drug efficacy, toxicity, an antibody reaction, interaction with a cell, interaction with an internal organ, an intracellular transporting property, an in-vivo transporting property, an adsorbing property, and a dissolving property.
(24) A synthetic material selection device including:
a processing unit that performs material selection for selecting synthesis target materials using material physical property information and execution possibility information of a database; and a control unit that instructs a control device to perform synthesis processing for the selected materials and updates the execution possibility information of the database based on a material synthesis processing result from the control device.
(25) A synthetic material selection program for executing processing for:

performing material selection for selecting synthesis target materials using material physical property information and execution possibility information of a database; instructing a control device to perform synthesis processing for the selected materials; and updating the execution possibility information of the database based on a synthesis processing result from the control device.

Reference Signs List

1    MATERIAL SEARCHING AND MANUFACTURING SYSTEM (INFORMATION PROCESSING SYSTEM)

10    MATERIAL MANUFACTURING DEVICE

11    SYNTHESIZING DEVICE

12    ANALYZING DEVICE

13    MEASURING DEVICE

20    CONTROL DEVICE

21    CONTROL INTERFACE

22    PHYSICAL PROPERTY DATABASE

23    EXECUTABILITY DATABASE

24    PHYSICAL PROPERTY PREDICTION MODEL

25    EXECUTION POSSIBILITY MODEL

26    SEARCH AGENT

N     CONTROL NETWORK


**Claims**

1.  A synthetic material selection method comprising:

    performing material selection for selecting synthesis target materials based on material physical property information and execution possibility information of a database;
    instructing a control device to perform synthesis processing for the selected materials; and
    updating the execution possibility information of the database based on a synthesis processing result from the control device.

2.  The synthetic material selection method according to claim 1, wherein
    the database includes two or more synthesis processing target materials.

3.  The synthetic material selection method according to claim 1, wherein,
    in the material selection, materials are selected not to include materials successfully synthesized in past.

4.  The synthetic material selection method according to claim 1, wherein
    the material selection is performed according to priority based on a first prediction value of a material based on the material physical property information.

5.  The synthetic material selection method according to claim 4, wherein
    the first prediction value is output from a physical property prediction model according to input based on a material feature value of each of two or more materials configuring the materials registered in the database.

6.  The synthetic material selection method according to claim 4, wherein
    the material selection is performed according to priority based on a second prediction value of a material based on the execution possibility information.

7.  The synthetic material selection method according to claim 6, wherein,
    in the material selection, materials are selected from materials whose the second prediction value is equal to or larger than a predetermined threshold.

8.  The synthetic material selection method according to claim 6, wherein

the second prediction value is output from an execution possibility model according to input based on a material feature value of each of the two or more materials configuring the materials registered in the database.

9. The synthetic material selection method according to claim 4, wherein
the selected materials are selected in order of the priority, the selected materials are designated in a list format including at least one item, and the items include identifiers and priority of the materials.

10. The synthetic material selection method according to claim 9, wherein
the control device controls synthesis processing for the materials provided in the list format according to the priority and provides the synthesis processing result for each of the materials, and the synthesis processing result is identifiers and success/failure information of synthesis of the materials for which the synthesis processing is controlled.

11. The synthetic material selection method according to claim 10, wherein
the control of the synthesis processing includes processing for instructing a synthesizing device to perform the synthesis processing and receiving the synthesis processing result from the synthesizing device.

12. The synthetic material selection method according to claim 11, wherein,
in the update of the execution possibility information, when the success/failure information of the synthesis is success, in the database, the execution possibility information is updated for the material for which the synthesis has been successful and the execution possibility model is updated based on the updated execution possibility information.

13. The synthetic material selection method according to claim 11, further comprising
processing for, while designating an identifier of the successfully synthesized material and a physical property to be measured, instructing, based on the success/failure information of the synthesis, the control device to perform physical property measurement processing and updating material physical property information of the successfully synthesized material in the database based on a result of the physical property measurement processing from the control device.

14. The synthetic material selection method according to claim 13, wherein
the update of the material physical property information includes processing for, when the result of the physical property measurement processing satisfies a target value, in the database, updating the material physical property information for the material satisfying the target value and updating a physical property prediction model based on the updated material physical property information.

15. The synthetic material selection method according to claim 13, wherein
the control device controls the physical property measurement processing on which an instruction is given for the successfully synthesized material specified by the identifier and provides the identifier and the measured physical property value.

16. The synthetic material selection method according to claim 13, wherein
the control of the physical property measurement processing includes processing for automatically moving a material synthesized by the synthesizing device to a measuring device, instructing the measuring device to perform the physical property measurement on which the instruction is given, and receiving the result of the physical property measurement processing from the measuring device.

17. The synthetic material selection method according to claim 1, wherein
the material physical property information of the database is structured to include at least two items, and one of the at least two items is an attribute item of the materials.

18. A material manufacturing method for manufacturing a material with a processing for:

performing material selection for selecting a synthesis target material using material physical property information and execution possibility information in a database;
instructing a control device to perform synthesis processing for the selected materials; and
updating the execution possibility information of the database based on a synthesis processing result from the control device.

19. A synthetic material selection data structure used in an information processing system including a storage unit and a processing unit, stored in the storage unit, and used for selecting synthesis target materials in the processing unit, the synthetic material selection data structure comprising:

an ID for identifying a material; priority information indicating priority based on a physical property value of a material to be predicted; execution possibility information indicating execution possibility of synthesis of the material; and synthesis success/failure information indicating success/failure of synthesis processing, wherein the synthetic material selection data structure is used in processing in which, while targeting a material group, the synthesis success/failure information of which indicates unprocessed, the processing unit searches for materials in descending order of the priority based on the priority information and selects a material, success/failure of the synthesis processing of which is indicated by the synthesis success/failure information satisfies a predetermined standard.

20. A manufacturing method comprising:

performing material selection according to execution possibility information output by a machine learning model;
instructing a control device to perform synthesis processing for the selected materials;
updating information of an executability database based on a synthesis processing result from the control device;
updating the machine learning model based on the updated execution possibility information of the database; and
manufacturing the updated machine learning model.

# FIG.1

1

MATERIAL MANUFACTURING DEVICE — 10

ANALYZING DEVICE — 12

SYNTHESIZING DEVICE — 11

MEASURING DEVICE — 13

EXTERNAL STORAGE DEVICE — 50

CONTROL NETWORK — N

CONTROL DEVICE

CONTROL INTERFACE — 21

20

PHYSICAL PROPERTY DB 22

EXECUTABILITY DB 23

PHYSICAL PROPERTY PREDICTION MODEL 24

PHYSICAL PROPERTY PREDICTION MODEL UPDATE REGION 24'

EXECUTION POSSIBILITY MODEL 25

EXECUTION POSSIBILITY MODEL UPDATE REGION 25'

SEARCH AGENT 26

STORAGE UNIT — 201

PROCESSING UNIT — 202

OPERATION UNIT — 203

DISPLAY UNIT — 204

# FIG.2

```
START
  │
  ▼
```
**S1** SET RANGE OF MATERIAL SEARCH SPACE AND SET TARGET VALUE
```
  │
  ▼
```
**S2** SET MATERIAL SEARCH SPACE
```
  │
  ▼
```
**S3** SELECT CANDIDATE MATERIALS
```
  │
  ▼
```
**S4** SYNTHESIZE
```
  │
  ▼
```
**S5** SUCCEEDED IN SYNTHESIS? ── NO
```
  │ YES
  ▼
```
**S6** MEASURE
```
  │
  ▼
```
**S7** SUCCEEDED IN MEASUREMENT? ── NO
```
  │ YES
  ▼
```
**S8** ACHIEVED TARGET VALUE?
  NO (left) ─── YES (down) ─── NO (right)

**S10** CONSTRUCT/UPDATE PHYSICAL PROPERTY PREDICTION MODEL

**S9** CONSTRUCT/UPDATE EXECUTION POSSIBILITY MODEL

**S11** CONSTRUCT/UPDATE PHYSICAL PROPERTY PREDICTION MODEL
```
  │
  ▼
```
**S12** CONSTRUCT/UPDATE EXECUTION POSSIBILITY MODEL
```
  │
  ▼
END
```

# FIG.3

START

$F_{PATH}$ — S21

$F_{Step=1}$ * — S22

*

⋮

$F_{Step=Ns}$ * — S23

$N_S$

$F_{MEAS}$ * — S24

END

# FIG.4

DB1

| ID | MOLECULE ID | DESCRIPTOR | | | | | | PHYSICAL PROPERTY VALUE | | MATERIAL ATTRIBUTE | | ... |
| --- | --- | --- | --- | --- | --- | --- | --- | --- | --- | --- | --- | --- |
| | | LabuteASA | Chi0v | Chi0n | Chi0 | Kappa1 | ... | Heat Capacity | ... | #1 | ... | ... |
| 1 | m011046 | 48.14077 | 4.670448 | 4.670448 | 5.776021 | 4.766589 | ... | 30.171 | ... | ... | ... | ... |
| 2 | m107356 | 53.81355 | 5.399812 | 5.399812 | 6.483128 | 5.086451 | ... | 32.772 | ... | ... | ... | ... |
| 3 | m052470 | 57.65724 | 6.5 | 6.5 | 7.328427 | 8.12 | ... | 40.33 | ... | ... | ... | ... |
| 4 | m129971 | 51.44016 | 4.741453 | 4.741453 | 6.267585 | 4.602967 | ... | 28.514 | ... | ... | ... | ... |
| 5 | m001698 | 43.95071 | 4.568534 | 4.568534 | 5.328427 | 4.644083 | ... | 26.425 | ... | ... | ... | ... |
| 6 | m125820 | 52.36183 | 5.010162 | 5.010162 | 6.690234 | 5.810208 | ... | 30.655 | ... | ... | ... | ... |
| 7 | m012809 | 49.02187 | 4.800585 | 4.800585 | 5.612884 | 4.566693 | ... | 28.913 | ... | ... | ... | ... |
| 8 | m115051 | 56.71196 | 6.131863 | 6.131863 | 6.690234 | 6.814943 | ... | 36.386 | ... | ... | ... | ... |
| ... | ... | ... | ... | ... | ... | ... | ... | ... | ... | ... | ... | ... |

# FIG.5

EP 4 428 866 A1

| ID | MOLECULE ID | DESCRIPTOR | | | | | EXECUTABILITY | | ··· DB2 |
|---|---|---|---|---|---|---|---|---|---|
| | | TPSA | qed | SlogP_VSA2 | SlogP_VSA5 | ··· | SYNTHESIS PASS/FAIL | ··· | ··· |
| 1 | m063618 | 37.15 | 0.277848 | 29.17693 | 13.84747 | ··· | 0 | ··· | ··· |
| 2 | m040261 | 21.59 | 0.478675 | 18.54647 | 25.68329 | ··· | 0 | ··· | ··· |
| 3 | m096134 | 0 | 0.46555 | 0 | 39.02785 | ··· | 1 | ··· | ··· |
| 4 | m095276 | 41.82 | 0.494861 | 29.75696 | 12.84164 | ··· | 0 | ··· | ··· |
| 5 | m033494 | 0 | 0.489954 | 0 | 32.10411 | ··· | 1 | ··· | ··· |
| 6 | m000216 | 25.78 | 0.452557 | 9.967957 | 0 | ··· | 0 | ··· | ··· |
| 7 | m007847 | 17.07 | 0.530189 | 5.783245 | 26.68912 | ··· | 0 | ··· | ··· |
| 8 | m098890 | 54.34 | 0.373354 | 32.38708 | 0 | ··· | 0 | ··· | ··· |
| 9 | m110357 | 43.47 | 0.436741 | 45.94934 | 6.923737 | ··· | 0 | ··· | ··· |
| 10 | m098090 | 37.15 | 0.38051 | 29.67985 | 13.34456 | ··· | 0 | ··· | ··· |

# FIG.6

DB3

| ID | MOLECULE ID | SMILES |
|----|-------------|--------|
| 1 | m001001 | Cn1cncc1O |
| 2 | m001002 | Cn1cc[nH]c1=O |
| 3 | m001003 | Cn1ccoc1=N |
| 4 | m001004 | Cn1ccoc1=O |
| 5 | m001005 | C#Cc1ccc[nH]1 |
| 6 | m001006 | C#Cc1ccco1 |
| 7 | m001007 | C#Cc1cnc[nH]1 |
| 8 | m001008 | C#Cc1cnco1 |
| 9 | m001009 | C#Cc1ncc[nH]1 |
| 10 | m001010 | C#Cc1ncco1 |

# FIG.7

EP 4 428 866 A1

| MOLECULE ID | m131002 |
|---|---|
| SMILES | O=CCOc1nnon1 |

MOLECULAR STRUCTURE
(SMILES)

| DESCRIPTOR NAME | VALUE |
|---|---|
| LabuteASA | 49.76079 |
| Chi0v | 4.350843 |
| Chi0n | 4.350843 |
| Chi0 | 6.527098 |
| Kappa1 | 5.672626 |

DESCRIPTOR

PHYSICAL PROPERTY
PREDICTION MODEL
(MACHINE LEARNING
MODEL)

PHYSICAL PROPERTY
PREDICTION MODEL

| PHYSICAL PROPERTY | PREDICTION VALUE |
|---|---|
| Heat Capacity (kcal/mol) | $\mu$ =26.033000 $\sigma$ =2.0 |

PREDICTION VALUE

# FIG.8

| MOLECULE ID | m121111 |
|---|---|
| SMILES | CCCC1COC(C)C1 |

MOLECULAR STRUCTURE
(SMILES)

| DESCRIPTOR NAME | VALUE |
|---|---|
| TPSA | 9.23 |
| qed | 0.553718 |
| SlogP_VSA2 | 12.71085 |
| SlogP_VSA5 | 33.10994 |

DESCRIPTOR

EXECUTABILITY
MODEL
(MACHINE LEARNING
MODEL)

EXECUTION
POSSIBILITY MODEL

| | PREDICTION VALUE |
|---|---|
| EXECUTION POSSIBILITY | 0.73 |

EXECUTION POSSIBILITY

EP 4 428 866 A1

# FIG.9

**FIRST TIME**

| | MOLECULE ID | priority | SYNTHESIS SUCCESS |
|---|---|---|---|
| 1 | m100275 | 8.401969 | 0 |
| 2 | m099997 | 8.307018 | 0 |
| 3 | m099724 | 8.280699 | 0 |
| 4 | m059059 | 8.230863 | 1 |
| 5 | m101538 | 8.164909 | 0 |
| 6 | m056516 | 8.158998 | 0 |
| 7 | m100681 | 8.129722 | 1 |
| 8 | m098931 | 8.070134 | 0 |
| 9 | m057677 | 8.052448 | 0 |
| 10 | m099259 | 8.006865 | 0 |

⟹ FAILURE (DB UNCHANGED)

**SECOND TIME**

| | MOLECULE ID | priority | SYNTHESIS SUCCESS |
|---|---|---|---|
| 1 | m100275 | 8.401969 | 0 |
| 2 | m099997 | 8.307018 | 0 |
| 3 | m099724 | 8.280699 | 0 |
| 4 | m059059 | 8.230863 | 1 |
| 5 | m101538 | 8.164909 | 0 |
| 6 | m056516 | 8.158998 | 0 |
| 7 | m100681 | 8.129722 | 1 |
| 8 | m098931 | 8.070134 | 0 |
| 9 | m057677 | 8.052448 | 0 |
| 10 | m099259 | 8.006865 | 0 |

⟹ FAILURE (DB UNCHANGED)

**THIRD TIME**

| | MOLECULE ID | priority | SYNTHESIS SUCCESS |
|---|---|---|---|
| 1 | m100275 | 8.401969 | 0 |
| 2 | m099997 | 8.307018 | 0 |
| 3 | m099724 | 8.280699 | 0 |
| 4 | m059059 | 8.230863 | 1 |
| 5 | m101538 | 8.164909 | 0 |
| 6 | m056516 | 8.158998 | 0 |
| 7 | m100681 | 8.129722 | 1 |
| 8 | m098931 | 8.070134 | 0 |
| 9 | m057677 | 8.052448 | 0 |
| 10 | m099259 | 8.006865 | 0 |

⟹ FAILURE (DB UNCHANGED)

**FOURTH TIME**

| | MOLECULE ID | priority | SYNTHESIS SUCCESS |
|---|---|---|---|
| 1 | m100275 | 8.401969 | 0 |
| 2 | m099997 | 8.307018 | 0 |
| 3 | m099724 | 8.280699 | 0 |
| 4 | m059059 | 8.230863 | 1 |
| 5 | m101538 | 8.164909 | 0 |
| 6 | m056516 | 8.158998 | 0 |
| 7 | m100681 | 8.129722 | 1 |
| 8 | m098931 | 8.070134 | 0 |
| 9 | m057677 | 8.052448 | 0 |
| 10 | m099259 | 8.006865 | 0 |

⟹ SUCCESS (DB UPDATED)

**FIFTH TIME**

| | MOLECULE ID | priority1 | SYNTHESIS SUCCESS |
|---|---|---|---|
| 1 | m100686 | 8.158031 | 0 |
| 2 | m118045 | 8.152681 | 0 |
| 3 | m099485 | 8.141112 | 0 |
| 4 | m059848 | 8.048926 | 0 |
| 5 | m060625 | 7.955551 | 0 |
| 6 | m057331 | 7.953258 | 1 |
| 7 | m059872 | 7.93976 | 0 |
| 8 | m084127 | 7.923226 | 0 |
| 9 | m109634 | 7.915219 | 0 |
| 10 | m058141 | 7.895821 | 0 |

⟹ FAILURE (DB UNCHANGED)

**SIXTH TIME**

| | MOLECULE ID | priority1 | SYNTHESIS SUCCESS |
|---|---|---|---|
| 1 | m100686 | 8.158031 | 0 |
| 2 | m118045 | 8.152681 | 0 |
| 3 | m099485 | 8.141112 | 0 |
| 4 | m059848 | 8.048926 | 0 |
| 5 | m060625 | 7.955551 | 0 |
| 6 | m057331 | 7.953258 | 1 |
| 7 | m059872 | 7.93976 | 0 |
| 8 | m084127 | 7.923226 | 0 |
| 9 | m109634 | 7.915219 | 0 |
| 10 | m058141 | 7.895821 | 0 |

EP 4 428 866 A1

# FIG.10

**FIRST TIME**

| | MOLE-CULE ID | priority | feasibility | SUC-CESS |
|---|---|---|---|---|
| 1 | m100275 | 8.401969 | 0.359791 | 0 |
| 2 | m099997 | 8.307018 | 0.288497 | 0 |
| 3 | m099724 | 8.280699 | 0.3041 | 0 |
| 4 | m059059 | 8.230863 | **0.545815** | 1 |
| 5 | m101538 | 8.164909 | 0.289338 | 0 |
| 6 | m056516 | 8.158998 | 0.323867 | 0 |
| 7 | m100681 | 8.129722 | 0.588041 | 1 |
| 8 | m098931 | 8.070134 | 0.267406 | 0 |
| 9 | m057677 | 8.052448 | 0.49412 | 0 |
| 10 | m099259 | 8.006865 | 0.354645 | 0 |

SUCCESS PHYSICAL PROPERTY DB: UPDATED EXECUTABILITY DB: UPDATED

**SECOND TIME**

| | MOLE-CULE ID | priority1 | feasibility1 | SUC-CESS |
|---|---|---|---|---|
| 1 | m100686 | 8.158031 | **0.547181** | 0 |
| 2 | m118045 | 8.152681 | 0.319512 | 0 |
| 3 | m099485 | 8.141112 | 0.331745 | 0 |
| 4 | m059848 | 8.048926 | 0.307558 | 0 |
| 5 | m060625 | 7.955551 | 0.021369 | 0 |
| 6 | m057331 | 7.953258 | 0.557112 | 1 |
| 7 | m059872 | 7.93976 | 0.309614 | 0 |
| 8 | m084127 | 7.923226 | 0.410081 | 0 |
| 9 | m109634 | 7.915219 | 0.287636 | 0 |
| 10 | m058141 | 7.895821 | 0.329093 | 0 |

FAILURE PHYSICAL PROPERTY DB: UNCHANGED EXECUTABILITY DB: **UPDATED**

**THIRD TIME**

| | MOLE-CULE ID | priority1 | feasibility2 | SUC-CESS |
|---|---|---|---|---|
| 1 | m100686 | 8.158031 | 0.485144 | 0 |
| 2 | m118045 | 8.152681 | 0.328208 | 0 |
| 3 | m099485 | 8.141112 | 0.401706 | 0 |
| 4 | m059848 | 8.048926 | 0.281703 | 0 |
| 5 | m060625 | 7.955551 | 0.021 | 0 |
| 6 | m057331 | 7.953258 | **0.562691** | 1 |
| 7 | m059872 | 7.93976 | 0.321053 | 0 |
| 8 | m084127 | 7.923226 | 0.577457 | 0 |
| 9 | m109634 | 7.915219 | 0.308154 | 0 |
| 10 | m058141 | 7.895821 | 0.414014 | 0 |

SUCCESS PHYSICAL PROPERTY DB: UPDATED EXECUTABILITY DB: UPDATED

**FOURTH TIME**

| | MOLE-CULE ID | priority2 | feasibility3 | SUC-CESS |
|---|---|---|---|---|
| 1 | m058131 | 7.971431 | 0.301096 | 0 |
| 2 | m099870 | 7.93129 | 0.398436 | 0 |
| 3 | m055570 | 7.906083 | 0.227538 | 0 |
| 4 | m057247 | 7.868201 | 0.465708 | 0 |
| 5 | m059872 | 7.866576 | 0.309089 | 0 |
| 6 | m055501 | 7.864107 | 0.301189 | 0 |
| 7 | m055512 | 7.852034 | 0.181992 | 0 |
| 8 | m100706 | 7.830578 | 0.000941 | 0 |
| 9 | m058141 | 7.817227 | 0.363749 | 0 |
| 10 | m100755 | 7.80803 | 0.172632 | 0 |
| 11 | m100099 | 7.797914 | 0.305102 | 0 |
| 12 | m056150 | 7.794978 | 0.19775 | 0 |
| 13 | m122879 | 7.794746 | **0.505407** | 1 |

SUCCESS PHYSICAL PROPERTY DB: UPDATED EXECUTABILITY DB: UPDATED

**FIFTH TIME**

| | MOLE-CULE ID | priority3 | feasibility4 | SUC-CESS |
|---|---|---|---|---|
| 1 | m057876 | 7.806809 | 0.333306 | 0 |
| 2 | m099856 | 7.780308 | 0.20514 | 0 |
| 3 | m133522 | 7.775522 | 0.493386 | 1 |
| 4 | m057295 | 7.749413 | 0.407896 | 0 |
| 5 | m057331 | 7.748417 | **0.520726** | 1 |
| 6 | m059945 | 7.730604 | 0.480065 | 1 |
| 7 | m099821 | 7.700144 | 0.359422 | 0 |
| 8 | m057709 | 7.677288 | 0.511901 | 0 |
| 9 | m099983 | 7.663628 | 0.711246 | 1 |
| 10 | m055501 | 7.659815 | 0.244398 | 0 |

SUCCESS PHYSICAL PROPERTY DB: UPDATED EXECUTABILITY DB: UPDATED

**SIXTH TIME**

| | MOLE-CULE ID | priority4 | feasibility5 | SUC-CESS |
|---|---|---|---|---|
| 1 | m057331 | 7.644805 | **0.507768** | 1 |
| 2 | m099870 | 7.636644 | 0.359825 | 0 |
| 3 | m058127 | 7.620178 | 0.49041 | 0 |
| 4 | m099821 | 7.592838 | 0.34242 | 0 |
| 5 | m057241 | 7.590556 | 0.463854 | 0 |
| 6 | m099029 | 7.589576 | 0.42946 | 0 |
| 7 | m059945 | 7.580756 | 0.538687 | 1 |
| 8 | m099025 | 7.557371 | 0.645307 | 1 |
| 9 | m099983 | 7.550303 | 0.696394 | 1 |
| 10 | m055512 | 7.547578 | 0.161265 | 0 |

EP 4 428 866 A1

# FIG.11

CANDIDATE MATERIAL TABLE

**FIRST TIME: FAILURE**

| | MOLE-CULE ID | priority | SYN-THE-SIS PASS/FAIL | MEAS-URE-MENT PASS/FAIL |
|---|---|---|---|---|
| 1 | **m100275** | **8.401969** | **0** | **n/a** |
| 2 | m099997 | 8.307018 | n/a | n/a |
| 3 | m099724 | 8.280699 | n/a | n/a |
| 4 | m059059 | 8.230863 | n/a | n/a |
| 5 | m101538 | 8.164909 | n/a | n/a |
| 6 | m056516 | 8.158998 | n/a | n/a |
| 7 | m100681 | 8.129722 | n/a | n/a |
| 8 | m098931 | 8.070134 | n/a | n/a |
| 9 | m057677 | 8.052448 | n/a | n/a |
| 10 | m099259 | 8.006865 | n/a | n/a |

**SECOND TIME: FAILURE**

| | MOLE-CULE ID | priority | SYN-THE-SIS PASS/FAIL | MEAS-URE-MENT PASS/FAIL |
|---|---|---|---|---|
| 1 | m100275 | 8.401969 | 0 | n/a |
| 2 | **m099997** | **8.307018** | **0** | **n/a** |
| 3 | m099724 | 8.280699 | n/a | n/a |
| 4 | m059059 | 8.230863 | n/a | n/a |
| 5 | m101538 | 8.164909 | n/a | n/a |
| 6 | m056516 | 8.158998 | n/a | n/a |
| 7 | m100681 | 8.129722 | n/a | n/a |
| 8 | m098931 | 8.070134 | n/a | n/a |
| 9 | m057677 | 8.052448 | n/a | n/a |
| 10 | m099259 | 8.006865 | n/a | n/a |

**THIRD TIME: FAILURE**

| | MOLE-CULE ID | priority | SYN-THE-SIS PASS/FAIL | MEAS-URE-MENT PASS/FAIL |
|---|---|---|---|---|
| 1 | m100275 | 8.401969 | 0 | n/a |
| 2 | m099997 | 8.307018 | 0 | n/a |
| 3 | **m099724** | **8.280699** | **0** | **n/a** |
| 4 | m059059 | 8.230863 | n/a | n/a |
| 5 | m101538 | 8.164909 | n/a | n/a |
| 6 | m056516 | 8.158998 | n/a | n/a |
| 7 | m100681 | 8.129722 | n/a | n/a |
| 8 | m098931 | 8.070134 | n/a | n/a |
| 9 | m057677 | 8.052448 | n/a | n/a |
| 10 | m099259 | 8.006865 | n/a | n/a |

...

PHYSICAL PROPERTY PREDICTION MODEL

Priority EVALUATION
(FROM PREDICTION AVERAGE $\mu$, PREDICTION VARIANCE $\sigma$)

INITIAL MODEL #0

LEARNING

PHYSICAL PROPERTY DATABASE

| | MOLE-CULE ID | PHYSICAL PROPERTY VALUE Heat Capacity |
|---|---|---|
| 1 | m039508 | 32.472 |
| 2 | m115634 | 35.339 |
| 3 | m067829 | 28.25 |
| 4 | m090420 | 33.39 |
| ... | ... | ... |
| n | m089578 | 31.609 |

INITIAL DATA #0

# FIG.12

**CANDIDATE MATERIAL TABLE**

FOURTH TIME: FAILURE

| | MOLE-CULE ID | priority | SYN-THE-SIS PASS/FAIL | MEAS-URE-MENT PASS/FAIL |
|---|---|---|---|---|
| 1 | m100275 | 8.401969 | 0 | n/a |
| 2 | m099997 | 8.307018 | 0 | n/a |
| 3 | m099724 | 8.280699 | 0 | n/a |
| 4 | **m059059** | **8.230863** | 1 | 1 |
| 5 | m101538 | 8.164909 | n/a | n/a |
| 6 | m056516 | 8.158998 | n/a | n/a |
| 7 | m100681 | 8.129722 | n/a | n/a |
| 8 | m098931 | 8.070134 | n/a | n/a |
| 9 | m057677 | 8.052448 | n/a | n/a |
| 10 | m099259 | 8.006865 | n/a | n/a |

⋮

FIFTH TIME: FAILURE

| | MOLE-CULE ID | priority1 | SYN-THE-SIS PASS/FAIL | MEAS-URE-MENT PASS/FAIL |
|---|---|---|---|---|
| 1 | **m100686** | **8.158031** | 0 | n/a |
| 2 | m118045 | 8.152681 | n/a | n/a |
| 3 | m099485 | 8.141112 | n/a | n/a |
| 4 | m059848 | 8.048926 | n/a | n/a |
| 5 | m060625 | 7.955551 | n/a | n/a |
| 6 | m057331 | 7.953258 | n/a | n/a |
| 7 | m059872 | 7.93976 | n/a | n/a |
| 8 | m084127 | 7.923226 | n/a | n/a |
| 9 | m109634 | 7.915219 | n/a | n/a |
| 10 | m058141 | 7.895821 | n/a | n/a |

SIXTH TIME: FAILURE

| | MOLE-CULE ID | priority1 | SYN-THE-SIS PASS/FAIL | MEAS-URE-MENT PASS/FAIL |
|---|---|---|---|---|
| 1 | m100686 | 8.158031 | 0 | n/a |
| 2 | **m118045** | **8.152681** | 0 | n/a |
| 3 | m099485 | 8.141112 | n/a | n/a |
| 4 | m059848 | 8.048926 | n/a | n/a |
| 5 | m060625 | 7.955551 | n/a | n/a |
| 6 | m057331 | 7.953258 | n/a | n/a |
| 7 | m059872 | 7.93976 | n/a | n/a |
| 8 | m084127 | 7.923226 | n/a | n/a |
| 9 | m109634 | 7.915219 | n/a | n/a |
| 10 | m058141 | 7.895821 | n/a | n/a |

Priority UPDATE

**PHYSICAL PROPERTY PREDICTION MODEL** — MODEL #1

DATA UPDATE

MODEL UPDATE

**PHYSICAL PROPERTY DATABASE**

DATA #1

| | MOLE-CULE ID | PHYSICAL PROPERTY VALUE Heat Capacity |
|---|---|---|
| 1 | m039508 | 32.472 |
| 2 | m115634 | 35.339 |
| 3 | m067829 | 28.25 |
| 4 | m090420 | 33.39 |
| ... | ... | ... |
| n | m089578 | 31.609 |
| **n+1** | **m059059** | **42.78** |

# FIG.13

EP 4 428 866 A1

**CANDIDATE MATERIAL TABLE**

FIRST TIME: SUCCESS

| | MOLE-CULE ID | priority | feasibility | SYNTHESIS PASS/FAIL | MEASURE-MENT PASS/FAIL |
|---|---|---|---|---|---|
| 1 | m100275 | 8.401969 | 0.359791 | n/a | n/a |
| 2 | m099997 | 8.307018 | 0.288497 | n/a | n/a |
| 3 | m099724 | 8.280699 | 0.3041 | n/a | n/a |
| 4 | **m059059** | **8.230863** | **0.545815** | **1** | **1** |
| 5 | m101538 | 8.164909 | 0.289338 | n/a | n/a |
| 6 | m056516 | 8.158998 | 0.323867 | n/a | n/a |
| 7 | m100681 | 8.129722 | 0.588041 | n/a | n/a |
| 8 | m098931 | 8.070134 | 0.267406 | n/a | n/a |
| 9 | m057677 | 8.052448 | 0.49412 | n/a | n/a |
| 10 | m099259 | 8.006865 | 0.354645 | n/a | n/a |

SECOND TIME: FAILURE

| | MOLE-CULE ID | priority1 | feasibility1 | SYNTHESIS PASS/FAIL | MEASURE-MENT PASS/FAIL |
|---|---|---|---|---|---|
| 1 | m100686 | 8.158031 | 0.547181 | 0 | n/a |
| 2 | m118045 | 8.152681 | 0.319512 | n/a | n/a |
| 3 | m099485 | 8.141112 | 0.331745 | n/a | n/a |
| 4 | m059848 | 8.048926 | 0.307558 | n/a | n/a |
| 5 | m060625 | 7.955551 | 0.021369 | n/a | n/a |
| 6 | m057331 | 7.953258 | 0.557112 | n/a | n/a |
| 7 | m059872 | 7.93976 | 0.309614 | n/a | n/a |
| 8 | m084127 | 7.923226 | 0.410081 | n/a | n/a |
| 9 | m109634 | 7.915219 | 0.287636 | n/a | n/a |
| 10 | m058141 | 7.895821 | 0.329093 | n/a | n/a |

Priority EVALUATION    Feasibility EVALUATION    Priority EVALUATION    Feasibility EVALUATION

**PHYSICAL PROPERTY PREDICTION MODEL** — **PHYSICAL PROPERTY PREDICTION MODEL #0** — **PHYSICAL PROPERTY PREDICTION MODEL #1**

INITIAL MODEL    DATA UPDATE    DATA UPDATE

**EXECUTION POSSIBILITY MODEL** — **EXECUTION POSSIBILITY MODEL #0** — **EXECUTION POSSIBILITY MODEL #1**

LEARNING    LEARNING    LEARNING    LEARNING

**PHYSICAL PROPERTY DB**

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |

INITIAL DATA

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| **n+1** | **m059059** | **42.78** |

**EXECUTABILITY DB**

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |

INITIAL DATA

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| **n+1** | **m059059** | **1** | **1** |

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| **n+2** | **m100686** | **0** | **n/a** |

# FIG.14

**THIRD TIME: SUCCESS**

| | MOLE-CULE ID | priority1 | feasibility2 | SYNTHESIS PASS/FAIL | MEASURE-MENT PASS/FAIL |
|---|---|---|---|---|---|
| 1 | m100686 | 8.158031 | 0.485144 | 0 | n/a |
| 2 | m118045 | 8.152681 | 0.328208 | n/a | n/a |
| 3 | m099485 | 8.141112 | 0.401706 | n/a | n/a |
| 4 | m059848 | 8.048926 | 0.281703 | n/a | n/a |
| 5 | m060625 | 7.955551 | 0.021 | n/a | n/a |
| 6 | m057331 | 7.953258 | 0.562691 | 1 | 1 |
| 7 | m059872 | 7.93976 | 0.321053 | n/a | n/a |
| 8 | m084127 | 7.923226 | 0.577457 | n/a | n/a |
| 9 | m109634 | 7.915219 | 0.308154 | n/a | n/a |
| 10 | m058141 | 7.895821 | 0.414014 | n/a | n/a |

**FOURTH TIME: SUCCESS**

| | MOLE-CULE ID | priority2 | feasibility3 | SYNTHESIS PASS/FAIL | MEASURE-MENT PASS/FAIL |
|---|---|---|---|---|---|
| 1 | m058131 | 7.971431 | 0.301096 | n/a | n/a |
| 2 | m099870 | 7.93129 | 0.398436 | n/a | n/a |
| 3 | m055570 | 7.906083 | 0.227538 | n/a | n/a |
| 4 | m057247 | 7.868201 | 0.465708 | n/a | n/a |
| 5 | m059872 | 7.866576 | 0.309089 | n/a | n/a |
| 6 | m055501 | 7.864107 | 0.301189 | n/a | n/a |
| 7 | m055512 | 7.852034 | 0.181992 | n/a | n/a |
| 8 | m100706 | 7.830578 | 0.000941 | n/a | n/a |
| ... | | | | ... | ... |
| 13 | m122879 | 7.794746 | 0.505407 | 1 | 1 |

CANDIDATE MATERIAL TABLE

Feasibility EVALUATION

Priority EVALUATION

Feasibility EVALUATION

PHYSICAL PROPERTY PREDICTION MODEL

PHYSICAL PROPERTY PREDICTION MODEL #2

DATA UPDATE

DATA UPDATE

DATA UPDATE

EXECUTION POSSIBILITY MODEL

EXECUTION POSSIBILITY MODEL #2

EXECUTION POSSIBILITY MODEL #3

LEARNING

LEARNING

LEARNING

LEARNING

PHYSICAL PROPERTY DB

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| n+1 | m059059 | 42.78 |
| n+2 | m057331 | 43.651 |

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| n+1 | m059059 | 42.78 |
| n+2 | m057331 | 43.651 |
| n+3 | m122879 | 44.302 |

EXECUTABILITY DB

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |
| n+3 | m057331 | 1 | 1 |

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |
| n+3 | m057331 | 1 | 1 |
| n+4 | m122879 | 1 | 1 |

EP 4 428 866 A1

# FIG.15

EP 4 428 866 A1

CANDIDATE MATERIAL TABLE

**FIFTH TIME: SUCCESS**

| | MOLE-CULE ID | priority1 | feasibility1 | SYNTHESIS PASS/FAIL | MEASURE-MENT PASS/FAIL |
|---|---|---|---|---|---|
| 1 | m057876 | 7.806809 | 0.333306 | n/a | n/a |
| 2 | m099856 | 7.780308 | 0.20514 | n/a | n/a |
| 3 | m133522 | 7.775522 | 0.493386 | n/a | n/a |
| 4 | m057295 | 7.749413 | 0.407896 | n/a | n/a |
| 5 | **m057331** | **7.748417** | **0.520726** | **1** | **1** |
| 6 | m059945 | 7.730604 | 0.480065 | n/a | n/a |
| 7 | m099821 | 7.700144 | 0.359422 | n/a | n/a |
| 8 | m057709 | 7.677288 | 0.511901 | n/a | n/a |
| 9 | m099983 | 7.663628 | 0.711246 | n/a | n/a |
| 10 | m055501 | 7.659815 | 0.244398 | n/a | n/a |

**SIXTH TIME: SUCCESS**

| | MOLE-CULE ID | priority1 | feasibility2 | SYNTHESIS PASS/FAIL | MEASURE-MENT PASS/FAIL |
|---|---|---|---|---|---|
| 1 | m100686 | 8.158031 | 0.485144 | 0 | n/a |
| 2 | m118045 | 8.152681 | 0.328208 | n/a | n/a |
| 3 | m099485 | 8.141112 | 0.401706 | n/a | n/a |
| 4 | m059848 | 8.048926 | 0.281703 | n/a | n/a |
| 5 | m060625 | 7.955551 | 0.021 | n/a | n/a |
| 6 | m057331 | 7.953258 | 0.562691 | 1 | 1 |
| 7 | m059872 | 7.93976 | 0.321053 | n/a | n/a |
| 8 | **m084127** | **7.923226** | **0.577457** | **1** | **1** |
| 9 | m109634 | 7.915219 | 0.308154 | n/a | n/a |
| 10 | m058141 | 7.895821 | 0.414014 | n/a | n/a |

PHYSICAL PROPERTY PREDICTION MODEL

PHYSICAL PROPERTY PREDICTION MODEL #3

PHYSICAL PROPERTY PREDICTION MODEL #4

PHYSICAL PROPERTY PREDICTION MODEL #5

Priority EVALUATION — Feasibility EVALUATION

DATA UPDATE

EXECUTION POSSIBILITY MODEL

EXECUTION POSSIBILITY MODEL #4

EXECUTION POSSIBILITY MODEL #5

EXECUTION POSSIBILITY MODEL #6

LEARNING

PHYSICAL PROPERTY DB

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| n+1 | m059059 | 42.78 |
| n+2 | m057331 | 43.651 |
| **n+3** | **m122879** | **44.302** |

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| n+1 | m059059 | 42.78 |
| n+2 | m057331 | 43.651 |
| n+3 | m122879 | 44.302 |
| **n+4** | **m057331** | **43.651** |

| | MOLE-CULE ID | PHYSICAL PROPERTY VALUE Heat Capacity |
|---|---|---|
| 1 | m039508 | 32.472 |
| ... | ... | ... |
| n | m089578 | 31.609 |
| n+1 | m059059 | 42.78 |
| n+2 | m057331 | 43.651 |
| n+3 | m122879 | 44.302 |
| n+4 | m057331 | 43.651 |
| **n+5** | **m084127** | **45.01** |

TARGET ACHIEVED: SEARCH COMPLETED

EXECUTABILITY DB

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |
| n+3 | m057331 | 1 | 1 |
| **n+4** | **m122879** | **1** | **1** |

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |
| n+3 | m057331 | 1 | 1 |
| n+4 | m122879 | 1 | 1 |
| **n+5** | **m057331** | **1** | **1** |

| | MOLE-CULE ID | SYNTHESIS PASS/FAIL | MEASURE-MENT PASS/FAIL |
|---|---|---|---|
| 1 | m039508 | 1 | 1 |
| ... | ... | ... | ... |
| n | m089578 | 1 | 1 |
| n+1 | m059059 | 1 | 1 |
| n+2 | m100686 | 0 | n/a |
| n+3 | m057331 | 1 | 1 |
| n+4 | m122879 | 1 | 1 |
| n+5 | m057331 | 1 | 1 |
| **n+6** | **m084127** | **1** | **1** |

# FIG.16

DB4

|  | MOLECULE ID | SYNTHESIS & MEASUREMENT PASS/FAIL |
|---|---|---|
| 1 | m100275 | 0 |
| 2 | m099997 | 0 |
| 3 | m099724 | 0 |
| 4 | m059059 | 1 |
| 5 | m101538 | 0 |
| 6 | m056516 | 0 |
| 7 | m100681 | 1 |
| 8 | m098931 | 0 |
| 9 | m057677 | 1 |
| 10 | m099259 | 0 |
| … | … | … |

# FIG.17

DB5

|  | MOLECULE ID | SYN-THESIS YIELD |
|---|---|---|
| 1 | m100275 | 0.0 |
| 2 | m099997 | 0.0 |
| 3 | m099724 | 0.0 |
| 4 | m059059 | 0.2 |
| 5 | m101538 | 0.0 |
| 6 | m056516 | 0.0 |
| 7 | m100681 | 0.6 |
| 8 | m098931 | 0.0 |
| 9 | m057677 | 0.8 |
| 10 | m099259 | 0.0 |
| … | … | … |

# FIG.18

DB6

| | MOLECULE ID | SYN-THESIS PASS/FAIL | ISOLA-TION PASS/FAIL | ABSORPTION SPECTRUM MEASUREMENT PASS/FAIL |
|---|---|---|---|---|
| 1 | m100275 | 0 | n/a | n/a |
| 2 | m099997 | 0 | n/a | n/a |
| 3 | m099724 | 0 | n/a | n/a |
| 4 | m059059 | 1 | 1 | 1 |
| 5 | m101538 | 0 | n/a | n/a |
| 6 | m056516 | 0 | n/a | n/a |
| 7 | m100681 | 1 | 1 | 1 |
| 8 | m098931 | 0 | n/a | n/a |
| 9 | m057677 | 1 | 0 | n/a |
| 10 | m099259 | 0 | n/a | n/a |
| ... | ... | ... | ... | ... |

# FIG.19

DB7

| | MOLECULE ID | SYN-THESIS PASS/FAIL | SOLVENT A SOLUBILITY | SOLVENT B SOLUBILITY | PHYSICAL PROPERTY A MEASURE-MENT | PHYSICAL PROPERTY B MEASURE-MENT |
|---|---|---|---|---|---|---|
| 1 | m100275 | 0 | n/a | n/a | n/a | n/a |
| 2 | m099997 | 0 | n/a | n/a | n/a | n/a |
| 3 | m099724 | 0 | n/a | n/a | n/a | n/a |
| 4 | m059059 | 1 | 0.8 | 0.6 | 1 | 0 |
| 5 | m101538 | 0 | n/a | n/a | n/a | n/a |
| 6 | m056516 | 0 | n/a | n/a | n/a | n/a |
| 7 | m100681 | 1 | 0.1 | 0.8 | 1 | 1 |
| 8 | m098931 | 0 | n/a | n/a | n/a | n/a |
| 9 | m057677 | 1 | 0.1 | 0.1 | 0 | 1 |
| 10 | m099259 | 0 | n/a | n/a | n/a | n/a |
| ... | ... | ... | ... | ... | ... | ... |

# FIG.20

DB8

|   | MOLECULE ID | SYNTHESIS PATH ID | SYN-THESIS PASS/ FAIL | ISOLA-TION PASS/ FAIL | ABSORPTION SPECTRUM MEASUREMENT PASS/FAIL |
|---|---|---|---|---|---|
| 1 | m100275 | r100275_01 | 0 | n/a | n/a |
| 2 | m099997 | r099997_01 | 0 | n/a | n/a |
| 3 | m099724 | r099724_01 | 0 | n/a | n/a |
| 4 | m059059 | r059059_01 | 1 | 1 | 1 |
| 5 | m101538 | r101538_01 | 0 | n/a | n/a |
| 6 | m056516 | r056516_01 | 0 | n/a | n/a |
| 7 | m100681 | r100681_01 | 1 | 1 | 1 |
| 8 | m098931 | r098931_01 | 0 | n/a | n/a |
| 9 | m057677 | r057677_01 | 1 | 0 | n/a |
| 10 | m099259 | r099259_01 | 0 | n/a | n/a |
| ... | ... | ... | ... | ... | ... |

# FIG.21

DB9

| | SYNTHESIS PATH ID | REACTION STEP 1 | REACTION STEP 2 | ... |
|---|---|---|---|---|
| 1 | r100275_01 | [MATERIAL MOLECULE ID1, MATERIAL MOLECULE ID2, ···], [CONDITION 1, CONDITION 2, ···] | - | ... |
| 2 | r099997_01 | [MATERIAL MOLECULE ID1, MATERIAL MOLECULE ID2, ···], [CONDITION 1, CONDITION 2, ···] | - | ... |
| 3 | r099724_01 | [MATERIAL MOLECULE ID1, MATERIAL MOLECULE ID2, ···], [CONDITION 1, CONDITION 2, ···] | [MATERIAL MOLECULE ID1, MATERIAL MOLECULE ID2, ···], [CONDITION 1, CONDITION 2, ···] | ... |
| 4 | r059059_01 | [MATERIAL MOLECULE ID1, MATERIAL MOLECULE ID2, ···], [CONDITION 1, CONDITION 2, ···] | - | ... |
| 5 | r101538_01 | [MATERIAL MOLECULE ID1, MATERIAL MOLECULE ID2, ···], [CONDITION 1, CONDITION 2, ···] | - | ... |
| 6 | r056516_01 | [MATERIAL MOLECULE ID1, MATERIAL MOLECULE ID2, ···], [CONDITION 1, CONDITION 2, ···] | - | ... |
| 7 | r100681_01 | [MATERIAL MOLECULE ID1, MATERIAL MOLECULE ID2, ···], [CONDITION 1, CONDITION 2, ···] | [MATERIAL MOLECULE ID1, MATERIAL MOLECULE ID2, ···], [CONDITION 1, CONDITION 2, ···] | ... |
| 8 | r098931_01 | [MATERIAL MOLECULE ID1, MATERIAL MOLECULE ID2, ···], [CONDITION 1, CONDITION 2, ···] | - | ... |
| 9 | r057677_01 | [MATERIAL MOLECULE ID1, MATERIAL MOLECULE ID2, ···], [CONDITION 1, CONDITION 2, ···] | - | ... |
| 10 | r099259_01 | [MATERIAL MOLECULE ID1, MATERIAL MOLECULE ID2, ···], [CONDITION 1, CONDITION 2, ···] | - | ... |
| ... | ... | ... | ... | ... |

EP 4 428 866 A1

# FIG.22

SEARCH AGENT
SYNTHESIS

CONTROL INTERFACE
SYNTHESIS

SYNTHESIZING DEVICE
SYNTHESIS

ANALYZING DEVICE
SYNTHESIS

S101 TRANSMIT SELECTED MATERIAL SYNTHESIS LIST TO SYNTHESIZING DEVICE VIA CONTROL INTERFACE

S102 INSTRUCT SYNTHESIZING DEVICE TO SYNTHESIZE MATERIALS DESIGNATED IN LIST

S103 WAIT FOR RESULT INFORMATION OF SYNTHESIS OF ONE MATERIAL IN LIST FROM SYNTHESIZING DEVICE

S104 PROCESS RESULT ACCORDING TO DETERMINATION IN STEP S5 IN FIG. 2

S105 ENDED SYNTHESIS PROCESSING FOR ALL MOLECULE IDs SHOWN IN LIST?
NO
YES

END

S111 TRANSMIT "SYNTHESIS FAILURE" IN CASE OF "SYNTHESIS IMPOSSIBLE", "ABNORMAL END", AND "SYNTHESIS FAILURE" AND TRANSMIT "SYNTHESIS SUCCESS" IN CASE OF "SYNTHESIS SUCCESS"

S120 NEXT SYNTHESIS TARGET *

S121 RECEIVE MATERIAL SYNTHESIS LIST {MOLECULE ID, ⋯}

S122 READ TARGET {MOLECULAR ID (, SYNTHESIS PATH ID)} ACCORDING TO PRIORITY OF LIST

S123 SATISFY CONDITION OF REACTION STEP DESIGNATED BY SYNTHESIS PATH ID?
NO "SYN-THESIS IMPOS-SIBLE"
YES

S124 MATERIAL SYNTHESIS OF MOLECULE ID

S127 NEXT REACTION STEP

S125 ABNORMAL END?
YES "ABNORMAL END"
NO

S126 NEXT REACTION STEP IS PRESENT?
YES
NO : GIVE INSTRUCTION ON ANALYSIS PROCESSING

S131 PROVIDE SYNTHESIZED MATERIAL FROM SYNTHESIZING DEVICE TO ANALYZING DEVICE AND PERFORM ANALYSIS PROCESSING

S128 PROVIDE NOTIFICATION OF ANALYSIS RESULT: SUCCESS "SYNTHESIS SUCCESS", FAILURE "SYNTHESIS FAILURE"

S129 MOLE-CULE ID OF NEXT SYNTHESIS TARGET IS STILL PRESENT IN LIST?
NO
YES *

58

# FIG.23

SEARCH AGENT
( MEASUREMENT )

S201
TRANSMIT MOLECULE ID FOR WHICH SYNTHESIS HAS BEEN SUCCESSFUL TO SYNTHESIZING DEVICE

S202
DESIGNATE PHYSICAL PROPERTY THAT SHOULD BE MEASURED TO SYNTHESIZING DEVICE AND GIVE INSTRUCTION ON MEASUREMENT OF MATERIAL

S203
WAIT FOR RESULT INFORMATION OF SYNTHESIS OF ONE MATERIAL IN LIST FROM SYNTHESIZING DEVICE

S204
PROCESS RESULT ACCORDING TO DETERMINATION IN STEP S7 IN FIG. 2

( END )

CONTROL INTERFACE
( MEASUREMENT )

S211
TRANSMIT "MEASUREMENT FAILURE" IN CASE OF "MEASUREMENT IMPOSSIBLE" AND TRANSMIT "MEASUREMENT SUCCESS" IN CASE OF "MEASUREMENT END"

SYNTHESIZING DEVICE
( MEASUREMENT )

S221
RECEIVE MOLECULE ID

S222
PROVIDE SYNTHESIZED MATERIAL DESIGNATED BY MOLECULE ID TO MEASURING DEVICE AND GIVE INSTRUCTION ON MEASUREMENT ABOUT DESIGNATED PHYSICAL PROPERTY

S223
PROVIDE NOTIFICATION OF MEASUREMENT RESULT: SUCCESS "MEASUREMENT END", FAILURE "MEASUREMENT IMPOSSIBLE"

MEASURING DEVICE
( MEASUREMENT )

S231
PERFORM MEASUREMENT PROCESSING FOR SYNTHESIZED MATERIAL ABOUT DESIGNATED PHYSICAL PROPERTY

EP 4 428 866 A1

# FIG.24

EP 4 428 866 A1

# FIG.25

INFORMATION PROCESSING DEVICE 100

| COMMUNICA-TION UNIT 110 | CONTROL UNIT 130 | STORAGE UNIT 120 |

# FIG.26

10A

| | | |
|---|---|---|
| ANALYZING DEVICE ⌇12 | SYNTHESIZING DEVICE ⌇11 | MEASURING DEVICE ⌇13 |

MT

MOVABLE

14

| DRIVING DEVICE ~121 | DRIVING DEVICE ~111 | DRIVING DEVICE ~131 |
|---|---|---|
| PROCESSOR ~122 | PROCESSOR ~112 | PROCESSOR ~132 |
| COMMUNI-CATION INTERFACE ~123 | COMMUNI-CATION INTERFACE ~113 | COMMUNI-CATION INTERFACE ~133 |

# FIG.27

10B

15    15

| ANALYZING DEVICE ⌇12 | SYNTHESIZING DEVICE ⌇11 | MEASURING DEVICE ⌇13 |
|---|---|---|

MT

DRIVING DEVICE ~111

| PROCESSOR ~122 | PROCESSOR ~112 | PROCESSOR ~132 |
|---|---|---|
| COMMUNI-CATION INTERFACE ~123 | COMMUNI-CATION INTERFACE ~113 | COMMUNI-CATION INTERFACE ~133 |

# FIG.28

# FIG.29

COMPUTER 1000

CPU 1100

RAM 1200

ROM 1300

1050

HDD 1400

PROGRAM DATA 1450

COMMUNICATION INTERFACE 1500

INPUT/OUTPUT INTERFACE 1600

1550

INPUT/OUTPUT DEVICE 1650

# FIG.30

# FIG.31

## INTERNATIONAL SEARCH REPORT

| International application No. |
| --- |
| **PCT/JP2022/039200** |

**A. CLASSIFICATION OF SUBJECT MATTER**

*G16C 60/00*(2019.01)i; *G06F 16/23*(2019.01)i

FI:   G16C60/00; G06F16/23

According to International Patent Classification (IPC) or to both national classification and IPC

**B. FIELDS SEARCHED**

Minimum documentation searched (classification system followed by classification symbols)

G16C60/00; G06F16/23

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
| --- | --- | --- |
| Y | JP 2005-166053 A (ACCELRYS SOFTWARE INC) 23 June 2005 (2005-06-23) paragraphs [0016]-[0081] | 1-20 |
| Y | 畑中 美穂, 化学におけるマテリアルズインフォマティクスの現状と課題, 人工知能, 01 May 2019, vol. 34, no. 3, pp. 351-357, ISSN: 2188-2266 pp. 351-357, (HATANAKA, Miho. Journal of the Japanese Society for Artificial Intelligence.), non-official translation (Current status and challenges of materials informatics in chemistry) | 1-20 |
| Y | JP 2003-014728 A (PFIZER PHARMACEUTICALS INC) 15 January 2003 (2003-01-15) paragraphs [0002]-[0026] | 1-20 |
| A | JP 2021-140701 A (TOYOTA CENTRAL RES & DEV) 16 September 2021 (2021-09-16) entire text, all drawings | 1-20 |

☐ Further documents are listed in the continuation of Box C.   ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **14 December 2022** | **27 December 2022** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

**INTERNATIONAL SEARCH REPORT**
Information on patent family members

International application No.

**PCT/JP2022/039200**

| Patent document cited in search report | | | Publication date (day/month/year) | Patent family member(s) | | | Publication date (day/month/year) |
|---|---|---|---|---|---|---|---|
| JP | 2005-166053 | A | 23 June 2005 | US | 2005/0182775 | A1 | |
| | | | | paragraphs [0037]-[0117] | | | |
| | | | | US | 2005/0187918 | A1 | |
| | | | | EP | 1538537 | A2 | |
| JP | 2003-014728 | A | 15 January 2003 | US | 2003/0069698 | A1 | |
| | | | | paragraphs [0003]-[0051] | | | |
| | | | | EP | 1167969 | A2 | |
| | | | | CA | 2350341 | A1 | |
| JP | 2021-140701 | A | 16 September 2021 | US | 2021/0280277 | A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017221444 A

**Non-patent literature cited in the description**

- **HISAKI IKEBATA ; KENTA HONGO ; TETSU ISOMURA ; RYO MAEZONO ; RYO YOSHIDA.** Bayesian molecular design with a chemical language model. *Journal of Computer-Aided Molecular Design,* 2017, vol. 31, 379-391

- **KIM, Y. ; KIM, E. ; ANTONO, E. et al.** Machine-learned metrics for predicting the likelihood of success in materials discovery. *npj Comput Mater,* 2020, vol. 6, 131